# EUROPEAN PATENT APPLICATION

(11) **EP 4 567 046 A1**
(43) Date of publication of application: **11.06.2025**
(21) Application number: 23849539.4
(22) Date of filing: 04.08.2023
(51) Int. Cl.: C07K 16/30, C07K 19/00, C12N 15/13, C12N 15/62, C12N 15/64, C12N 5/10, A61K 39/395, A61K 35/17, A61P 35/00, A61K 47/68, C07K 16/46, G01N 33/577

(54) **ANTIBODY TARGETING EGFRVIII AND USE THEREOF IN CELL IMMUNOTHERAPY**

(30) Priority: 05.08.2022 CN 202210936403
(71) Applicant: Beijing DCTY Biotech Co., Ltd., Beijing 102200 (CN)
(72) Inventor: SONG, Yujie, Beijing 102200 (CN); LI, Shuang, Beijing 102200 (CN); ZHOU, Zishan, Beijing 102200 (CN); ZHANG, Rong, Beijing 102200 (CN)
(74) Representative: Di Giovine, Paolo
(86) International application number: PCT/CN2023/111305
(87) International publication number: WO 2024/027835

(57) **Abstract**

Provided is an antibody targeting EGFRvIII or an antigen-binding fragment thereof. Further provided are a CAR comprising the antigen-binding fragment targeting EGFRvIII, and treatment and detection use thereof.

## Description

### Cross-reference to Related Application

The present application claims priority of the Chinese patent application no. CN202210936403.1 filed on August 5, 2022, which is hereby incorporated herein by reference in its entirety.

### Technical Field

The present application relates to an antibody targeting EGFRvIII or an antigen-binding fragment thereof. This application further relates to a CAR including the antigen-binding fragment targeting EGFRvIII and use thereof.

### Background

The cellular immunotherapy is an emerging technology for treating a tumor, which shows a promising utilization prospect in the field of tumor treatment. Engineered T cells have been successfully used to treat blood tumors. Due to their unprecedented efficacies in clinical trials, as of now, there are 8 chimeric antigen receptor (CAR)-T cell therapies in the world which have been approved for use in the treatment of B-cell malignancies and myeloma.

Although the cellular immunotherapy has achieved remarkable efficacies in treating blood tumors, its use in treatment of solid tumors is limited by some factors, for example, the presence of immunosuppressive microenvironment in a solid tumor, and the poor persistence and infiltration abilities of CAR-T cells in a tumor. These limiting factors call for novel modifications and breakthroughs for cellular immunotherapy products (Akhavan *et al*., 2019).

Another key factor for cellular immunotherapy is selection of the target, which determines the safety and efficacy of a cellular therapy product. Tumor antigens may be divided into tumor associated antigens (TAAs) and tumor specific antigens (TSAs). The TAA is expressed in normal tissues, and a cellular therapy product targeting a TAA may easily induce an off-target toxicity, while the cellular therapy product targeting a TSA is safer.

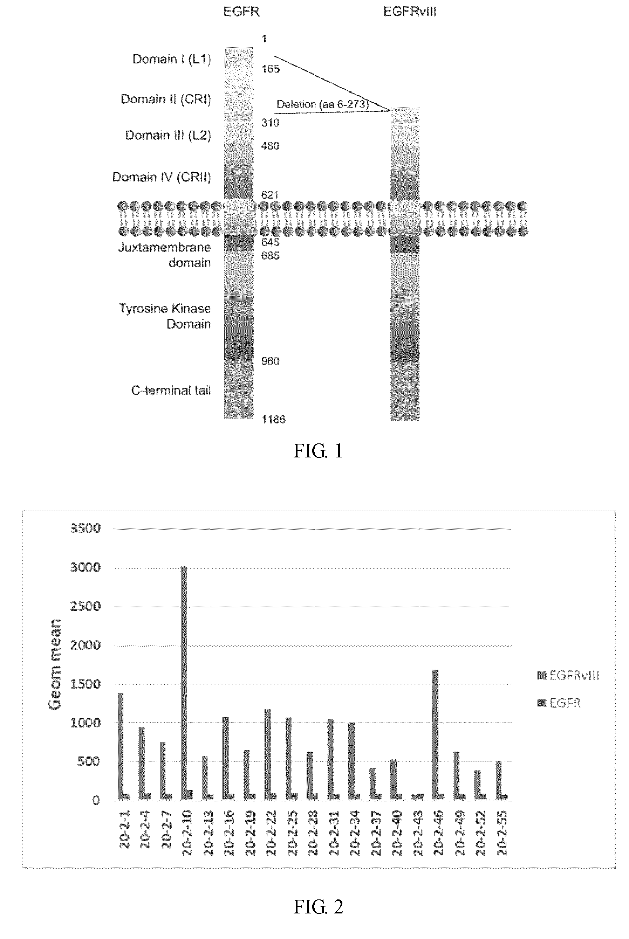

The EGFRvIII antigen is a tumor-specific target antigen and is the most common extracellular-region-mutant form of the EGFR gene (Chistiakov *et al*., 2017). It is based on removal of 801 bases from exons 2-7 of the EGFR coding region, lacking 267 amino acids in the extracellular region compared with the wild-type EGFR, thereby generating a fusion region with a new glycine residue between the exons 1 and 8 (FIG 1; An *et al*., 2018), forming a tumor-specific deletion mutation. Compared with the wild-type EGFR, EGFRvIII is ligand-independent and constitutively activated (Chistiakov *et al*., 2017). This mutation is found in a variety of cancers such as breast cancer, lung cancer, and head and neck cancer, but most commonly in glioblastoma (Chistiakov *et al*., 2017; Del Vecchio *et al*., 2012).

Glioblastoma accounts for approximately 15% of brain tumors, and nearly 210,000 people worldwide are expected to be diagnosed each year. It is a malignant grade 4 tumor having features such as high aggressiveness, proneness to recurrence, and a high rate of death or disability. It progresses so rapidly that its 5-year overall survival rate is one of the lowest among all human cancers, and there is no effective treatment. Currently, scientific workers are planning to solve this worldwide problem through immunotherapy (Feldman *et al*., 2022; Londhe and Date, 2020).

Research results have shown that EGFRvIII is expressed in glioblastoma but not in normal tissues. Patients with a higher level of malignancy have a higher EGFRvIII expression rate. Therefore, use of the CAR-T cells targeting the EGFRvIII antigen to treat a brain glioma has the advantages such as high specificity, low off-target toxicity, and reduced side effects (Zhu *et al*., 2021).

There have been clinical studies which demonstrate that anti-tumor medicaments targeting EGFRvIII have significant safety and preliminary efficacies (O'Rourke *et al*., 2017). Antibody medicament is an effective anti-cancer medicament that has been relatively well developed in recent years. It can target a tumor cell with a particular antigen and stimulate a patient's immune system to fight cancer cells through effects such as ADCC and CDC.

Currently, there is still an urgent need for an anti-tumor medicament targeting EGFRvIII with better safety and efficacy in the market.

### Summary of the Invention

In an aspect, provided herein is an antibody targeting EGFRvIII or antigen-binding fragment thereof, wherein the antibody comprises a heavy chain variable region (HCVR) which includes an HCDR1, an HCDR2, and an HCDR3 selected from one of the following combinations:
(1) HCDR1 whose amino acid sequence is DFSMH (SEQ ID NO: 1);
   HCDR2 whose amino acid sequence is WINTETGEPSYADDFKG (SEQ ID NO: 2); and
   HCDR3 whose amino acid sequence is YGYDVRGDY (SEQ ID NO: 3);
(2) HCDR1 whose amino acid sequence is DYYMH (SEQ ID NO: 4);
   HCDR2 whose amino acid sequence is WIDPENGNTIYDPKFQG (SEQ ID NO: 5); and
   HCDR3 whose amino acid sequence is GWFAY (SEQ ID NO: 6);
(3) HCDR1 whose amino acid sequence is DYSIH (SEQ ID NO: 7);
   HCDR2 whose amino acid sequence is WINTETGEPTYADDFKG (SEQ ID NO: 8); and
   HCDR3 whose amino acid sequence is YGYDVRGDY (SEQ ID NO: 9);
(4) HCDR1 whose amino acid sequence is DYYLH (SEQ ID NO: 10);
   HCDR2 whose amino acid sequence is WIDPENGNTIYDPKFQG (SEQ ID NO: 11); and
   HCDR3 whose amino acid sequence is GYLTY (SEQ ID NO: 12);
(5) HCDR1 whose amino acid sequence is RYWMH (SEQ ID NO: 13);
   HCDR2 whose amino acid sequence is EINPSNGRANYNEKFMS (SEQ ID NO: 14); and
   HCDR3 whose amino acid sequence is GREITTGFAY (SEQ ID NO: 15);
(6) HCDR1 whose amino acid sequence is DYYMH (SEQ ID NO: 16);
   HCDR2 whose amino acid sequence is WIDPENGNTIYDPKFQG (SEQ ID NO: 17); and
   HCDR3 whose amino acid sequence is GYLVY (SEQ ID NO: 18);
(7) HCDR1 whose amino acid sequence is DYYMH (SEQ ID NO: 19);
   HCDR2 whose amino acid sequence is WIDPENGNTIYDPKFQG (SEQ ID NO: 20); and
   HCDR3 whose amino acid sequence is GYLAY (SEQ ID NO: 21);
(8) HCDR1 whose amino acid sequence is DYYMH (SEQ ID NO: 22);
   HCDR2 whose amino acid sequence is WIDPENGNTIYDPKFQG (SEQ ID NO: 23); and
   HCDR3 whose amino acid sequence is GWFAY (SEQ ID NO: 25);
(9) HCDR1 whose amino acid sequence is DYYMH (SEQ ID NO: 25);
   HCDR2 whose amino acid sequence is WIDPENGNTIYDPKFQG (SEQ ID NO: 26); and
   HCDR3 whose amino acid sequence is GYLVY (SEQ ID NO: 27);
(10) HCDR1 whose amino acid sequence is DYYMH (SEQ ID NO: 28);
   HCDR2 whose amino acid sequence is WIDPENGNTIYDPKFQG (SEQ ID NO: 29); and
   HCDR3 whose amino acid sequence is GWFAY (SEQ ID NO: 30);
(11) HCDR1 whose amino acid sequence is DYYMH (SEQ ID NO: 31);
   HCDR2 whose amino acid sequence is WIDPENGNTIYDPKFQG (SEQ ID NO: 32); and
   HCDR3 whose amino acid sequence is GYLVY (SEQ ID NO: 33);
(12) HCDR1 whose amino acid sequence is DYYMH (SEQ ID NO: 34);
   HCDR2 whose amino acid sequence is WIDPENGNTIYDPKFQG (SEQ ID NO: 35); and
   HCDR3 whose amino acid sequence is GYLVY (SEQ ID NO: 36);
(13) HCDR1 whose amino acid sequence is NYAMS (SEQ ID NO: 37);
   HCDR2 whose amino acid sequence is TITSGGSYTYYPDSVKG (SEQ ID NO: 38); and
   HCDR3 whose amino acid sequence is KDYGNYWFAY (SEQ ID NO: 39);
(14) HCDR1 whose amino acid sequence is GYAMS (SEQ ID NO: 40);
   HCDR2 whose amino acid sequence is TITSGGSYTYYPDSVKG (SEQ ID NO: 41); and
   HCDR3 whose amino acid sequence is KDYGNYWFAY (SEQ ID NO: 42);
(15) HCDR1 whose amino acid sequence is GYAMS (SEQ ID NO: 43);
   HCDR2 whose amino acid sequence is TITSGGSYTYYPDSVKG (SEQ ID NO: 44); and
   HCDR3 whose amino acid sequence is KDYGNYWFAY (SEQ ID NO: 45);
(16) HCDR1 whose amino acid sequence is SGYSWH (SEQ ID NO: 46);
   HCDR2 whose amino acid sequence is YIHYSGSTNYNPPLKS (SEQ ID NO: 47); and
   HCDR3 whose amino acid sequence is GVVSNYAMGN (SEQ ID NO: 48);
(17) HCDR1 whose amino acid sequence is TYWMH (SEQ ID NO: 49);
   HCDR2 whose amino acid sequence is YINPNTAYTEYNQNFKD (SEQ ID NO: 50); and
   HCDR3 whose amino acid sequence is GAYYRTYYAMDY (SEQ ID NO: 51);
(18) HCDR1 whose amino acid sequence is NYGMN (SEQ ID NO: 52);
   HCDR2 whose amino acid sequence is WINTYTGEPTYADDFKG (SEQ ID NO: 53); and
   HCDR3 whose amino acid sequence is EEFYSRGAMDY (SEQ ID NO: 54); and
(19) HCDR1 whose amino acid sequence is DYYIN (SEQ ID NO: 55);
   HCDR2 whose amino acid sequence is WIYPGSGNTKYNEKFKG (SEQ ID NO: 56); and
   HCDR3 whose amino acid sequence is SSRCDF (SEQ ID NO: 57), or
   the antibody comprises a variant of an HCDR sequence combination in any one of (1)-(19), wherein the variant has at least 90% sequence identity with the HCDR sequences in any one of (1)-(19), or comprises a total of at least 1 and no more than 10 or no more than 5, 4, 3, or 2 amino acid changes to the HCDR sequences.

In some embodiments, the antibody further comprises a light chain variable region (LCVR) which includes an LCDR1, an LCDR2, and an LCDR3 selected from one of the following combinations:
(1) LCDR1 whose amino acid sequence is SASSSISSNYLH (SEQ ID NO: 58);
   LCDR2 whose amino acid sequence is GTSNLAS (SEQ ID NO: 59); and
   LCDR3 whose amino acid sequence is HQGSSIPLT (SEQ ID NO: 60);
(2) LCDR1 whose amino acid sequence is KSSQSLLNSSNQKNHLA (SEQ ID NO: 61);
   LCDR2 whose amino acid sequence is FASTRAS (SEQ ID NO: 62); and
   LCDR3 whose amino acid sequence is QQHYSTPLT (SEQ ID NO: 63);
(3) LCDR1 whose amino acid sequence is SASSGISSNYLH (SEQ ID NO: 64);
   LCDR2 whose amino acid sequence is STSNLAS (SEQ ID NO: 65); and
   LCDR3 whose amino acid sequence is HQGSDIPLT (SEQ ID NO: 66);
(4) LCDR1 whose amino acid sequence is KSSQNLLNSSNQKNYLA (SEQ ID NO: 67);
   LCDR2 whose amino acid sequence is FASTRYS (SEQ ID NO: 68); and
   LCDR3 whose amino acid sequence is QQHYSTPLT (SEQ ID NO: 69);
(5) LCDR1 whose amino acid sequence is KASQSVSNDVV (SEQ ID NO: 70);
   LCDR2 whose amino acid sequence is YASNRYT (SEQ ID NO: 71); and
   LCDR3 whose amino acid sequence is QQDYSSPWT (SEQ ID NO: 72);
(6) LCDR1 whose amino acid sequence is KSSQSLLNSSNQKNYLA (SEQ ID NO: 73);
   LCDR2 whose amino acid sequence is FASTRES (SEQ ID NO: 74); and
   LCDR3 whose amino acid sequence is QQHYSIPLT (SEQ ID NO: 75);
(7) LCDR1 whose amino acid sequence is KSSQSLLNSSNQKNYLA (SEQ ID NO: 76);
   LCDR2 whose amino acid sequence is FASTRKS (SEQ ID NO: 77); and
   LCDR3 whose amino acid sequence is QQHYSTPLT (SEQ ID NO: 78);
(8) LCDR1 whose amino acid sequence is KSSQSLLNSSNQKNHLA (SEQ ID NO: 79);
   LCDR2 whose amino acid sequence is FASTRQS (SEQ ID NO: 80); and
   LCDR3 whose amino acid sequence is QQHYSTPLT (SEQ ID NO: 81);
(9) LCDR1 whose amino acid sequence is KSSQSLLNSSNQKNYLA (SEQ ID NO: 82);
   LCDR2 whose amino acid sequence is FASTRQS (SEQ ID NO: 83); and
   LCDR3 whose amino acid sequence is QQHYSTPLT (SEQ ID NO: 84);
(10) LCDR1 whose amino acid sequence is KSSQSLLNSSNQKNHLA (SEQ ID NO: 85);
   LCDR2 whose amino acid sequence is FASTRGS (SEQ ID NO: 86); and
   LCDR3 whose amino acid sequence is QQHYSTPLT (SEQ ID NO: 87);
(11) LCDR1 whose amino acid sequence is KSSQSLLNSSNQKNYLA (SEQ ID NO: 88);
   LCDR2 whose amino acid sequence is FASTRDS (SEQ ID NO: 89); and
   LCDR3 whose amino acid sequence is QQHYSTPLT (SEQ ID NO: 90);
(12) LCDR1 whose amino acid sequence is KSSQSLLNSSNQKNYLA (SEQ ID NO: 91);
   LCDR2 whose amino acid sequence is FASTRES (SEQ ID NO: 92); and
   LCDR3 whose amino acid sequence is QQHYSTPLT (SEQ ID NO: 93);
(13) LCDR1 whose amino acid sequence is RSSQSLVHSDGNTYLH (SEQ ID NO: 94);
   LCDR2 whose amino acid sequence is KVSNRFS (SEQ ID NO: 95); and
   LCDR3 whose amino acid sequence is SQSIHVPWT (SEQ ID NO: 96);
(14) LCDR1 whose amino acid sequence is SASSSVSYMH (SEQ ID NO: 97);
   LCDR2 whose amino acid sequence is STSNLAS (SEQ ID NO: 98); and LCDR3 whose amino acid sequence is QQRSSYPLT (SEQ ID NO: 99);
(15) LCDR1 whose amino acid sequence is RSSQSLVHSDGNTYLH (SEQ ID NO: 100);
   LCDR2 whose amino acid sequence is KVSNRFS (SEQ ID NO: 101); and
   LCDR3 whose amino acid sequence is SQTTQVPWT (SEQ ID NO: 102);
(16) LCDR1 whose amino acid sequence is ITNTDIDDDMN (SEQ ID NO: 103);
   LCDR2 whose amino acid sequence is EGNTLRP (SEQ ID NO: 104); and
   LCDR3 whose amino acid sequence is LQSDDLPLT (SEQ ID NO: 105);
(17) LCDR1 whose amino acid sequence is KASQSVDYDGDSYMN (SEQ ID NO: 106);
   LCDR2 whose amino acid sequence is AASNLES (SEQ ID NO: 107); and
   LCDR3 whose amino acid sequence is LQSNEDPYT (SEQ ID NO: 108);
(18) LCDR1 whose amino acid sequence is RSSQFIVHSNGNTYLE (SEQ ID NO: 109);
   LCDR2 whose amino acid sequence is KISNRFS (SEQ ID NO: 110); and
   LCDR3 whose amino acid sequence is FQGSHVPFT (SEQ ID NO: 111); and
(19) LCDR1 whose amino acid sequence is KASEDIYNRLA (SEQ ID NO: 112);
   LCDR2 whose amino acid sequence is GATSLET (SEQ ID NO: 113); and
   LCDR3 whose amino acid sequence is QQYWSSPLT (SEQ ID NO: 114), or
   the antibody comprises a variant of an LCDR sequence combination in any one of (1)-(19), wherein the variant has at least 90% sequence identity with the LCDR sequences in any one of (1)-(19), or comprises a total of at least 1 and no more than 10 or no more than 5, 4, 3, or 2 amino acid changes to the LCDR sequences.

In some embodiments, the amino acid sequence of the heavy chain variable region is selected from any of the following:
(1) the sequence shown in SEQ ID NO: 115 or a heavy chain variable region sequence having at least 90%, 95%, 96%, 97%, 98% or 99% sequence identity therewith;
(2) the sequence shown in SEQ ID NO: 117 or a heavy chain variable region sequence having at least 90%, 95%, 96%, 97%, 98% or 99% sequence identity therewith;
(3) the sequence shown in SEQ ID NO: 119 or a heavy chain variable region sequence having at least 90%, 95%, 96%, 97%, 98% or 99% sequence identity therewith;
(4) the sequence shown in SEQ ID NO: 121 or a heavy chain variable region sequence having at least 90%, 95%, 96%, 97%, 98% or 99% sequence identity therewith;
(5) the sequence shown in SEQ ID NO: 123 or a heavy chain variable region sequence having at least 90%, 95%, 96%, 97%, 98% or 99% sequence identity therewith;
(6) the sequence shown in SEQ ID NO: 125 or a heavy chain variable region sequence having at least 90%, 95%, 96%, 97%, 98% or 99% sequence identity therewith;
(7) the sequence shown in SEQ ID NO: 127 or a heavy chain variable region sequence having at least 90%, 95%, 96%, 97%, 98% or 99% sequence identity therewith;
(8) the sequence shown in SEQ ID NO: 129 or a heavy chain variable region sequence having at least 90%, 95%, 96%, 97%, 98% or 99% sequence identity therewith;
(9) the sequence shown in SEQ ID NO: 131 or a heavy chain variable region sequence having at least 90%, 95%, 96%, 97%, 98% or 99% sequence identity therewith;
(10) the sequence shown in SEQ ID NO: 133 or a heavy chain variable region sequence having at least 90%, 95%, 96%, 97%, 98% or 99% sequence identity therewith;
(11) the sequence shown in SEQ ID NO: 135 or a heavy chain variable region sequence having at least 90%, 95%, 96%, 97%, 98% or 99% sequence identity therewith;
(12) the sequence shown in SEQ ID NO: 137 or a heavy chain variable region sequence having at least 90%, 95%, 96%, 97%, 98% or 99% sequence identity therewith;
(13) the sequence shown in SEQ ID NO: 139 or a heavy chain variable region sequence having at least 90%, 95%, 96%, 97%, 98% or 99% sequence identity therewith;
(14) the sequence shown in SEQ ID NO: 141 or a heavy chain variable region sequence having at least 90%, 95%, 96%, 97%, 98% or 99% sequence identity therewith;
(15) the sequence shown in SEQ ID NO: 143 or a heavy chain variable region sequence having at least 90%, 95%, 96%, 97%, 98% or 99% sequence identity therewith;
(16) the sequence shown in SEQ ID NO: 145 or a heavy chain variable region sequence having at least 90%, 95%, 96%, 97%, 98% or 99% sequence identity therewith;
(17) the sequence shown in SEQ ID NO: 147 or a heavy chain variable region sequence having at least 90%, 95%, 96%, 97%, 98% or 99% sequence identity therewith;
(18) the sequence shown in SEQ ID NO: 149 or a heavy chain variable region sequence having at least 90%, 95%, 96%, 97%, 98% or 99% sequence identity therewith; and
(19) the sequence shown in SEQ ID NO: 151 or a heavy chain variable region sequence having at least 90%, 95%, 96%, 97%, 98% or 99% sequence identity therewith.

In some embodiments, the amino acid sequence of the heavy chain variable region and the amino acid sequence of the light chain variable region of the antibody are selected from any of the following combinations:
(1) the sequence shown in SEQ ID NO: 115 or a heavy chain variable region sequence having at least 90%, 95%, 96%, 97%, 98% or 99% sequence identity therewith, and the sequence shown in SEQ ID NO: 116 or a light chain variable region sequence having at least 90%, 95%, 96%, 97%, 98% or 99% sequence identity therewith;
(2) the sequence shown in SEQ ID NO: 117 or a heavy chain variable region sequence having at least 90%, 95%, 96%, 97%, 98% or 99% sequence identity therewith, and the sequence shown in SEQ ID NO: 118 or a light chain variable region sequence having at least 90%, 95%, 96%, 97%, 98% or 99% sequence identity therewith;
(3) the sequence shown in SEQ ID NO: 119 or a heavy chain variable region sequence having at least 90%, 95%, 96%, 97%, 98% or 99% sequence identity therewith, and the sequence shown in SEQ ID NO: 120 or a light chain variable region sequence having at least 90%, 95%, 96%, 97%, 98% or 99% sequence identity therewith;
(4) the sequence shown in SEQ ID NO: 121 or a heavy chain variable region sequence having at least 90%, 95%, 96%, 97%, 98% or 99% sequence identity therewith, and the sequence shown in SEQ ID NO: 122 or a light chain variable region sequence having at least 90%, 95%, 96%, 97%, 98% or 99% sequence identity therewith;
(5) the sequence shown in SEQ ID NO: 123 or a heavy chain variable region sequence having at least 90%, 95%, 96%, 97%, 98% or 99% sequence identity therewith, and the sequence shown in SEQ ID NO: 124 or a light chain variable region sequence having at least 90%, 95%, 96%, 97%, 98% or 99% sequence identity therewith;
(6) the sequence shown in SEQ ID NO: 125 or a heavy chain variable region sequence having at least 90%, 95%, 96%, 97%, 98% or 99% sequence identity therewith, and the sequence shown in SEQ ID NO: 126 or a light chain variable region sequence having at least 90%, 95%, 96%, 97%, 98% or 99% sequence identity therewith;
(7) the sequence shown in SEQ ID NO: 127 or a heavy chain variable region sequence having at least 90%, 95%, 96%, 97%, 98% or 99% sequence identity therewith, and the sequence shown in SEQ ID NO: 128 or a light chain variable region sequence having at least 90%, 95%, 96%, 97%, 98% or 99% sequence identity therewith;
(8) the sequence shown in SEQ ID NO: 129 or a heavy chain variable region sequence having at least 90%, 95%, 96%, 97%, 98% or 99% sequence identity therewith, and the sequence shown in SEQ ID NO: 130 or a light chain variable region sequence having at least 90%, 95%, 96%, 97%, 98% or 99% sequence identity therewith;
(9) the sequence shown in SEQ ID NO: 131 or a heavy chain variable region sequence having at least 90%, 95%, 96%, 97%, 98% or 99% sequence identity therewith, and the sequence shown in SEQ ID NO: 132 or a light chain variable region sequence having at least 90%, 95%, 96%, 97%, 98% or 99% sequence identity therewith;
(10) the sequence shown in SEQ ID NO: 133 or a heavy chain variable region sequence having at least 90%, 95%, 96%, 97%, 98% or 99% sequence identity therewith, and the sequence shown in SEQ ID NO: 134 or a light chain variable region sequence having at least 90%, 95%, 96%, 97%, 98% or 99% sequence identity therewith;
(11) the sequence shown in SEQ ID NO: 135 or a heavy chain variable region sequence having at least 90%, 95%, 96%, 97%, 98% or 99% sequence identity therewith, and the sequence shown in SEQ ID NO: 136 or a light chain variable region sequence having at least 90%, 95%, 96%, 97%, 98% or 99% sequence identity therewith;
(12) the sequence shown in SEQ ID NO: 137 or a heavy chain variable region sequence having at least 90%, 95%, 96%, 97%, 98% or 99% sequence identity therewith, and the sequence shown in SEQ ID NO: 138 or a light chain variable region sequence having at least 90%, 95%, 96%, 97%, 98% or 99% sequence identity therewith;
(13) the sequence shown in SEQ ID NO: 139 or a heavy chain variable region sequence having at least 90%, 95%, 96%, 97%, 98% or 99% sequence identity therewith, and the sequence shown in SEQ ID NO: 140 or a light chain variable region sequence having at least 90%, 95%, 96%, 97%, 98% or 99% sequence identity therewith;
(14) the sequence shown in SEQ ID NO: 141 or a heavy chain variable region sequence having at least 90%, 95%, 96%, 97%, 98% or 99% sequence identity therewith, and the sequence shown in SEQ ID NO: 142 or a light chain variable region sequence having at least 90%, 95%, 96%, 97%, 98% or 99% sequence identity therewith;
(15) the sequence shown in SEQ ID NO: 143 or a heavy chain variable region sequence having at least 90%, 95%, 96%, 97%, 98% or 99% sequence identity therewith, and the sequence shown in SEQ ID NO: 144 or a light chain variable region sequence having at least 90%, 95%, 96%, 97%, 98% or 99% sequence identity therewith;
(16) the sequence shown in SEQ ID NO: 145 or a heavy chain variable region sequence having at least 90%, 95%, 96%, 97%, 98% or 99% sequence identity therewith, and the sequence shown in SEQ ID NO: 146 or a light chain variable region sequence having at least 90%, 95%, 96%, 97%, 98% or 99% sequence identity therewith;
(17) the sequence shown in SEQ ID NO: 147 or a heavy chain variable region sequence having at least 90%, 95%, 96%, 97%, 98% or 99% sequence identity therewith, and the sequence shown in SEQ ID NO: 148 or a light chain variable region sequence having at least 90%, 95%, 96%, 97%, 98% or 99% sequence identity therewith;
(18) the sequence shown in SEQ ID NO: 149 or a heavy chain variable region sequence having at least 90%, 95%, 96%, 97%, 98% or 99% sequence identity therewith, and the sequence shown in SEQ ID NO: 150 or a light chain variable region sequence having at least 90%, 95%, 96%, 97%, 98% or 99% sequence identity therewith; and
(19) the sequence shown in SEQ ID NO: 151 or a heavy chain variable region sequence having at least 90%, 95%, 96%, 97%, 98% or 99% sequence identity therewith, and the sequence shown in SEQ ID NO: 152 or a light chain variable region sequence having at least 90%, 95%, 96%, 97%, 98% or 99% sequence identity therewith.

In some embodiments, the antibody binds to an EGFRvIII antigen with a KD value of less than 10⁻⁶ M and preferably less than 10⁻⁷ M, as determined by surface plasmon resonance (SPR).

In some embodiments, the antibody is selected from a murine antibody, a chimeric antibody, a humanized antibody, or a human antibody.

In some embodiments, the humanized antibody binds to an EGFRvIII antigen with a KD value of less than 10⁻⁶ M and preferably less than 10⁻⁷ M, as determined by surface plasmon resonance (SPR).

In some embodiments, the antigen-binding fragment comprises an Fab, an Fab', an F(ab')₂, a single domain antibody, or a single chain antibody.

In some embodiments, the humanized antibody is a single chain antibody whose amino acid sequence is selected from any of the following:
(1) the sequence shown in SEQ ID NO: 167 or a heavy chain variable region sequence having at least 90%, 95%, 96%, 97%, 98% or 99% sequence identity therewith;
(2) the sequence shown in SEQ ID NO: 169 or a heavy chain variable region sequence having at least 90%, 95%, 96%, 97%, 98% or 99% sequence identity therewith;
(3) the sequence shown in SEQ ID NO: 171 or a heavy chain variable region sequence having at least 90%, 95%, 96%, 97%, 98% or 99% sequence identity therewith;
(4) the sequence shown in SEQ ID NO: 173 or a heavy chain variable region sequence having at least 90%, 95%, 96%, 97%, 98% or 99% sequence identity therewith.
(5) the sequence shown in SEQ ID NO: 175 or a heavy chain variable region sequence having at least 90%, 95%, 96%, 97%, 98% or 99% sequence identity therewith;
(6) the sequence shown in SEQ ID NO: 177 or a heavy chain variable region sequence having at least 90%, 95%, 96%, 97%, 98% or 99% sequence identity therewith;
(7) the sequence shown in SEQ ID NO: 179 or a heavy chain variable region sequence having at least 90%, 95%, 96%, 97%, 98% or 99% sequence identity therewith;
(8) the sequence shown in SEQ ID NO: 181 or a heavy chain variable region sequence having at least 90%, 95%, 96%, 97%, 98% or 99% sequence identity therewith.
(9) the sequence shown in SEQ ID NO: 183 or a heavy chain variable region sequence having at least 90%, 95%, 96%, 97%, 98% or 99% sequence identity therewith;
(10) the sequence shown in SEQ ID NO: 185 or a heavy chain variable region sequence having at least 90%, 95%, 96%, 97%, 98% or 99% sequence identity therewith; and
(11) the sequence shown in SEQ ID NO: 187 or a heavy chain variable region sequence having at least 90%, 95%, 96%, 97%, 98% or 99% sequence identity therewith.

In some embodiments, the antibody or antigen-binding fragment thereof further comprises an Fc fragment.

In some embodiments, the antigen-binding fragment is a single chain antibody; preferably, a linker for linking the heavy chain variable region with the light chain variable region of the single chain antibody comprises the sequence shown in SEQ ID NO: 166.

In another aspect, provided herein is a chimeric antigen receptor (CAR) whose extracellular antigen-binding domain includes one or more antibody molecules targeting EGFRvIII or antigen-binding fragments thereof, wherein the HCDR1, HCDR2, and HCDR3 of the heavy chain variable region of the antibody molecule are selected from one of the following combinations:
(1) HCDR1 whose amino acid sequence is DFSMH (SEQ ID NO: 1);
   HCDR2 whose amino acid sequence is WINTETGEPSYADDFKG (SEQ ID NO: 2); and
   HCDR3 whose amino acid sequence is YGYDVRGDY (SEQ ID NO: 3);
(2) HCDR1 whose amino acid sequence is DYYMH (SEQ ID NO: 4);
   HCDR2 whose amino acid sequence is WIDPENGNTIYDPKFQG (SEQ ID NO: 5); and
   HCDR3 whose amino acid sequence is GWFAY (SEQ ID NO: 6);
(3) HCDR1 whose amino acid sequence is DYSIH (SEQ ID NO: 7);
   HCDR2 whose amino acid sequence is WINTETGEPTYADDFKG (SEQ ID NO: 8); and
   HCDR3 whose amino acid sequence is YGYDVRGDY (SEQ ID NO: 9);
(4) HCDR1 whose amino acid sequence is DYYLH (SEQ ID NO: 10);
   HCDR2 whose amino acid sequence is WIDPENGNTIYDPKFQG (SEQ ID NO: 11); and
   HCDR3 whose amino acid sequence is GYLTY (SEQ ID NO: 12);
(5) HCDR1 whose amino acid sequence is RYWMH (SEQ ID NO: 13);
   HCDR2 whose amino acid sequence is EINPSNGRANYNEKFMS (SEQ ID NO: 14); and
   HCDR3 whose amino acid sequence is GREITTGFAY (SEQ ID NO: 15);
(6) HCDR1 whose amino acid sequence is DYYMH (SEQ ID NO: 16);
   HCDR2 whose amino acid sequence is WIDPENGNTIYDPKFQG (SEQ ID NO: 17); and
   HCDR3 whose amino acid sequence is GYLVY (SEQ ID NO: 18);
(7) HCDR1 whose amino acid sequence is DYYMH (SEQ ID NO: 19);
   HCDR2 whose amino acid sequence is WIDPENGNTIYDPKFQG (SEQ ID NO: 20); and
   HCDR3 whose amino acid sequence is GYLAY (SEQ ID NO: 21);
(8) HCDR1 whose amino acid sequence is DYYMH (SEQ ID NO: 22);
   HCDR2 whose amino acid sequence is WIDPENGNTIYDPKFQG (SEQ ID NO: 23); and
   HCDR3 whose amino acid sequence is GWFAY (SEQ ID NO: 25);
(9) HCDR1 whose amino acid sequence is DYYMH (SEQ ID NO: 25);
   HCDR2 whose amino acid sequence is WIDPENGNTIYDPKFQG (SEQ ID NO: 26); and
   HCDR3 whose amino acid sequence is GYLVY (SEQ ID NO: 27);
(10) HCDR1 whose amino acid sequence is DYYMH (SEQ ID NO: 28);
   HCDR2 whose amino acid sequence is WIDPENGNTIYDPKFQG (SEQ ID NO: 29); and
   HCDR3 whose amino acid sequence is GWFAY (SEQ ID NO: 30);
(11) HCDR1 whose amino acid sequence is DYYMH (SEQ ID NO: 31);
   HCDR2 whose amino acid sequence is WIDPENGNTIYDPKFQG (SEQ ID NO: 32); and
   HCDR3 whose amino acid sequence is GYLVY (SEQ ID NO: 33);
(12) HCDR1 whose amino acid sequence is DYYMH (SEQ ID NO: 34);
   HCDR2 whose amino acid sequence is WIDPENGNTIYDPKFQG (SEQ ID NO: 35); and
   HCDR3 whose amino acid sequence is GYLVY (SEQ ID NO: 36);
(13) HCDR1 whose amino acid sequence is NYAMS (SEQ ID NO: 37);
   HCDR2 whose amino acid sequence is TITSGGSYTYYPDSVKG (SEQ ID NO: 38); and
   HCDR3 whose amino acid sequence is KDYGNYWFAY (SEQ ID NO: 39);
(14) HCDR1 whose amino acid sequence is GYAMS (SEQ ID NO: 40);
   HCDR2 whose amino acid sequence is TITSGGSYTYYPDSVKG (SEQ ID NO: 41); and
   HCDR3 whose amino acid sequence is KDYGNYWFAY (SEQ ID NO: 42);
(15) HCDR1 whose amino acid sequence is GYAMS (SEQ ID NO: 43);
   HCDR2 whose amino acid sequence is TITSGGSYTYYPDSVKG (SEQ ID NO: 44); and
   HCDR3 whose amino acid sequence is KDYGNYWFAY (SEQ ID NO: 45);
(16) HCDR1 whose amino acid sequence is SGYSWH (SEQ ID NO: 46);
   HCDR2 whose amino acid sequence is YIHYSGSTNYNPPLKS (SEQ ID NO: 47); and
   HCDR3 whose amino acid sequence is GVVSNYAMGN (SEQ ID NO: 48);
(17) HCDR1 whose amino acid sequence is TYWMH (SEQ ID NO: 49);
   HCDR2 whose amino acid sequence is YINPNTAYTEYNQNFKD (SEQ ID NO: 50); and
   HCDR3 whose amino acid sequence is GAYYRTYYAMDY (SEQ ID NO: 51);
(18) HCDR1 whose amino acid sequence is NYGMN (SEQ ID NO: 52);
   HCDR2 whose amino acid sequence is WINTYTGEPTYADDFKG (SEQ ID NO: 53); and
   HCDR3 whose amino acid sequence is EEFYSRGAMDY (SEQ ID NO: 54); and
(19) HCDR1 whose amino acid sequence is DYYIN (SEQ ID NO: 55);
   HCDR2 whose amino acid sequence is WIYPGSGNTKYNEKFKG (SEQ ID NO: 56); and
   HCDR3 whose amino acid sequence is SSRCDF (SEQ ID NO: 57), or
   the antibody comprises a variant of an HCDR sequence combination in any one of (1)-(19), wherein the variant has at least 90% sequence identity with the HCDR sequences in any one of (1)-(19), or comprises a total of at least 1 and no more than 10 or no more than 5, 4, 3, or 2 amino acid changes to the HCDR sequences.

In some embodiments, the antibody further comprises a light chain variable region (LCVR) which includes an LCDR1, an LCDR2, and an LCDR3 selected from one of the following combinations:
(1) LCDR1 whose amino acid sequence is SASSSISSNYLH (SEQ ID NO: 58);
   LCDR2 whose amino acid sequence is GTSNLAS (SEQ ID NO: 59); and
   LCDR3 whose amino acid sequence is HQGSSIPLT (SEQ ID NO: 60);
(2) LCDR1 whose amino acid sequence is KSSQSLLNSSNQKNHLA (SEQ ID NO: 61);
   LCDR2 whose amino acid sequence is FASTRAS (SEQ ID NO: 62); and
   LCDR3 whose amino acid sequence is QQHYSTPLT (SEQ ID NO: 63);
(3) LCDR1 whose amino acid sequence is SASSGISSNYLH (SEQ ID NO: 64);
   LCDR2 whose amino acid sequence is STSNLAS (SEQ ID NO: 65); and
   LCDR3 whose amino acid sequence is HQGSDIPLT (SEQ ID NO: 66);
(4) LCDR1 whose amino acid sequence is KSSQNLLNSSNQKNYLA (SEQ ID NO: 67);
   LCDR2 whose amino acid sequence is FASTRYS (SEQ ID NO: 68); and LCDR3 whose amino acid sequence is QQHYSTPLT (SEQ ID NO: 69);
(5) LCDR1 whose amino acid sequence is KASQSVSNDVV (SEQ ID NO: 70);
   LCDR2 whose amino acid sequence is YASNRYT (SEQ ID NO: 71); and
   LCDR3 whose amino acid sequence is QQDYSSPWT (SEQ ID NO: 72);
(6) LCDR1 whose amino acid sequence is KSSQSLLNSSNQKNYLA (SEQ ID NO: 73);
   LCDR2 whose amino acid sequence is FASTRES (SEQ ID NO: 74); and
   LCDR3 whose amino acid sequence is QQHYSIPLT (SEQ ID NO: 75);
(7) LCDR1 whose amino acid sequence is KSSQSLLNSSNQKNYLA (SEQ ID NO: 76);
   LCDR2 whose amino acid sequence is FASTRKS (SEQ ID NO: 77); and
   LCDR3 whose amino acid sequence is QQHYSTPLT (SEQ ID NO: 78);
(8) LCDR1 whose amino acid sequence is KSSQSLLNSSNQKNHLA (SEQ ID NO: 79);
   LCDR2 whose amino acid sequence is FASTRQS (SEQ ID NO: 80); and
   LCDR3 whose amino acid sequence is QQHYSTPLT (SEQ ID NO: 81);
(9) LCDR1 whose amino acid sequence is KSSQSLLNSSNQKNYLA (SEQ ID NO: 82);
   LCDR2 whose amino acid sequence is FASTRQS (SEQ ID NO: 83); and
   LCDR3 whose amino acid sequence is QQHYSTPLT (SEQ ID NO: 84);
(10) LCDR1 whose amino acid sequence is KSSQSLLNSSNQKNHLA (SEQ ID NO: 85);
   LCDR2 whose amino acid sequence is FASTRGS (SEQ ID NO: 86); and
   LCDR3 whose amino acid sequence is QQHYSTPLT (SEQ ID NO: 87);
(11) LCDR1 whose amino acid sequence is KSSQSLLNSSNQKNYLA (SEQ ID NO: 88);
   LCDR2 whose amino acid sequence is FASTRDS (SEQ ID NO: 89); and
   LCDR3 whose amino acid sequence is QQHYSTPLT (SEQ ID NO: 90);
(12) LCDR1 whose amino acid sequence is KSSQSLLNSSNQKNYLA (SEQ ID NO: 91);
   LCDR2 whose amino acid sequence is FASTRES (SEQ ID NO: 92); and
   LCDR3 whose amino acid sequence is QQHYSTPLT (SEQ ID NO: 93);
(13) LCDR1 whose amino acid sequence is RSSQSLVHSDGNTYLH (SEQ ID NO: 94);
   LCDR2 whose amino acid sequence is KVSNRFS (SEQ ID NO: 95); and
   LCDR3 whose amino acid sequence is SQSIHVPWT (SEQ ID NO: 96);
(14) LCDR1 whose amino acid sequence is SASSSVSYMH (SEQ ID NO: 97);
   LCDR2 whose amino acid sequence is STSNLAS (SEQ ID NO: 98); and
   LCDR3 whose amino acid sequence is QQRSSYPLT (SEQ ID NO: 99);
(15) LCDR1 whose amino acid sequence is RSSQSLVHSDGNTYLH (SEQ ID NO: 100);
   LCDR2 whose amino acid sequence is KVSNRFS (SEQ ID NO: 101); and
   LCDR3 whose amino acid sequence is SQTTQVPWT (SEQ ID NO: 102);
(16) LCDR1 whose amino acid sequence is ITNTDIDDDMN (SEQ ID NO: 103);
   LCDR2 whose amino acid sequence is EGNTLRP (SEQ ID NO: 104); and
   LCDR3 whose amino acid sequence is LQSDDLPLT (SEQ ID NO: 105);
(17) LCDR1 whose amino acid sequence is KASQSVDYDGDSYMN (SEQ ID NO: 106);
   LCDR2 whose amino acid sequence is AASNLES (SEQ ID NO: 107); and
   LCDR3 whose amino acid sequence is LQSNEDPYT (SEQ ID NO: 108);
(18) LCDR1 whose amino acid sequence is RSSQFIVHSNGNTYLE (SEQ ID NO: 109);
   LCDR2 whose amino acid sequence is KISNRFS (SEQ ID NO: 110); and
   LCDR3 whose amino acid sequence is FQGSHVPFT (SEQ ID NO: 111); and
(19) LCDR1 whose amino acid sequence is KASEDIYNRLA (SEQ ID NO: 112);
   LCDR2 whose amino acid sequence is GATSLET (SEQ ID NO: 113); and
   LCDR3 whose amino acid sequence is QQYWSSPLT (SEQ ID NO: 114), or
   the antibody comprises a variant of an LCDR sequence combination in any one of (1)-(19), wherein the variant has at least 90% sequence identity with the LCDR sequences in any one of (1)-(19), or comprises a total of at least 1 and no more than 10 or no more than 5, 4, 3, or 2 amino acid changes to the LCDR sequences.

In some embodiments, the amino acid sequence of the heavy chain variable region is selected from any of the following:
(1) the sequence shown in SEQ ID NO: 115 or a heavy chain variable region sequence having at least 90%, 95%, 96%, 97%, 98% or 99% sequence identity therewith;
(2) the sequence shown in SEQ ID NO: 117 or a heavy chain variable region sequence having at least 90%, 95%, 96%, 97%, 98% or 99% sequence identity therewith;
(3) the sequence shown in SEQ ID NO: 119 or a heavy chain variable region sequence having at least 90%, 95%, 96%, 97%, 98% or 99% sequence identity therewith;
(4) the sequence shown in SEQ ID NO: 121 or a heavy chain variable region sequence having at least 90%, 95%, 96%, 97%, 98% or 99% sequence identity therewith;
(5) the sequence shown in SEQ ID NO: 123 or a heavy chain variable region sequence having at least 90%, 95%, 96%, 97%, 98% or 99% sequence identity therewith;
(6) the sequence shown in SEQ ID NO: 125 or a heavy chain variable region sequence having at least 90%, 95%, 96%, 97%, 98% or 99% sequence identity therewith;
(7) the sequence shown in SEQ ID NO: 127 or a heavy chain variable region sequence having at least 90%, 95%, 96%, 97%, 98% or 99% sequence identity therewith;
(8) the sequence shown in SEQ ID NO: 129 or a heavy chain variable region sequence having at least 90%, 95%, 96%, 97%, 98% or 99% sequence identity therewith;
(9) the sequence shown in SEQ ID NO: 131 or a heavy chain variable region sequence having at least 90%, 95%, 96%, 97%, 98% or 99% sequence identity therewith;
(10) the sequence shown in SEQ ID NO: 133 or a heavy chain variable region sequence having at least 90%, 95%, 96%, 97%, 98% or 99% sequence identity therewith;
(11) the sequence shown in SEQ ID NO: 135 or a heavy chain variable region sequence having at least 90%, 95%, 96%, 97%, 98% or 99% sequence identity therewith;
(12) the sequence shown in SEQ ID NO: 137 or a heavy chain variable region sequence having at least 90%, 95%, 96%, 97%, 98% or 99% sequence identity therewith;
(13) the sequence shown in SEQ ID NO: 139 or a heavy chain variable region sequence having at least 90%, 95%, 96%, 97%, 98% or 99% sequence identity therewith;
(14) the sequence shown in SEQ ID NO: 141 or a heavy chain variable region sequence having at least 90%, 95%, 96%, 97%, 98% or 99% sequence identity therewith;
(15) the sequence shown in SEQ ID NO: 143 or a heavy chain variable region sequence having at least 90%, 95%, 96%, 97%, 98% or 99% sequence identity therewith;
(16) the sequence shown in SEQ ID NO: 145 or a heavy chain variable region sequence having at least 90%, 95%, 96%, 97%, 98% or 99% sequence identity therewith;
(17) the sequence shown in SEQ ID NO: 147 or a heavy chain variable region sequence having at least 90%, 95%, 96%, 97%, 98% or 99% sequence identity therewith;
(18) the sequence shown in SEQ ID NO: 149 or a heavy chain variable region sequence having at least 90%, 95%, 96%, 97%, 98% or 99% sequence identity therewith; and
(19) the sequence shown in SEQ ID NO: 151 or a heavy chain variable region sequence having at least 90%, 95%, 96%, 97%, 98% or 99% sequence identity therewith.

In some embodiments, the amino acid sequence of the heavy chain variable region and the amino acid sequence of the light chain variable region of the antibody are selected from any of the following combinations:
(1) the sequence shown in SEQ ID NO: 115 or a heavy chain variable region sequence having at least 90%, 95%, 96%, 97%, 98% or 99% sequence identity therewith, and the sequence shown in SEQ ID NO: 116 or a light chain variable region sequence having at least 90%, 95%, 96%, 97%, 98% or 99% sequence identity therewith;
(2) the sequence shown in SEQ ID NO: 117 or a heavy chain variable region sequence having at least 90%, 95%, 96%, 97%, 98% or 99% sequence identity therewith, and the sequence shown in SEQ ID NO: 118 or a light chain variable region sequence having at least 90%, 95%, 96%, 97%, 98% or 99% sequence identity therewith;
(3) the sequence shown in SEQ ID NO: 119 or a heavy chain variable region sequence having at least 90%, 95%, 96%, 97%, 98% or 99% sequence identity therewith, and the sequence shown in SEQ ID NO: 120 or a light chain variable region sequence having at least 90%, 95%, 96%, 97%, 98% or 99% sequence identity therewith;
(4) the sequence shown in SEQ ID NO: 121 or a heavy chain variable region sequence having at least 90%, 95%, 96%, 97%, 98% or 99% sequence identity therewith, and the sequence shown in SEQ ID NO: 122 or a light chain variable region sequence having at least 90%, 95%, 96%, 97%, 98% or 99% sequence identity therewith;
(5) the sequence shown in SEQ ID NO: 123 or a heavy chain variable region sequence having at least 90%, 95%, 96%, 97%, 98% or 99% sequence identity therewith, and the sequence shown in SEQ ID NO: 124 or a light chain variable region sequence having at least 90%, 95%, 96%, 97%, 98% or 99% sequence identity therewith;
(6) the sequence shown in SEQ ID NO: 125 or a heavy chain variable region sequence having at least 90%, 95%, 96%, 97%, 98% or 99% sequence identity therewith, and the sequence shown in SEQ ID NO: 126 or a light chain variable region sequence having at least 90%, 95%, 96%, 97%, 98% or 99% sequence identity therewith;
(7) the sequence shown in SEQ ID NO: 127 or a heavy chain variable region sequence having at least 90%, 95%, 96%, 97%, 98% or 99% sequence identity therewith, and the sequence shown in SEQ ID NO: 128 or a light chain variable region sequence having at least 90%, 95%, 96%, 97%, 98% or 99% sequence identity therewith;
(8) the sequence shown in SEQ ID NO: 129 or a heavy chain variable region sequence having at least 90%, 95%, 96%, 97%, 98% or 99% sequence identity therewith, and the sequence shown in SEQ ID NO: 130 or a light chain variable region sequence having at least 90%, 95%, 96%, 97%, 98% or 99% sequence identity therewith;
(9) the sequence shown in SEQ ID NO: 131 or a heavy chain variable region sequence having at least 90%, 95%, 96%, 97%, 98% or 99% sequence identity therewith, and the sequence shown in SEQ ID NO: 132 or a light chain variable region sequence having at least 90%, 95%, 96%, 97%, 98% or 99% sequence identity therewith;
(10) the sequence shown in SEQ ID NO: 133 or a heavy chain variable region sequence having at least 90%, 95%, 96%, 97%, 98% or 99% sequence identity therewith, and the sequence shown in SEQ ID NO: 134 or a light chain variable region sequence having at least 90%, 95%, 96%, 97%, 98% or 99% sequence identity therewith;
(11) the sequence shown in SEQ ID NO: 135 or a heavy chain variable region sequence having at least 90%, 95%, 96%, 97%, 98% or 99% sequence identity therewith, and the sequence shown in SEQ ID NO: 136 or a light chain variable region sequence having at least 90%, 95%, 96%, 97%, 98% or 99% sequence identity therewith;
(12) the sequence shown in SEQ ID NO: 137 or a heavy chain variable region sequence having at least 90%, 95%, 96%, 97%, 98% or 99% sequence identity therewith, and the sequence shown in SEQ ID NO: 138 or a light chain variable region sequence having at least 90%, 95%, 96%, 97%, 98% or 99% sequence identity therewith;
(13) the sequence shown in SEQ ID NO: 139 or a heavy chain variable region sequence having at least 90%, 95%, 96%, 97%, 98% or 99% sequence identity therewith, and the sequence shown in SEQ ID NO: 140 or a light chain variable region sequence having at least 90%, 95%, 96%, 97%, 98% or 99% sequence identity therewith;
(14) the sequence shown in SEQ ID NO: 141 or a heavy chain variable region sequence having at least 90%, 95%, 96%, 97%, 98% or 99% sequence identity therewith, and the sequence shown in SEQ ID NO: 142 or a light chain variable region sequence having at least 90%, 95%, 96%, 97%, 98% or 99% sequence identity therewith;
(15) the sequence shown in SEQ ID NO: 143 or a heavy chain variable region sequence having at least 90%, 95%, 96%, 97%, 98% or 99% sequence identity therewith, and the sequence shown in SEQ ID NO: 144 or a light chain variable region sequence having at least 90%, 95%, 96%, 97%, 98% or 99% sequence identity therewith;
(16) the sequence shown in SEQ ID NO: 145 or a heavy chain variable region sequence having at least 90%, 95%, 96%, 97%, 98% or 99% sequence identity therewith, and the sequence shown in SEQ ID NO: 146 or a light chain variable region sequence having at least 90%, 95%, 96%, 97%, 98% or 99% sequence identity therewith;
(17) the sequence shown in SEQ ID NO: 147 or a heavy chain variable region sequence having at least 90%, 95%, 96%, 97%, 98% or 99% sequence identity therewith, and the sequence shown in SEQ ID NO: 148 or a light chain variable region sequence having at least 90%, 95%, 96%, 97%, 98% or 99% sequence identity therewith;
(18) the sequence shown in SEQ ID NO: 149 or a heavy chain variable region sequence having at least 90%, 95%, 96%, 97%, 98% or 99% sequence identity therewith, and the sequence shown in SEQ ID NO: 150 or a light chain variable region sequence having at least 90%, 95%, 96%, 97%, 98% or 99% sequence identity therewith; and
(19) the sequence shown in SEQ ID NO: 151 or a heavy chain variable region sequence having at least 90%, 95%, 96%, 97%, 98% or 99% sequence identity therewith, and the sequence shown in SEQ ID NO: 152 or a light chain variable region sequence having at least 90%, 95%, 96%, 97%, 98% or 99% sequence identity therewith.

In some embodiments, the antibody binds to an EGFRvIII antigen with a KD value of less than 10⁻⁶ M and preferably less than 10⁻⁷ M, as determined by surface plasmon resonance (SPR).

In some embodiments, the antibody is selected from a murine antibody, a chimeric antibody, a humanized antibody, or a human antibody.

In some embodiments, the humanized antibody binds to an EGFRvIII antigen with a KD value of less than 10⁻⁶ M and preferably less than 10⁻⁷ M, as determined by surface plasmon resonance (SPR).

In some embodiments, the antigen-binding fragment is a single chain antibody; preferably, a linker for linking the heavy chain variable region with the light chain variable region of the single chain antibody comprises the sequence shown in SEQ ID NO: 166.

In some embodiments, the humanized antibody is a single chain antibody whose amino acid sequence is selected from any of the following:
(1) the sequence shown in SEQ ID NO: 167 or a heavy chain variable region sequence having at least 90%, 95%, 96%, 97%, 98% or 99% sequence identity therewith;
(2) the sequence shown in SEQ ID NO: 169 or a heavy chain variable region sequence having at least 90%, 95%, 96%, 97%, 98% or 99% sequence identity therewith;
(3) the sequence shown in SEQ ID NO: 171 or a heavy chain variable region sequence having at least 90%, 95%, 96%, 97%, 98% or 99% sequence identity therewith;
(4) the sequence shown in SEQ ID NO: 173 or a heavy chain variable region sequence having at least 90%, 95%, 96%, 97%, 98% or 99% sequence identity therewith;
(5) the sequence shown in SEQ ID NO: 175 or a heavy chain variable region sequence having at least 90%, 95%, 96%, 97%, 98% or 99% sequence identity therewith;
(6) the sequence shown in SEQ ID NO: 177 or a heavy chain variable region sequence having at least 90%, 95%, 96%, 97%, 98% or 99% sequence identity therewith;
(7) the sequence shown in SEQ ID NO: 179 or a heavy chain variable region sequence having at least 90%, 95%, 96%, 97%, 98% or 99% sequence identity therewith;
(8) the sequence shown in SEQ ID NO: 181 or a heavy chain variable region sequence having at least 90%, 95%, 96%, 97%, 98% or 99% sequence identity therewith;
(9) the sequence shown in SEQ ID NO: 183 or a heavy chain variable region sequence having at least 90%, 95%, 96%, 97%, 98% or 99% sequence identity therewith;
(10) the sequence shown in SEQ ID NO: 185 or a heavy chain variable region sequence having at least 90%, 95%, 96%, 97%, 98% or 99% sequence identity therewith; and
(11) the sequence shown in SEQ ID NO: 187 or a heavy chain variable region sequence having at least 90%, 95%, 96%, 97%, 98% or 99% sequence identity therewith.

In some embodiments, the CAR further comprises a hinge region, a transmembrane region, a co-stimulatory domain and an activation domain; preferably, the hinge region is a CD8 hinge region, the transmembrane region is a CD8α transmembrane region, the co-stimulatory domain is a 41BB co-stimulatory domain or a CD28 co-stimulatory domain, the activation domain is a CD3ζ activation domain.

In some embodiments, the hinge region comprises the sequence shown in SEQ ID NO: 156; the transmembrane region comprises the sequence shown in SEQ ID NO: 158; the co-stimulatory domain comprises the sequence shown in SEQ ID NO: 160 and/or SEQ ID NO: 162; the activation domain comprises the sequence shown in SEQ ID NO: 164.

In some embodiments, the CAR further comprises a signal peptide; preferably, the signal peptide is a CD8α signal peptide; and more preferably, the signal peptide comprises the sequence shown in SEQ ID NO: 154.

In some embodiments, the signal peptide comprises the sequence shown in SEQ ID NO: 154.

In another aspect, provided herein is a nucleic acid molecule encoding: 1) the above antibody or antigen-binding fragment thereof, or the heavy chain variable region and/or the light chain variable region of the above antibody or antigen-binding fragment thereof; or 2) the above CAR.

In some embodiments, the nucleic acid molecule comprises a nucleotide sequence shown in any one of SEQ ID NO: 153, 155, 157, 159, 161, 163, 165, 168, 170, 172, 174, 176, 178, 180, 182, 184, 186, or 188.

In another aspect, provided herein is an expression vector comprising the above nucleic acid molecule; preferably, the expression vector is a lentiviral vector.

In another aspect, provided herein is a cell comprising the above expression vector.

In another aspect, provided herein is a cell expressing the above CAR.

In some embodiments, the cell is an immune cell.

In some embodiments, the cell is a T cell or an NK cell.

In some embodiments, the cell is further engineered to express a dominant negative receptor (DNR) or a chimeric switch receptor (CSR); preferably, the DNR is dnTGFβRII and/or dnPD1; preferably, an intracellular domain of the CSR is from IL7Rα; more preferably, the dnTGFβRII comprises the sequence shown in SEQ ID NO: 193, and the intracellular domain of the CSR comprises the sequence shown in SEQ ID NO: 197.

In another aspect, provided herein is a pharmaceutical composition comprising: 1) the antibody or antigen-binding fragment thereof, the nucleic acid molecule, the expression vector or the cell as described above; and 2) a pharmaceutically acceptable carrier.

In another aspect, provided herein is use of the antibody or antigen-binding fragment thereof, the nucleic acid molecule, the expression vector or the cell as described above in the preparation of a medicament for treating a tumor.

In some embodiments, the tumor expresses EGFRvIII.

In some embodiments, the tumor is glioblastoma.

In another aspect, provided herein is a method for treating a tumor in a subject, comprising administering to the subject a therapeutically effective amount of the antibody or antigen-binding fragment thereof, the nucleic acid molecule, the expression vector, the cell or the pharmaceutical composition as described above.

In some embodiments, the tumor expresses EGFRvIII.

In some embodiments, the tumor is glioblastoma.

In another aspect, provided herein is a multispecific antibody molecule comprising at least a first functional portion and a second functional portion, wherein the first functional portion comprises the above antibody or antigen-binding fragment thereof, and the second functional portion has a different antigen-binding specificity from the first functional portion.

In some embodiments, the second functional portion has a binding specificity for a T cell.

In another aspect, provided herein is an immunoconjugate comprising the above antibody or antigen-binding fragment thereof which is linked with a therapeutic agent.

In some embodiments, the therapeutic agent is a drug.

In some embodiments, the therapeutic agent is a cytotoxin.

In some embodiments, the therapeutic agent is a radioactive isotope.

In another aspect, provided herein is a kit for determining the presence or content of EGFRvIII in a sample, wherein the kit comprises the above antibody or antigen-binding fragment thereof.

### Description of the Drawings

FIG 1 is a schematic diagram of the structure of EGFRvIII.
FIG 2 shows the results of the abilities of the positive subclone-based supernatants in binding to target cells as determined by flow cytometry.
FIG 3 shows the results of the affinities of some of the purified murine antibodies as determined by the SPR method.
FIG 4 is a schematic diagram of the structure of the CAR used herein.
FIG 5 shows the results of the CAR-positive rates of CAR T cells with murine antibody sequences.
FIG 6 shows the efficiencies of the CAR T cells with murine antibody sequences in killing the U87MG-EGFRvIII target cells.
FIG 7 shows the efficiencies of the CAR T cells with murine antibody sequences in killing the U87MG-EGFR target cells.
FIG 8 shows the *in vivo* tumor inhibition efficiencies of the CAR T cells with murine antibody sequences.
FIG 9 shows the results of the affinities of the purified humanized antibodies from clone #28, as determined by the SPR method.
FIG 10 shows the results of the affinities of the purified humanized antibodies from clone #40-2, as determined by the SPR method.
FIG 11 shows the CAR-positive rates of CAR T cells prepared with the humanized antibodies from clone #28.
FIG 12 shows the CAR-positive rates of CAR T cells prepared with the humanized antibodies from clone #40-2.
FIG 13 shows the efficiencies of the CAR T cells with the humanized antibodies from clone #28 in killing the U87MG EGFRvIII target cells.
FIG 14 shows the efficiencies of the CAR T cells with the humanized antibodies from clone #28 in killing the U87MG EGFR target cells.
FIG 15 shows the efficiencies of the CAR T cells with the humanized antibodies from clone #40-2 in killing the U87MG EGFRvIII target cells.
FIG 16 shows the efficiencies of the CAR T cells with the humanized antibodies from clone #40-2 in killing the U87MG EGFR target cells.
FIG 17 shows the *in vivo* tumor inhibition efficiencies of the CAR T cells with humanized antibodies from the clones.
FIG 18 shows the *in vivo* tumor inhibition efficiencies of the CAR T cells with humanized antibodies from the clones.
FIG 19 shows the efficiencies of the CAR NK cells prepared with the humanized antibodies from clone #28 in killing the target cells. (A) for the U87MG EGFRvIII target cells; and (B) for the U87MG EGFR target cells.
FIG 20 shows a schematic diagram of the CAR constructs with a DNR (A) or a CSR (B).
FIG 21 shows the determination results of the CAR-positive rates of the fourth-generation CAR T cells constructed according to the present application. (A) RII cells; (B) T7R cells; and (C) CAR T C cells.
FIG 22 shows the determination results of the killing efficiencies of the CAR T cells. (A) Killing efficiency without TGFβ treatment; (B) killing efficacy with TGFβ treatment; and (C) change of killing efficiency.
FIG 23 shows the study results of *in vivo* efficacies of the CAR T cells. In the figure, "CAR-T-C" refers to the CAR T cells in the control group; "RII" refers to the CAR T cells expressing the dominant negative receptor dnTGF-βRII; and "T7R" refers to the CAR T cells expressing the chimeric switch receptor dnTGF-βRII-IL7Rα.

### Detailed Description

Unless otherwise stated, all technical or scientific terms used herein have the meaning as commonly understood by one of ordinary skill in the art.

Antibody refers to an immunoglobulin secreted by a plasma cell (effector B cells) and used by the immune system of an organism to neutralize a foreign substance (such as a polypeptide, a virus, or bacteria). This foreign substance is correspondingly called an antigen. The basic structure of a typical antibody molecule is a tetramer consists of two identical heavy chains and two identical light chains. According to the difference in amino acid sequence conservation, the heavy chains and the light chains can be divided into variable regions (V) at the amino terminuses and constant regions (C) at the carboxyl terminuses. A variable region of a heavy chain and a variable region of a light chain can form an antigen-binding site (Fv) through interaction. In a variable region, there are amino acid residues in certain regions whose composition and order are more variable compared with other regions (the framework regions, FRs) in the variable region, called the hypervariable regions (HVRs). The hypervariable regions are actually key sites for antibody-antigen binding. As their sequences are complementary to the antigenic determinant(s), they are also called complementarity-determining regions (CDRs). Both the heavy chain and the light chain have three complementarity-determining regions called HCDR1, HCDR2, and HCDR3, and LCDR1, LCDR2, and LCDR3 respectively. Further, it is known in the art that various modifications can be made to antibody molecules, for example, PEGylation, glycosylation, formation of an antibody molecule conjugate (such as an ADC), addition of a tag for purification, or fusion with another protein such as formation of a bispecific antibody. The antibody derivatives obtained by modifying the antibody molecules provided in this disclosure should also be included within the scope of the present invention.

When the CDR sequences of an antibody are known, one skilled in the art can easily replace part of the antibody molecule sequence (such as a constant region or a framework region) with a sequence from other species to form a chimeric antibody, for example through the DNA recombination technology. This chimeric antibody can substantially retain the binding specificity of the original antibody. For example, when the original antibody is a murine antibody, its constant region can be replaced with the constant region of a human antibody, or vice versa; when the original antibody is a human antibody, its constant region can be replaced with that of a mouse antibody, in order to reduce the immunogenicity of the antibody when used in a different species or in order to employ a particular function of the constant region, such as an ADCC related activity. In order to further reduce the immunogenicity of an antibody or for other purposes, all sequences except the CDR sequences in an antibody molecule can be replaced with the corresponding sequences (which may include one or several amino acid mutations as appropriate) of another antibody molecule (from the same or a different species) while substantially retaining the binding specificity of the original antibody. In a specific example, the murine antibodies are often humanized through CDR grafting by one skilled in the art.

An "antigen-binding fragment" of an antibody molecule refers to the amino acid fragment in the antibody molecule that participates in specific antigen binding, for example, an Fab, an Fab' and an (Fab')₂, etc. One skilled in the art knows how to obtain these antigen-binding fragments. For example, a typical antibody molecule can be digested with papain to obtain an Fab fragment, and digested with pepsin to obtain an (Fab')₂, and an (Fab')₂ can be treated with a reducing agent to break the disulfide bonds between the hinge regions to form an Fab' fragment. A single chain antibody and a single domain antibody having an antigen-binding ability is a single peptide chain antibody molecule and can be regarded as an "antigen-binding fragment" of a typical antibody molecule.

An "Fc fragment" refers to the trunk of a "Y"-shaped antibody molecule, that is, the fragment crystallizable (Fc) which includes the second and third constant domains of a heavy chain (the CH2 and CH3 domains). The Fc region of an antibody can be obtained by hydrolyzing an antibody molecule with a proteolytic enzyme (such as papain). In some examples, the Fc region may comprise a hinge, a CH2, and a CH3. When the Fc region comprises a hinge, it can mediate dimerization between two Fc-containing polypeptides. The Fc fragment can be derived from IgG, IgM, IgD, IgE, or IgA. In some examples, the Fc region is derived from IgG1, IgG2, IgG3 or IgG4. The "Fc fragment" also comprises a variant Fc fragment derived from a natural Fc fragment which is modified but still retains its effector functions. A "variant Fc fragment" comprises an amino acid sequence that has at least one amino acid change in the amino acid sequence of a natural Fc fragment. In some examples, compared with the parent Fc fragment (the natural Fc fragment), the variant Fc fragment has at least one amino acid substitution, for example, about 1 to about 10 amino acids being substituted and preferably about 1 to about 5 amino acids being substituted in the parent Fc fragment. In some examples, the variant Fc fragment has at least about 80% sequence identity, at least about 90% sequence identity, at least about 95%, at least about 96%, at least about 97%, at least about 98%, or at least about 99% sequence identity with the parent Fc fragment. The effector functions of the "Fc fragment" can comprise binding to an Fc receptor, Clq binding, complement-dependent cytotoxicity (CDC), antibody-dependent cell-mediated cytotoxicity (ADCC), and mediating phagocytosis and the like.

A single chain antibody (single chain fragment variable, scFv) is composed of a heavy chain variable region and a light chain variable region of an antibody which are linked via a short peptide to form a single peptide chain. When folded correctly, the variable regions from the heavy chain and the light chain form an Fv segment through non-covalent interaction, and as a result, the scFv can retain its affinity for an antigen well.

A "single domain antibody (sdAb)", also known as "VHH antibody", refers to an antibody molecule which comprises a heavy chain variable region without a light chain and has an antigen-binding ability. Based on its structure, a single domain antibody can also be regarded as an antigen-binding fragment of an antibody molecule. It was first found in camelids, and subsequently, more single domain antibodies having an antigen-binding ability are found by researchers through screening of antibody libraries (such as phage display libraries). The single domain antibody has some advantages over an ordinary antibody molecule (for example, a typical tetrameric antibody molecule) or antigen-binding fragment thereof, including but not limited to: a smaller molecular weight, which allows for easy access to a tissue or a site that is difficult for an common antibody molecule to reach, or access to an epitope in a protein or a polypeptide which is difficult for a common antibody molecule to contact when used in human; being more stable, for example it can tolerate a change in temperature or pH and an effect of a denaturant and a protease.

A "bispecific antibody" refers to an antibody molecule which has two different binding sites and can recognize and bind to two different antigens respectively. For example, one binding site of the bispecific antibody can be used to bind an immune cell (such as a T cell) and the other binding site to a tumor cell, thereby enhancing the effect of an immune cell in killing a tumor cell while reducing side effects such as an off-target toxicity. Such a bifunctional antibody usually has a higher efficacy as a medicament for treating a tumor than a monoclonal antibody medicament. Similarly, an antibody molecule can be engineered to include multiple different binding sites to generate a "trispecific antibody", a "tetraspecific antibody" and the like. A "multispecific antibody" used herein covers these bispecific antibody, trispecific antibody, tetraspecific antibody and the like. Preferably, the multispecific antibody adopted is a bispecific antibody.

A "humanized antibody" herein refers to an antibody that is less immunogenic in human while retaining the reactivity of a non-human antibody. For example, it can be achieved by retaining the non-human CDR region while replacing the remainder of the antibody with the human counterpart (that is, the constant region and the framework region portion of the variable regions).

An "immunoconjugate" refers to an antibody targeting EGFRvIII or antigen-binding fragment thereof disclosed herein which is conjugated to a therapeutic agent. The therapeutic agent is, for example, a cytotoxin, a drug (such as an immunosuppressant) or a radio-toxin.

A "fusion protein" refers to an artificially produced (for example, through the genetic engineering technology) protein molecule consisting of at least two different peptide segments. These peptide segments do not exist in nature or in the same protein molecule. Common examples of fusion proteins which comprise an antibody fragment include an antibody-cytokine fusion protein, an antibody-cytotoxin fusion protein (also known as an immunotoxin), an enzyme-labeled antibody for an immunoassay, and a chimeric antigen receptor (CAR).

"Targeting" or "specifically binding" refers to that compared with other molecules present in the environment, a molecule (such as an antibody or antigen-binding fragment thereof) has a higher binding affinity for another molecule (such as a tumor cell surface antigen). "Targeting" or "specifically binding" does not exclude that the molecule may have binding affinities for more than one molecule, for example, a bispecific antibody may have high affinities for two different antigens. The modifier "anti" preceding an antigen indicates that the subsequent antibody "targets" or "specifically binds" to the antigen. For example, an anti-EGFRvIII antibody indicates that the antibody "targets" or "specifically binds to" EGFRvIII. Preferably, the anti-EGFRvIII antibody does not "target" or "specifically bind to" a normal EGFR molecule. An antibody "specifically binds to an antigen" or the "antigen-binding specificity" of an antibody refers to that the antibody can bind to a target antigen with a higher or relatively high binding affinity as compared with other antigens. The binding ability of an antibody to a target antigen can be qualitatively or quantitatively determined by various methods, for example, by measuring the K_{D} value of an antibody binding to a target antigen through the surface plasmon resonance (SPR), or by measuring the ability of an antibody competing with other antibodies for binding to a target antigen. Here, Kd is the equilibrium dissociation constant, which can be used to measure the binding affinity of an antibody to its antigen. A smaller K_{D} value indicates a stronger affinity. An antibody specifically binding to a target antigen does not mean that it cannot bind to another antigen, for example, the cross reaction in immunology is also known in the art.

A "binding complex" refers to a complex which is formed between and comprises a molecule and the target to which it binds. Examples of a binding complex may include an antigen-antibody complex, a ligand-receptor complex, and a protein dimer, etc. The forces involved in the formation of a binding complex mainly include non-covalent forces such as hydrogen bonds, van der Waals forces, and ionic bonds. By detecting a complex formed by an antigen and an antibody, the presence or the content or concentration of an antigen or antibody of interest in a sample can be determined.

A "peptide segment" refers to a polypeptide fragment which is a short amino acid sequence having a length of for example about 2 to 20 amino acids, and is usually part of a polypeptide or protein.

EC50 (concentration for 50% of maximal effect) refers to the concentration that induces 50% of the maximal effect. For example, when used to represent the binding ability of an antibody molecule to a corresponding antigen on a cell in a FACS assay, it can refer to the concentration of the antibody molecule at which half of the maximal fluorescence intensity is produced. A lower EC50 value indicates a greater binding affinity for an antigen on a cell.

A "tag for purification" refers to an amino acid sequence expressed together with a protein or polypeptide of interest in the form of a fusion protein and used for purifying the protein or polypeptide of interest, including but not limited to the His6 tag, the Flag tag, the MBP (maltose binding protein) tag, the GST (glutathione S-transferase) tag, and the SUMO (small ubiquitin related modifier), etc. These tags can be removed through enzymatic cleavage after purification, or they (for example, the His6 tag) can be retained if they do not affect the normal functions of the protein or polypeptide of interest in use.

A "detectable tag" refers to an amino acid sequence or a chemical group linked to a protein or a polypeptide which is used to indicate the presence or content of the protein or the polypeptide in a sample, or to track the location of the protein or the polypeptide in a subject or a cell. Examples of a detectable tag include various enzymes that can be used in an immunoassay, such as horseradish peroxidase (HRP) and alkaline phosphatase (ALP); fluorescent groups (such as FAM or FITC) or fluorescent proteins (such as GFP); and radioactive isotopes (such as ³H, ¹⁴C, or ³⁵S). When the detectable tag is an enzyme, the presence or content of a protein or polypeptide linked to the enzyme can be determined by the enzymatic activity of the enzyme.

The terms "polypeptide" and "protein" are used interchangeably and refer to a polymer of amino acid residues. Such polymers of amino acid residues may comprise natural or non-natural amino acid residues, and include but are not limited to peptides, oligopeptides, dimers, trimers and multimers consisting of amino acid residues. Full-length proteins and fragments thereof are covered by the definition. The terms also include post-expression modifications of a polypeptide, such as glycosylation, sialylation, acetylation, phosphorylation and similar modifications. In addition, for the purposes of the present invention, "polypeptide" refers to a protein that includes a modification to a natural sequence, such as deletion, addition and substitution (which are generally conservative in practice), so long as the protein retains the desired activity. These modifications may be intentional, such as those induced via site-directed mutagenesis; or accidental, such as those resulted from mutations of the host producing the protein or errors due to PCR amplification.

When referring to an antibody or antigen-binding fragment thereof, a "variant" or "functional variant" may be used. Compared with their parent sequences (the antibodies or the antigen-binding fragments thereof), these variants or functional variants may include insertion, deletion or substitution of one or more (1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more) amino acid residues while retaining at least 50%, 60%, 70%, 80%, or 90% of the binding affinities of their parent sequence(s) for the corresponding antigen(s) (such as EGFRvIII), or having an affinity even higher than that of their parent sequence(s). One skilled in the art knows that VH or VL framework sequence can be varied considerably, as long as they can maintain the spatial positions of the corresponding CDR sequences. For example, a framework region sequence in a VH amino acid sequence can be changed, for example, by interchanging with a framework region of an antibody molecule from a different species to obtain a functional variant that substantially retains the antigen-binding ability. In addition, it is also possible to change a minority of residues (such as 1, 2 or 3 amino acid residues) in a CDR sequence and then determine the ability of the modified antibody molecule in binding to a corresponding antigen, to obtain a functional variant which still has an antigen-binding ability.

The terms "nucleic acid molecule", "nucleic acid", and "polynucleotide" herein are used interchangeably to refer to a polymer of nucleotides. Such a polymer of nucleotides may contain natural and/or non-natural nucleotides and include but are not limited to a DNA, an RNA and a PNA. A "nucleic acid sequence" refers to a linear sequence of nucleotides contained in a nucleic acid molecule or a polynucleotide.

The term "vector" refers to a nucleic acid molecule that can be engineered to contain a polynucleotide of interest (for example, a coding sequence for a polypeptide of interest) or a nucleic acid molecule that can replicate in a host cell (for example, a nucleic acid, a plasmid, or a virus). A vector may comprise one or more of the following components: an origin of replication, one or more regulatory sequences (such as a promoter and/or an enhancer) that regulate the expression of a polynucleotide of interest, and/or one or more selectable marker genes (for example, an antibiotic resistance gene, and a gene useful in a colorimetric assay, such as a β-galactose gene). The term "expression vector" refers to a vector used to express a polypeptide of interest in a host cell.

A "host cell" refers to a cell that can be or has been the recipient of a vector or an isolated polynucleotide. A host cell can be a prokaryotic cell or a eukaryotic cell. Exemplary eukaryotic cells include mammalian cells, such as primate or non-primate cells; fungal cells, such as yeasts; plant cells; and insect cells. Non-limiting exemplary mammalian cells include, but are not limited to, NSO cells, 293 and CHO cells, and derived cells thereof, such as 293-6E, CHO-DG44, CHO-K1, CHO-S and CHO-DS cells. A host cell includes the progeny of a single host cell, and the progeny is not necessarily identical to its original parent cell (in terms of morphology or genomic DNA complementarity) due to a natural, accidental, or intentional mutation. A host cell may also include a cell transfected with a nucleic acid molecule or an expression vector provided herein in a living organism.

"Treatment" refers to treating a subject to obtain a beneficial or desired clinical outcome. "Treatment" used herein encompasses various treating means, including administration of any possible medicament to a subject, surgery and radiation, etc. For purposes of the present invention, the beneficial or desired clinical outcome includes but is not limited to any one or more of the following: alleviation of one or more symptoms, attenuation of disease severity, prevention or delay of spread of a disease (for example, metastasis, e.g. metastasis to the lung or the lymph node) or disease recurrence, delay or slowing of disease progression, improvement of a diseased condition, inhibition of a disease or its progression, blockage of disease development, and remission (either partial or complete remission). The methods provided herein encompass any one or more of these aspects of treatment. Based on the above description, "treatment" does not require complete elimination of all symptoms of a condition or disease or complete remission thereof.

The term "therapeutically effective amount" refers to an amount of an active compound sufficient to induce a biological or medical response expected by a clinician in a subject. The "therapeutically effective amount" of the fusion protein of the present invention can be determined by one skilled in the art based on the route of administration, the subject's weight, age or condition, and other factors. For example, a typical daily dosage may range from 0.01 mg to 100 mg or more of an active ingredient per kg of body weight.

When referring to a pharmaceutical composition, the term "pharmaceutically acceptable carrier" as used refers to a solid or liquid diluent, filler, antioxidant, stabilizer and the like that can be administered safely and is suitable for administering to a human and/or an animal without an undue adverse side effect while also suitable for maintaining the activity of the pharmaceutical or the active agent therein. Depending on the route of administration, various carriers well known in the art may be administered, including but not limited to sugars, starch, cellulose and derivatives thereof, maltose, gelatin, talc, calcium sulfate, vegetable oils, synthetic oils, polyols, alginic acid, phosphate buffer, emulsifier, isotonic saline, and/or pyrogen-free water. The pharmaceutical compositions provided herein can be prepared into clinically acceptable dosage forms such as a powder and an injection. The pharmaceutical compositions of the present invention can be administered to a subject by any appropriate route, for example, administered by oral administration, intravenous infusion, intramuscular injection, subcutaneous injection, subperitoneal, rectal, sublingual administration, or inhalation, transdermal administration, etc.

A "subject" refers to an animal such as a mammal including but not limited to a human, a rodent, a simian, a feline, a canine, an equid, a bovid, a swine, a sheep, a goat, an experimental mammal, a farm mammal, a sporting mammal and a pet mammal. A subject may be male or female of any appropriate age, including infant, child, young adult, adult, and elderly subjects. In some examples, a subject refers to an individual in need of treatment of a disease or a condition. In some examples, a subject receiving a treatment can be a patient who has a condition with which the treatment is associated, or is at risk of developing the condition. In a particular example, the subject is a human, such as a human patient. The term is often used interchangeably with "patient", "subject to be tested", "subject to be treated" or the like.

A "biological sample" means a certain amount of substance derived from a living or previously living organism. The substance includes but is not limited to blood (for example, whole blood), plasma, serum, urine, amniotic fluid, synovial fluid, an endothelial cell, a leukocyte, a monocyte, or other cells, an organ, a tissue, a bone marrow, a lymph node, and a spleen.

When referring to an amino acid or nucleotide sequence, the term "sequence identity" refers to a measure of the degree of the identity between two amino acid or nucleotide sequences (such as a query sequence and a reference sequence) which is usually expressed in percentage. Generally, sequence alignment is performed and a gap (if any) is introduced before calculating the percent identity between two amino acid or nucleotide sequences. If the amino acid residues or bases in the two sequences are the same at a certain aligned position, the two sequences are considered to be identical or matched at that position; if not, they are considered to be not identical or mismatched at that position. In some algorithms, the number of matching positions is divided by the total number of positions in the alignment window to obtain the sequence identity. In other algorithms, the number of gaps and/or the gap length is also taken into account. For the purposes of the present invention, the publicly available alignment software BLAST (which can be found on the website ncbi.nlm.nih.gov) can be used to obtain optimal sequence alignment and calculate the sequence identity between two amino acid or nucleotide sequences by using default settings.

Chimeric antigen receptor (CAR), also known as chimeric T cell receptor or chimeric immune receptor, is an engineered protein receptor molecule that can confer a desired specificity on an immune effector cell, for example, an ability to bind to a specific tumor antigen. The chimeric antigen receptor usually consists of an extracellular antigen-binding domain, a transmembrane domain, and an intracellular signaling domain. In most cases, the antigen-binding domain is an scFv sequence segment which is responsible for recognizing and binding to a specific antigen. The intracellular signaling domain usually includes an immunoreceptor tyrosine-based activation motif (ITAM), such as the signaling domain derived from the CD3ζ molecule, which is responsible for activating immune effector cells to produce a killing effect. In addition, the chimeric antigen receptor may also include a signal peptide at the amino terminus which is responsible for the intracellular localization of a nascent protein, and a hinge region between the antigen-binding domain and the transmembrane domain. The intracellular signaling domain may include, in addition to a signaling domain, a co-stimulatory domain derived from for example a 4-1BB molecule.

A "CAR-T cell" refers to a T cell expressing a CAR, which are usually obtained by transduction of a T cell with an expression vector encoding a CAR. A commonly used expression vector is a viral vector, such as a lentiviral expression vector. A chimeric antigen receptor-modified T cell (CAR-T) is not restricted by the major histocompatibility complex. It has a specific and targeted killing activity and an ability of persistent expansion. In addition to the T cell, other lymphocytes such as the NK cell can also be transformed with an expression vector encoding a CAR to obtain a targeting killer cell expressing the CAR.

A "dominant negative receptor (DNR)" refers to a receptor that can bind to its ligand but does not induce a signaling cascade within a cell. A DNR usually has a complete ligand-binding region but lacks a region for an intracellular enzymatic activity (such as a kinase activity). It can be a mutated form of a full-length receptor or a truncated form of the receptor. After a CAR T cell immunotherapy, some cancers, especially solid cancers, may upregulate the inhibitory ligands that bind to the inhibitory receptors on CAR T cells. This adaptive resistance compromises the efficacy of the CAR T cell therapy. Some cancers, especially solid cancers, are known to secrete transforming growth factor beta (TGF-β), thereby creating an immunosuppressive environment. TGF-β can induce or promote metastasis and effectively suppress the immune system. Therefore, in some embodiments, we use a truncated form of the TGF-β receptor TGFBRII as a TGF-β DNR to improve the anti-tumor performance of the CAR T cells disclosed herein. In some embodiments, the CAR and the TGF-β DNR are co-expressed on the surface of a T cell by using a 2A self-cleaving peptide. In some embodiments, the CAR and the TGF-β DNR are respectively expressed on the surface of a T cell by using two expression vectors. We find that when the TGF-β DNR is introduced into the CAR T cells disclosed herein, the cytotoxicity of these cells to some EGFRvIII-positive cancer cells can be enhanced. Similarly, in some embodiments, we use a truncated form of the PD-1 receptor as a PD-1 DNR to improve the anti-tumor performance of the CAR T cells disclosed herein.

A "chimeric switch receptor (CSR)" refers to a receptor that is created to reverse the results of an original signaling pathway, thereby conferring a desired activity on an immune cell (such as a CAR T cell), for example, an ability to overcome the immunosuppressive tumor microenvironment and allow the immune cell to have greater *in vivo* persistence. In some embodiments, a CSR can utilize an inhibitory molecule expressed by a cancer cell to further stimulate a CAR T cell. In a non-limiting example, a CAR T cell can be engineered to express a CSR formed by fusion of the extracellular ligand-binding domain of PD-1 and the transmembrane and intracellular co-stimulatory signaling domains of CD28. When this CAR T cell is administered to a subject with a cancer that expresses EGFRvIII and PD-L1, the expressed CAR can bind to EGFRvIII, while the simultaneously expressed CSR can bind to PD-L1. The properties of the PD-1/CD28 chimeric switch receptor fusion protein prevent the normal PD1/PD-L1-mediated T cell inhibition and instead promotes signaling through the CD28 domains, thereby stimulating the CAR T cell. Thus, switching the transmembrane and intracellular domains of PD-1 with those of CD28 turns PD-L1 into a co-stimulatory ligand for the CAR T cell. This will induce enhanced toxicity to a cancer cell expressing PD-L1. In another non-limiting example, the transmembrane and intracellular domains of TGFBRII can be switched with those of the IL7Rα receptor, thereby switching the inhibitory signal from TGF-β into a stimulating signal for the CAR T cell and promote the proliferation or activity of the CAR T cell in a tumor microenvironment.

### Anti-EGFRvIII antibody and antigen-binding fragment thereof

Provided herein is an anti-EGFRvIII antibody that specifically binds to EGFRvIII. The anti-EGFRvIII antibody binds to an EGFRvIII antigen with a relatively high binding affinity. As set forth in the Examples below, the ability of the anti-EGFRvIII antibody to bind to EGFRvIII can be measured by a method such as the surface plasmon resonance (SPR). In addition, it can also be determined by other methods known in the art that measure protein interactions, such as bio-layer interferometry (BLI) technology, enzyme-linked immunosorbent assay (ELISA) and flow cytometry-based fluorescent-activated cell sorting (FACS).

Provided herein are CDR sequences of multiple anti-EGFRvIII antibodies, for example, their heavy chain CDR sequences are sequences shown in SEQ ID NOs: 1-3, 4-6, 7-9, 10-12, 13-15, 16-18, 19-21, 22-24, 25-27, 28-30, 31-33, 34-36, 37-39, 40-42, 43-45, 46-48, 49-51, 52-54 or 55-57 respectively. Preferably, the corresponding light chain CDR sequences are sequences shown in SEQ ID NOs: 58-60, 61-63, 64-66, 67-69, 70-72, 73-75, 76-78, 79-81, 82-84, 85-87, 88-90, 91-93, 94-96, 97-99, 100-102, 103-105, 106-108, 109-111 or 112-114 respectively.

In some embodiments, the heavy chain variable region of the anti-EGFRvIII antibody provided by the present invention comprises an amino acid sequence having at least 90% sequence identity (for example, at least 95%, at least 98%, at least 99% or even 100% sequence identity) with the sequence shown in SEQ ID NO: 115, 117, 119, 121, 123, 125, 127, 129, 131, 133, 135, 137, 139, 141, 143, 145, 147, 149 or 151. Preferably, the corresponding light chain variable region comprises an amino acid sequence having at least 90% sequence identity (for example, at least 95%, at least 98%, at least 99% or even 100% sequence identity) with the sequence shown in SEQ ID NO: 116, 118, 120, 122, 124, 126, 128, 130, 132, 134, 136, 138, 140, 142, 144, 146, 148, 150 or 152.

Based on the CDR sequences of the anti-EGFRvIII antibodies provided herein, one skilled in the art can construct various polypeptide constructs having an ability to bind to EGFRvIII, including combining framework regions (FRs) from different antibody molecules with these CDR sequences. These framework regions comprise natural framework region sequences from human antibodies or animal (for example, mouse, rat, sheep, or camel) antibodies. These framework regions may also include framework region sequence variants resulted from modifications to the natural framework region sequences. Polypeptide constructs that specifically bind to EGFRvIII can be easily obtained by combining the CDR sequences provided herein with different framework region sequences to form heavy chain variable regions, and then determining their abilities of binding to EGFRvIII. Preferably, humanized antibodies, such as humanized single chain antibodies (scFVs), can be constructed based on these CDR sequences.

In some embodiments, the humanized single chain antibody (scFV) provided by the present invention comprises an amino acid sequence having at least 90% sequence identity (for example, at least 95%, at least 98%, at least 99% or even 100% sequence identity) with the sequence shown in SEQ ID NO: 169, 171, 173, 175, 177, 179, 181, 183, 185 or 187.

It is expected that the anti-EGFRvIII antibody or antigen-binding fragment thereof described herein may comprise a conservative amino acid substitution. The conservative amino acid substitution can be generally described as substitution of an amino acid residue with another amino acid residue of a similar chemical structure which has little or essentially no effect on the function, activity, or other biological properties of the polypeptide. The conservative amino acid substitution is well known in the art. For example, the conservative substitution can be a substitution of one amino acid in any of the following groups (a)-(e) with another amino acid in the same group: (a) small aliphatic non-polar or weakly polar residues: Ala, Ser, Thr, Pro and Gly; (b) polar, negatively charged residues and their (uncharged) amides: Asp, Asn, Glu and Gln; (c) polar, positively charged residues: His, Arg and Lys; (d) large aliphatic non-polar residues: Met, Leu, Ile, Val and Cys; and (e) aromatic residues: Phe, Tyr and Trp. Therefore, one skilled in the art can understand that based on the specific sequences provided herein, a few amino acids can be substituted, deleted, or added, followed by verifying or screening the resulted products for their abilities or biological activities to bind to the corresponding antigen EGFRvIII, to obtain corresponding variants of the antibody molecules targeting EGFRvIII provided herein. These variants should also be included within the scope of the present invention. For example, the antibody molecules or the single chain antibodies provided herein may have at least 1 and no more than 10, such as no more than 5, 4, 3, 2 or 1 amino acid alterations in their full-length, variable region, or CDR sequences.

In some embodiments related to the use of the anti-EGFRvIII antibody, an antigen-binding fragment of the anti-EGFRvIII antibody is used to bind to the corresponding antigen (EGFRvIII). Preferably, the antigen-binding fragment is a single chain antibody (scFv). For example, when the antibody is used in a CAR, one or more single chain antibodies (scFv) constituted therefrom can be used as an extracellular antigen-binding domain of the CAR.

### Fusion protein comprising the anti-EGFRvIII antibody or antigen-binding fragment thereof

Th anti-EGFRvIII antibody or antigen-binding fragment thereof can further form a fusion protein with other polypeptide(s).

In some embodiments, an antigen-binding fragment having an ability to bind to EGFRvIII can be linked to an Fc fragment (modified or derived from a different species or genus) to form a fusion protein, *i.e.* a typical antibody molecule form. The Fc fragment can be located at the C-terminus or the N-terminus of the antigen-binding fragment of the anti-EGFRvIII antibody. Preferably, the Fc fragment can be located at the C-terminus of the antigen-binding fragment of the anti-EGFRvIII antibody. The fusion protein formed by the antigen-binding fragment of the EGFRvIII antibody and the Fc fragment has the ability to specifically bind to EGFRvIII and also the effector functions of the Fc fragment such as mediating a complement-dependent cytotoxicity (CDC), an antibody-dependent cell-mediated cytotoxicity (ADCC), or phagocytosis. In addition, fusion with an Fc fragment can increase the *in vivo* half-life of the antigen-binding fragment of the anti-EGFRvIII antibody, so as to increase the dosing interval when it is used as a therapeutic medicament.

In some embodiments, the anti-EGFRvIII antibody or antigen-binding fragment thereof can be linked to a protein tag to form a fusion protein. A protein tag may comprise a tag for purification or a detectable tag. A tag for purification includes but is not limited to the His6 tag, the Flag tag, the MBP tag, the GST tag, and the SUMO tag. A detectable tag can be used to indicate the presence or content of the anti-EGFRvIII antibody or antigen-binding fragment thereof in a sample, or to track its location in a subject or a cell. Examples of detectable tags include various enzymes useful in an immunoassay, such as horseradish peroxidase (HRP) or alkaline phosphatase (ALP); and fluorescent proteins such as GFP. Since the anti-EGFRvIII antibody or antigen-binding fragment thereof has an ability of specifically binding to EGFRvIII, the presence or content of EGFRvIII in a sample can be determined by obtaining the amount of the anti-EGFRvIII antibody or antigen-binding fragment thereof via the amount of the detectable tag linked thereto.

In other embodiments, the anti-EGFRvIII antibody or antigen-binding fragment thereof can be linked to a cytokine or a therapeutic protein to form a fusion protein. In this case, with the ability of specifically binding to EGFRvIII, the anti-EGFRvIII antibody or antigen-binding fragment thereof can be used to purposefully deliver a cytokine or a therapeutic protein to a specific tissue or cell (such as a tumor tissue expressing EGFRvIII) to achieve the therapeutic effect of the cytokine or the therapeutic protein.

### Multispecific antibody comprising the anti-EGFRvIII antibody or antigen-binding fragment thereof

In some embodiments, provided herein is a multispecific antibody molecule comprising at least one EGFRvIII-binding domain (or functional unit) and one or more additional binding domains. The one or more additional binding domains can bind to a second antigen or protein other than EGFRvIII.

In some embodiments, provided herein is a multispecific antibody molecule comprising at least two EGFRvIII-binding domains. The two EGFRvIII-binding domains are different in their amino acid sequences, and preferably bind to different epitopes on EGFRvIII respectively.

In some embodiments, the EGFRvIII-binding domain is in the form of a single chain antibody; the one or more additional binding domains can be a single domain antibody, a single chain antibody or other antigen-binding fragment(s).

In some embodiments, the second antigen is a tumor associated antigen (TAA) or a tumor microenvironment associated antigen (TMEAA). In some embodiments, the second antigen is an immunomodulatory antigen, wherein the antigen is associated with the enhancement or inhibition of a signaling pathway in an immune cell. In some embodiments, the second antigen is a molecule on the surface of a T cell, such as a component of a T cell receptor complex, such as CD3 (including the γ, δ, ε, ζ, and η chains).

Preferably, the multispecific binding peptide is a bispecific antibody molecule.

In some embodiments, the multispecific binding peptide further comprises an Fc fragment. The presence of the Fc fragment can facilitate multimerization of the binding domains and may provide relevant effector functions.

### Immunoconjugate (or antibody conjugate) comprising the anti-EGFRvIII antibody or antigen-binding fragment thereof

Provided herein is a conjugate comprising at least one anti-EGFRvIII antibody or antigen-binding fragment thereof that specifically binds to EGFRvIII provided herein and one or more of other functional moieties. The other moieties may be a chemical group, for example a therapeutic agent such as a cytotoxic agent, or may be a tracer. In some examples, the moiety can be a targeting moiety, a small molecule drug (for example, a non-polypeptide drug of less than 500 Da), a toxin, a cytostatic agent, a cytotoxic agent, an immunosuppressant, a radioactive agent suitable for diagnostic purposes, or a radioactive metal ion for therapeutic purposes, or the like.

In some embodiments, the immunoconjugate is an antibody-drug conjugate (ADC) comprising one or more anti-EGFRvIII antibodies or antigen-binding fragments thereof provided herein and a therapeutic agent, which is cytotoxic, inhibits cell growth, or provides some therapeutic benefits. In some embodiments, the cytotoxic agent is a chemotherapeutic agent, a drug, a growth inhibitor, a toxin (for example, an enzymatically active toxin derived from a bacterium, a fungus, a plant or an animal, or a fragment thereof), or a radioactive isotope (namely, corresponding to a radioconjugate). In some examples, the antibody-drug conjugate provided by the present invention allows for targeted delivery of the drug moiety to a tumor. In some cases, this can result in targeted killing of tumor cells.

In some examples, the therapeutic agent includes, for example, daunomycin, doxorubicin, methotrexate, vindesine, or maytansinoid. In some examples, the therapeutic agent has an intracellular activity. In some examples, the immunoconjugate comprising the anti-EGFRvIII antibody or antigen-binding fragment thereof can be internalized by a cell, and the therapeutic agent has an activity of blocking synthesis of a cellular protein or a nucleic acid, thereby causing cell growth arrest or death.

In some embodiments, the immunoconjugate comprises one or more anti-EGFRvIII antibodies or antigen-binding fragments thereof provided herein and a tracer. This immunoconjugate can be used for research or diagnostic purposes, for example, for detecting a cancer in a living organism. The tracer can produce a detectable signal directly or indirectly. For example, the tracer may be a radioactive isotope, such as ³H, ¹⁴C, ³²P, ³⁵S, or ¹²³I, a fluorescent (fluorophore) or chemiluminescent (chromophore) compound, such as a fluorescent isothiocyanate, rhodamine, or fluorescein; a developer; or a metal ion. In some embodiments, the tracer is a radioactive atom for scintillation imaging studies such as ⁹⁹Tc or ¹²³I, or a spin label for nuclear magnetic resonance (NMR) imaging (also known as magnetic resonance imaging, MRI), such as ⁸⁹Zr, ¹²³I, ¹⁹F, ¹³C, ¹⁵N, ¹⁷O, or ⁸⁹Zr which can be complexed with various metal chelators and bound to antibodies, for example, for PET imaging.

The linkage between the anti-EGFRvIII antibody or antigen-binding fragment thereof and another functional moiety can be a covalent or non-covalent linkage. Examples of the non-covalent linkage may comprise the one through a biotin-avidin system. Examples of the covalent linkage may comprise various chemical linkers including a peptide linker, a cleavable linker, or a non-cleavable linker. Exemplary linker components include 6-maleimidocaproyl ("MC"), maleimidopropionyl ("MP"), valine-citrulline ("val-cit"), alanine-phenylalanine ("ala-phe"), p-aminobenzyloxycarbonyl ("PAB"), N-succinimidyl 4-(2-pyridylthio)pentanoate ("SPP"), N-succinimidyl 4-(N-maleimidomethyl)cyclohexane-1-carboxylate ("SMCC") and N-succinimidyl(4-iodo-acetyl)aminobenzoate ("SIAB").

In some embodiments, the linker may comprise an amino acid residue. Exemplary amino acid linker components include dipeptides, tripeptides, tetrapeptides, or pentapeptides. Exemplary dipeptides include valine-citrulline (vc or val-cit), or alanine-phenylalanine (afa-phe). Exemplary tripeptides include glycine-valine-citrulline (gly-val-cit) and glycine-glycine-glycine (gly-gly-gly). The amino acid residues comprised in the amino acid linker components include naturally occurring residues as well as non-naturally occurring amino acid analogues such as citrulline. The amino acid linker components can be designed and optimized in terms of selectivity, to facilitate enzymatic cleavage by a specific enzyme such as a tumor-associated protease, cathepsins B, C and D, or a cytoplasmic protease.

The conjugate of the anti-EGFRvIII antibody or antigen-binding fragment thereof and a cytotoxic agent can be prepared by using a variety of bifunctional protein conjugating agents, such as N-succinimidyl-3-(2-pyridyldithiol)propionate (SPDP), imidothiolanes (IT), bifunctional derivatives of imide esters (such as dimethyl adipimidate HCl), active esters (such as disuccinimidyl matrix), aldehydes (such as glutaraldehyde), bisazido compounds (such as bis(p-azidobenzoyl)hexanediamine), bisdiazo derivatives (such as bis-(p-diazobenzoyl)-ethylenediamine), diisocyanates (such as toluene 2,6-diisocyanate) and bi-active fluorine compounds (such as 1,5-difluoro-2,4-dinitrobenzene).

### Chimeric antigen receptor (CAR) comprising the anti-EGFRvIII antibody or antigen-binding fragment thereof

The chimeric antigen receptor (CAR) provided herein is an artificially constructed chimeric protein comprising an extracellular antigen-binding domain that specifically binds to EGFRvIII, a transmembrane domain, and one or more intracellular signaling domains. The characteristics of these CARs include their abilities to redirect T cell specificity and responsiveness to EGFRvIII-expressing cells in a non-MHC-restricted manner. The non-MHC-restricted EGFRvIII recognition enables T cells or NK cells expressing the disclosed CARs to recognize antigens independent of antigen processing and kill the EGFRvIII-expressing cells (such as tumor cells).

The intracellular signaling domain may comprise a signaling domain of a T cell receptor, a T cell co-stimulatory signaling domain, or both. The signaling domain of a T cell receptor may comprise the intracellular domain of a T cell receptor, such as the intracellular portion of the CD3 ζ protein. The co-stimulatory signaling domain is an intracellular domain of a co-stimulatory molecule. The co-stimulatory molecule is a cell surface molecule other than the antigen receptor or its ligand, which is required for an effective lymphocyte response to an antigen.

In some embodiments, the CAR provided herein comprises an extracellular antigen-binding domain that specifically binds to EGFRvIII. For example, the antigen-binding domain may be an scFv, which may be the heavy chain and light chain variable regions of any antibody or antigen-binding fragment thereof linked by a peptide linker and which specifically binds to EGFRvIII. As another example, for constructing a CAR using a single domain antibody that specifically binds to EGFRvIII, the extracellular antigen-binding domain may comprise the VHH from the single domain antibody without any light chain variable region.

In some embodiments, the extracellular antigen-binding domain of the CAR provided herein comprises one or more antigen-binding fragments from the anti-EGFRvIII antibody provided herein, such as an non-humanized or humanized single chain antibody (scFv).

When the extracellular antigen-binding domain comprises two or more antigen-binding fragments, they may be connected in tandem directly or via a peptide linker. Using two or more antigen-binding fragments connected in tandem as the extracellular antigen-binding domain or part thereof facilitates the constructed CAR in recognizing and binding to a target molecule such as EGFRvIII. Preferably, these antigen-binding fragments respectively bind to different epitopes on EGFRvIII.

The CAR may comprise a signal peptide sequence, for example, located N-terminal to the antigen-binding domain. Any suitable signal peptide sequence can be used. In an embodiment, the signal peptide sequence is a CD8α signal peptide. Although the signal peptide sequence can promote the expression of the CAR on a cell surface, the presence of the signal peptide sequence in the expressed CAR is not necessary for the functions of the CAR. When the CAR is expressed on a cell surface, the signal peptide sequence may be cleaved from the CAR. Thus, in some embodiments, the CAR lacks a signal peptide sequence.

Between the antigen-binding domain and the transmembrane domain of the CAR, there may be a hinge region (or a spacer domain) which comprises a polypeptide sequence. The hinge region may comprise up to 300 amino acids, preferably 10 to 100 amino acids. In an embodiment, the CAR provided herein comprises a hinge region from a CD8 α protein.

The CAR may comprise a transmembrane domain fused to the extracellular domain of the CAR. In an embodiment, the transmembrane domain that is naturally associated with another element in the CAR (such as a cross-linking region or an intracellular signaling domain) is used.

The transmembrane domain can be derived from a natural or synthetic source. When the source is natural, the domain may be derived from any membrane-binding or transmembrane protein. Exemplary transmembrane domains for use in the disclosed CAR may include at least the α, β, or ζ chain of a T cell receptor, or a transmembrane domain of CD28, CD3ε, CD45, CD4, CD5, CDS, CD9, CD16, CD22, CD33, CD37, CD64, CD80, CD86, CD134, CD137, or CD154. Alternatively, the transmembrane domain can be synthetic. In this case, it will primarily comprise hydrophobic residues such as leucine and valine. In some embodiments, a triplet of phenylalanine, tryptophan, and valine will be present at each end of the synthetic transmembrane domain.

Optionally, a short oligopeptide or polypeptide linker having a length of preferably 2 to 10 amino acids may form a linkage between the transmembrane domain and the T cell intracellular signaling domain and/or the T cell co-stimulatory domain of the CAR. Exemplary linker sequences include one or more glycine-serine doublets.

In some embodiments, the transmembrane domain comprises the transmembrane domain of a T cell receptor, such as the CD8 α transmembrane domain.

The intracellular region of the CAR comprises one or more T cell intracellular signaling domains, which is responsible for activating at least one normal effector function of the CAR-expressing T cell. Exemplary T cell signaling domains are provided herein and are known to one of ordinary skill in the art.

Although the entire T cell intracellular signaling domain can be used in a CAR, in many cases, it is not necessary to use the entire chain. A truncated portion of a T cell intracellular signaling domain can be used in place of the entire chain as long as it transduces a relevant T cell effector function signal.

Examples of the T cell intracellular signaling domains for use in CARs comprise a cytoplasmic sequence of a T cell receptor (TCR) and a co-stimulatory molecule that cooperates to initiate signal transduction upon antigen-receptor binding, as well as any derivatives or variants of these sequences and any synthetic sequence having the same function.

The signaling domain of a T cell receptor regulates the activation of the complex of the T cell receptor in a stimulatory or inhibitory manner. The CAR disclosed herein may comprise a cytoplasmic signaling sequence which acts in a stimulatory manner and may comprise a signaling motif known as the immunoreceptor tyrosine-based activation motif or ITAM. Examples of primary cytoplasmic signaling sequences containing the ITAM which may be included in the disclosed CARs include intracellular domains from CD3ζ, FcRγ, FcRβ, CD3γ, CD3δ, CD3ε, CDS, CD22, CD79a, CD79b, and CD66d proteins. In some embodiments, the cytoplasmic signaling molecule in the CAR comprises the T cell intracellular signaling domain from CD3ζ.

The intracellular domain of the CAR provided herein may comprise a CD3ζ chain portion and an intracellular co-stimulatory signaling domain. The co-stimulatory signaling domain may comprise an intracellular domain of a co-stimulatory molecule. The co-stimulatory molecule is a cell surface molecule other than the antigen receptor or its ligand, which is required for an effective lymphocyte response to an antigen. Examples of such a molecule include CD27, CD28, 4-1BB (CD137), OX40 (CD134), CD30, CD40, PD-1, ICOS, lymphocyte function associated antigen-1 (LFA-1), CD2, CD7, LIGHT, NKG2C and B7-H3.

In some embodiments, the CAR may comprise a CD3ζ signaling domain, a CD8 signaling domain, a CD28 signaling domain, a 4-1BB signaling domain, or a combination of two or more thereof. In an embodiment, the intracellular domain comprises a CD3-ζ signaling domain and a CD28 signaling domain. In another embodiment, the cytoplasmic domain comprises a CD3ζ signaling domain and a 4-1BB signaling domain. In yet another embodiment, the cytoplasmic domain comprises a CD3-ζ signaling domain as well as signaling domains of CD28 and CD137. One of ordinary skill in the art can change the order of one or more T cell signaling domains on the CAR as needed. The cytoplasmic signaling sequences in the cytoplasmic signaling portion of the CAR according to the present invention can be linked to each other in a random or specified order. Optionally, a short polypeptide linker having a length of preferably between 2 and 10 amino acids can form the linkage. The glycine-serine doublet provides a particularly suitable linker. In addition, between a signaling domain and a transmembrane domain of the CAR, there may be a spacer domain which comprises a polypeptide sequence. The spacer domain may comprise up to 300 amino acids, preferably 10 to 100 amino acids, and most preferably 25 to 50 amino acids.

The CARs provided herein (as parent CARs) can be modified by one skilled in the art, for example, by fusing them with other polypeptides, or retaining only fragments of the CARs provided herein, wherein these fusion molecules or fragments have at least part of the biological activities of the parent CARs, for example, the activity of recognizing target cells (such as tumor cells expressing EGFRvIII) or detecting, treating, or preventing a disease.

The CARs provided herein may also comprise additional amino acids at the amino or carboxyl terminus or both which are absent in the amino acid sequences of the parent CARs. Ideally, the additional amino acids do not interfere with the biological functions of the CARs or the functional portions thereof, such as recognizing target cells, detecting a cancer, or treating or preventing a cancer. More ideally, the additional amino acids enhance the biological activities compared to those of the parent CARs.

Also provided is a functional variant of the CAR described herein having a substantial or significant sequence identity or similarity with the parent CAR, wherein the functional variant retains a biological activity of the CAR from which the variant is derived. The functional variant encompasses, for example, those variants of the CARs described herein (parent CARs) that retain the ability to recognize target cells to a similar, the same, or a greater extent compared to the parent CARs. Referring to the parent CAR, the functional variant may have, for example, at least about 30%, about 50%, about 75%, about 80%, about 85%, about 90%, about 91%, about 92%, about 93%, about 94%, about 95%, about 96%, about 97%, about 98%, about 99% or higher identity with the parent CAR in terms of amino acid sequence.

For example, the functional variant may comprise the amino acid sequence of a parent CAR having at least one conservative amino acid substitution. Alternatively or additionally, the functional variant may comprise the amino acid sequence of a parent CAR having at least one non-conservative amino acid substitution. In this case, it is preferred that the non-conservative amino acid substitution does not interfere with or inhibit the biological activities of the functional variant. The non-conservative amino acid substitution may enhance a biological activity of the functional variant, so that the biological activity of the functional variant is increased compared with that of the parent CAR.

The CAR provided herein may comprise a synthetic amino acid in place of one or more naturally occurring amino acids. Such a synthetic amino acid is known in the art and comprises, for example, aminocyclohexanecarboxylic acid, norleucine, α-amino-n-decanoic acid, homoserine, S-acetamidomethyl-cysteine, trans-3- and trans-4-hydroxyproline, 4-aminophenylalanine, 4-nitrophenylalanine, 4-chlorophenylalanine, 4-carboxyphenylalanine, β-phenylserine, β-hydroxyphenylalanine, phenylglycine, α-naphthylalanine, cyclohexylalanine, cyclohexylglycine, dihydroindole-2-carboxylic acid, 1,2,3,4-tetrahydroisoquinoline-3-carboxylic acid, aminomalonic acid, aminomalonic acid monoamide, N'-benzyl-N'-methyl-lysine, N',N'-dibenzyl-lysine, 6-hydroxylysine, ornithine, α-aminocyclopentanecarboxylic acid, α-aminocyclohexanecarboxylic acid, oc-aminocycloheptanecarboxylic acid, -(2-amino-2-norbornane)-carboxylic acid, γ-diaminobutyric acid, α,β-diaminopropionic acid, homophenylalanine and α-tert-butylglycine.

The CAR provided herein can be glycosylated, amidated, carboxylated, phosphorylated, esterified, N-acylated, cyclized via for example a disulfide bond, converted into an acid addition salt and/or optionally dimerized or polymerized, or conjugated.

Methods of producing a chimeric antigen receptor, T cells comprising such a receptor and uses thereof (for example, for treating a cancer) are known in the art. For example, nucleic acid molecules encoding the disclosed chimeric antigen-binding receptors can be included in expression vectors (such as lentiviral vectors) for expression in host cells such as T cells to prepare the disclosed CARs. In some embodiments, a method of using the chimeric antigen receptor comprises isolating a T cell from a subject, transforming the T cell with an expression vector (such as a lentiviral vector) encoding the chimeric antigen receptor, and administering the engineered T cell which expresses the chimeric antigen receptor to the subject for treatment, for example, to treat a tumor in the subject.

The CAR provided herein may also be used in combination with a safety mechanism for controlling an activity of the CAR or the CAR-expressing cell. In an embodiment, the CAR can be co-expressed with "EGFRt". The "EGFRt" refers to an encoded truncated human epidermal growth factor receptor polypeptide that lacks the distal membrane EGF-binding domain and the cytoplasmic signaling tail but retains the extracellular epitope recognized by an anti-EGFR antibody. The EGFRt can be used as a non-immunogenic tool having the function of selecting a genetically modified cell as well as a tracking marker. On one hand, it can be used as a marker molecule for a CAR-T cell, and on the other hand, it can also be used to eliminate a CAR-T cell in the body when necessary, for example, through the ADCC pathway mediated by an EGFR antibody (such as cetuximab) (see US8802374B2); that is, it is used as a safety switch during clinical translation. In addition to using tEGFR, the CAR-T cell provided herein can also be considered for use in combination with a safety switch of another mechanism. For example, the CAR provided herein can be engineered so that a chemical inducer of dimerization (CID) can be used to control the CAR activity. In an example, a complete CAR provided herein can be expressed in separate fusion proteins (for example, the CD3ζ intracellular domain is expressed separately from other parts), and an FKBP/FRB-based binding domain and rapamycin (or a rapamycin analogue) responsible for a dimerization mechanism are used to reconstitute the complete CAR within a cell, thereby allowing control of the integrity and activity of the CAR by the presence or absence of rapamycin (for example, see description in the U.S. Patent No. 11,084,880). In addition, when using a self-cleaving peptide, which enables expression of a suicide-capable fusion protein at the same time, the activity of a CAR-T cell can be controlled by degrading the CAR molecule or causing CAR-T cell death. In this aspect, examples include use of the PROTAC technology to degrade a CAR molecule and use of a protein having an ability to induce apoptosis, such as caspase-9.

### Pharmaceutical composition comprising the anti-EGFRvIII antibody or antigen-binding fragment thereof and method for treatment

The anti-EGFRvIII antibody or antigen-binding fragment thereof as well as the fusion protein, the multispecific antibody molecule, and the immunoconjugate (which can be formulated into a pharmaceutical composition together with a pharmaceutically acceptable carrier) comprising the anti-EGFRvIII antibody or antigen-binding fragment thereof can be administered to a subject for preventing or treating a tumor. The effective dosage for this usage may depend on the severity of the disease and the overall condition of the patient's own immune system. Dosing regimens will also vary with the condition of the disease and the subject, and generally range from a single bolus dose or continuous infusion to multiple doses per day (for example, every 4-6 hours). Clinicians skilled in the art can readily determine whether a subject is a candidate for such a treatment based on, for example, clinical trials, physical examination, and the subject's family history of disease.

In some embodiments, the anti-EGFRvIII antibody or antigen-binding fragment thereof disclosed herein as well as the fusion protein, the multispecific antibody molecule, and the immunoconjugate comprising the anti-EGFRvIII antibody or antigen-binding fragment thereof can be administered in combination with one or more of other medicaments (such as an anti-tumor agent).

In some embodiments, the disease or condition to be treated is a tumor, preferably glioblastoma. The anti-EGFRvIII antibody or antigen-binding fragment thereof (as well as the fusion protein, the multispecific antibody molecule, and the immunoconjugate comprising the anti-EGFRvIII antibody or antigen-binding fragment thereof) disclosed herein can be administered by any appropriate method or route, optionally in combination with another anti-tumor agent. For example, routes of administration include oral, intravenous, intraperitoneal, subcutaneous or intramuscular administration.

The nucleic acid molecule encoding the anti-EGFRvIII antibody or antigen-binding fragment thereof, the expression vector comprising the nucleic acid molecule, and the host cell transfected with only the nucleic acid molecule or the expression vector provided herein can also be used in various ways for the above therapeutic purposes. For example, the expression vector can be introduced into a subject by means of a gene therapy known in the art to express a protein or a polypeptide of interest (the anti-EGFRvIII antibody or antigen-binding fragment thereof) to achieve a therapeutic purpose.

### Detection, diagnostic or therapeutic kit comprising the anti-EGFRvIII antibody or antigen-binding fragment thereof

The anti-EGFRvIII antibody or antigen-binding fragment thereof as well as other forms of the molecule (such as the fusion protein or the bispecific antibody molecule) comprising the anti-EGFRvIII antibody or antigen-binding fragment thereof provided herein can specifically bind to EGFRvIII in a sample. The content (or presence) of EGFRvIII in the sample can be conveniently determined by determining the amount of the formed complex between the anti-EGFRvIII antibody or antigen-binding fragment thereof and EGFRvIII, or the amount of the anti-EGFRvIII antibody or antigen-binding fragment thereof in the formed complex.

As described above, for this purpose, the anti-EGFRvIII antibody or antigen-binding fragment thereof provided herein can be linked to various detectable tags to facilitate detection by various means, including but not limited to using a bioluminescent, fluorescent, or radioactive label, or obtaining the amount of a product from an enzymatic reaction. After the EGFRvIII content in the sample is determined, it can be compared with the normal EGFRvIII content in the normal population to determine the disease condition or severity in the subject who provides the sample. Further, the change of the EGFRvIII content over time obtained by repeated detections during the treatment of the subject can be used to determine whether the treatment is effective, thereby providing a basis for changing the treatment protocol.

The anti-EGFRvIII antibody or antigen-binding fragment thereof as well as other forms of the molecule (such as the fusion protein or the bispecific antibody molecule) comprising the anti-EGFRvIII antibody or antigen-binding fragment thereof, or the cell expressing the CAR provided herein can be placed in a container to form a detection, diagnostic, or therapeutic kit. Such a container can be in the form of a box, an ampoule, a vial, a tube, a bag or another suitable container known in the art. The container can be made of plastic, glass, laminated paper, metal foil, or another material suitable for preserving a medicament. If necessary, instructions for use are also provided along with the container. The instructions may generally comprise information on how to use the anti-EGFRvIII antibody or antigen-binding fragment thereof, the composition or the CAR-T cell comprising the anti-EGFRvIII antibody or antigen-binding fragment thereof to treat or prevent a tumor (such as glioblastoma). For example, the instructions may include description of the therapeutic agent (such as the anti-EGFRvIII antibody or antigen-binding fragment thereof), dosage regimens for treating or preventing neoplasia (such as glioblastoma), precautions, warnings, indications, contraindications, adverse effects, animal pharmacology, clinical studies, and/or references. The instructions can be printed directly on the container (if present), or provided as a label attached to the container, or as a separate paper sheet, booklet, card or folded print in or along with the container.

In some embodiments, the anti-EGFRvIII antibody or antigen-binding fragment thereof provided herein binds to EGFRvIII with an affinity close to or superior to that of an existing control antibody. The anti-EGFRvIII antibody or antigen-binding fragment thereof provided herein is humanized and binds to EGFRvIII with an affinity close to or superior to that of an existing control antibody.

In the present studies, mice are immunized in various ways of immunization to compare the specificities and affinities of the antibodies obtained from immunization in the various ways of immunization to select the best conditions for immunization. Through positive and negative screening methods, a series of new murine monoclonal antibodies against the EGFRvIII antigen of interest are obtained. Their affinities are measured by ELISA, FACS, SPR or another method to select high-affinity antibodies which are then sequenced. The antibody sequences are designed to prepare CAR T cells targeting EGFRvIII. They are subjected to *in vitro* and *in vivo* efficacy studies to select the best antibody sequences, and the best murine antibody sequences selected are then humanized. The affinities of the humanized antibodies are determined through the SPR technology to select the humanized antibodies having the best affinities which are then sequenced. The humanized antibodies having a relatively high affinity are selected to prepare a series of CAR T cells targeting an EGFRvIII antigen. And *in vitro* and *in vivo* efficacy studies are carried out to select the best humanized antibody sequences. The best antibody sequences selected are used to prepare CAR NK cells, and the efficacies of the CAR NK cells in treating a solid tumor are determined through *in vitro* efficacy studies.

In order to overcome the effects of a solid tumor in suppressing a cellular immunotherapy, the present studies also performed gene modification based on the second-generation CAR T cells to improve their resistance to the inhibition from the tumor microenvironment, and their persistence and abilities of infiltration into a solid tumor. In the present studies, the self-developed EGFRvIII-targeting CAR T cells simultaneously express dnTGFβRII, or convert the inhibitory signal into an IL7 activation signal through dnTGFβRII, wherein the added element significantly improves the anti-tumor efficiencies of the CAR T cells or resists the inhibitory effects of TGFβ.

### Example

### 1. Experiment workflow

### 1.1 Development of murine antibody

### 1.1.1 Mouse immunization

Before immunization of the mice, 100 µl of blood was collected from the mice, left to stand at 37°C for 5 min, and then centrifuged to collect the serum as a negative serum control. The mice were immunized in 3 ways. Group 1 was immunized using a combination of a protein + a polypeptide, group 2 using target cells overexpressing EGFRvIII, and group 3 using a polypeptide. The adjuvant and the antigen were mixed at a ratio of 1:1 and emulsified. The emulsified immunogen was injected subcutaneously into the mice. One week after the second immunization, blood was collected from the mice, the serum was separated, and the antibody titer was tested. Additional immunization was performed based on the antibody titer until the serum titer met the requirements, and the final vaccination was performed.

### 1.1.2 Titer testing

The serum titer was tested by flow cytometry. The positive cells and negative cells were F98npEGFRvIII and F98npEGFR cells (ATCC) respectively. The cells were digested, collected in a centrifuge tube, put in a centrifuge machine, and centrifuged at 300 g for 5 min. The supernatant was discarded, and an appropriate amount of 1 x PBS was added for washing twice. The supernatant was discarded, and the cells were resuspended with 1 x PBS. 50 µl of cell suspension containing about 2 x 10^5 cells was added to a 96-well plate. To each well was added a 1/50 or 1/500 diluted serum respectively, with C097VEJ300 as a positive control antibody, mouse IgG as an isotype control and PBS as a negative control. To each well was added 100 µl of diluted fluorescent secondary antibody Alexa Fluor 647 AffiniPure Goat Anti-Mouse IgG, Fcγ fragment specific (min X Hu Bov Hrs SrProt). After incubation under shaking at 4°C for 30 min, the 96-well plate was taken out, and 150 µl of 1 x PBS was added to each well. Washing was carried out for 2 times, and the supernatant was discarded. 150 µl of 1 x PBS was added to each well to resuspend the cells for flow cytometry, and the reading of the flow cytometer was obtained.

### 1.1.3 Preparation of hybridoma cells

The two mice showing the best titer test results were sacrificed, and their spleens were taken out with autoclaved forceps in an ultra-clean workbench and then immersed in sterile physiological saline. The mouse spleen was fully minced with sterile scissors, then transferred to a sterile 70-µm cell mesh, and fully ground with the inner tube of a disposable sterile syringe. The mouse spleen cells ground on the cell mesh were washed with the RPMI-1640 culture medium for 3 times, and the washing fluid was collected to obtain the corresponding cell suspension. Centrifugation was carried out at 1,000 rpm for 5 min to collect the spleen cells. The well-growing SP 2/0 cells were resuspended, collected, and centrifuged at 1,000 rpm for 5 min. The cells were collected, washed and counted. Mouse spleen cells and SP 2/0 cells were mixed at a ratio of 5:1 and centrifuged. The cell pellets were flicked gently to make them looser, and placed in a water bath at 37°C for 1 min. A PEG-1450 solution (37°C) was added dropwise and slowly. The sample obtained after the dropwise addition was centrifuged and the supernatant was discarded. After fusion with the spleen cells of each mouse using the HAT screening medium, the cell suspension thus prepared was diluted, inoculated into a 96-well plate with addition of 150 µl liquid/well and placed in a CO₂ cell culture incubator for incubation. The medium was replenished every 3-5 days. After an appropriate cell density was reached at the bottom of the plate, the supernatant was taken for testing.

### 1.1.4 Titer testing of antibody in the hybridoma-based supernatant

The supernatants were screened for positive cells through the mirrorball experimental method. The positive cells and the negative cells were F98npEGFRvIII and F98npEGFR respectively. The supernatant samples and the controls were diluted and added to a 384-well plate. About 1 x 10^3 cells were added to each well, and an appropriate amount of CFSE was added to stain the cells. After mixing well, a secondary antibody of the corresponding species was added, and incubation was carried out at room temperature. Detection was performed with an instrument to obtain readings. The positive clones were selected and further screened using the FACS method.

### 1.1.5 Preparation and detection of subclones

The positive clones were subcloned using the limiting dilution method, and the cell subclones were cultured with 1 x HT medium in an incubator at 37°C with 5% carbon dioxide. The subclones were screened using flow cytometry.

### 1.1.6 Expression, purification and detection of antibodies from subclones

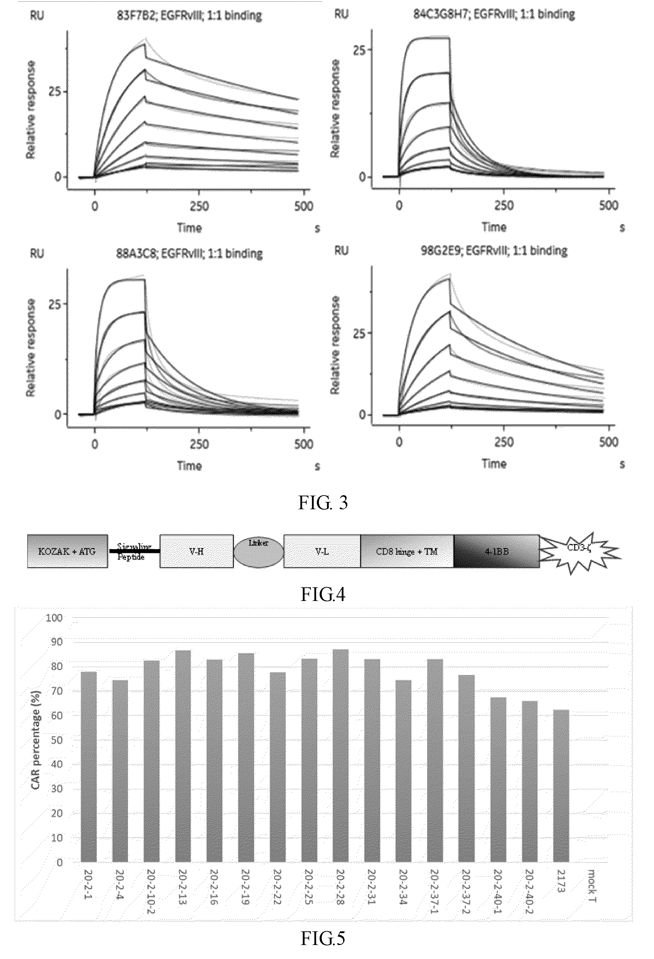

The subclone-based supernatants were taken and subjected to protein affinity purification. The purified monoclonal antibodies were detected to obtain their concentrations and diluted into a series of gradient concentrations for SPR detection, and the EC50 was calculated (FIG 3).

### 1.1.7 Sequencing of cloned candidate antibodies

A total RNA extraction kit was used to extract the total RNA from the monoclonal hybridoma cells. A reverse transcription kit was used to perform reverse transcription with the extracted total RNA as a template. The antibody sequences were amplified by the PCR method and sequenced.

### 1.1.8 Determination of antibody affinity by flow cytometry

(1) The F98npEGFRvIII cells were added at 1*10^5 cells/well, washed twice with 2 mL of 2% BAS BSA, and resuspended with 100 µL of PBS.
(2) The highest antigen concentration added corresponds to an amount of 16 ug. It was diluted to 8 concentrations in a gradient. The dilution system was shown in the following table.

| Amount of protein added (µg) | | | Dilution fold |
|---|---|---|---|
| | 16 | | 2 |
| | 8 | | 2 |
| | 4 | | 2 |
| | 1 | | 4 |
| | 0.25 | | 4 |
| | 0.0625 | | 4 |
| | 0.015625 | | 4 |
| | 0.0039063 | | 4 |
| | 0.0009766 | | 4 |

(3) The above diluted antibodies were added and allowed for reaction at 4°C for 30 min.
(4) The cells were washed three times with 2 mL of 2% BSA. Then the BV421-labeled anti-human IgG Fc secondary antibody was added and allowed for reaction at 4°C for 30 min; the BV421 anti-mouse IgG was used as the secondary antibody for mouse antibodies.
(5) The cells were washed twice with PBS and resuspended in 500 µL of PBS to get ready for detection and analysis on the machine.
(6) The mean fluorescence intensity (MFI) was calculated. The MFI values were plotted with the antibody concentrations, and the EC50 values were calculated to compare the affinities of different antibodies.

### 1.2 Preparation and functional studies of murine antibody-based CAR T cells

### 1.2.1 Design of CAR structure

The CAR structure comprised a developed ScFv structure of a murine antibody sequence against an EGFRvIII antigen, and further comprised but not limited to the CD8 hinge region and transmembrane region, the 4-1BB activation signal and the CD3z activation domain (FIG 4). In addition to this, other modifications were possible, including secretion of a cytokine, secretion of a chemokine, modification of an immune checkpoint inhibitor gene, blocking of an inhibitory signal from tumor microenvironment and conversion of the inhibitory signal to an activation signal, etc. for the fourth-generation CAR T cells.

### 1.2.2 Construction of expression plasmids

The synthesized CAR gene sequences were linked to the lentiviral expression plasmids through molecular cloning. The competent Stbl3 cells were transfected with the synthesized plasmids, kept under shaking, and then used to extract the plasmids in large quantities for subsequent lentiviral packaging.

### 1.2.3 Lentiviral packaging and titer testing

The various CAR plasmids and the packaging plasmids were co-transfected into the 293T cells respectively. The cell supernatants were collected 48 h after transfection and centrifuged at 4,000 rpm for 10 min. The supernatants were collected and filtered with a 0.45 µm filter membrane. The lentivirus pellets were collected using an ultracentrifuge machine. The centrifugation was carried out at 100,000×g and 4°C for 90 min without using a brake. After concentration, the lentivirus was added into 0.5-mL cryogenic storage vials at 200 µl per vial, cryopreserved at -80°C separately, and then tested for its titer by flow cytometry.

### 1.2.4 Preparation of CAR T cells

20 mL of the peripheral blood of a healthy person was taken and added to a 50-mL centrifuge tube. 1 mL of RosetteSep^{™} Cocktail was added to the blood and mixed well to obtain an antibody concentration of 50 µL/mL. 20 mL of GE Ficoll solution was added into another 50-mL centrifuge tube. 20 mL of diluted blood was drawn carefully using a pipette and slowly added to the upper layer of the Ficoll solution in a ratio of 1:1. The centrifuge tube with blood sample added was put into a centrifuge machine and centrifuged at 400 g for 30 min. A sterile pipette was used to draw and remove the upper layer of plasma and platelets without touching the layer containing mononuclear cells. The second layer, a ring-shaped milky white layer containing lymphocytes, was drawn carefully and added into another centrifuge tube. Each tube of lymphocyte suspension was added with PBS three-fold of the volume of the suspension to wash the cells, and centrifuged at 400 g for 10 min. The supernatant was discarded, and the thus obtained cells were resuspended with 5 mL of PBS and centrifuged at 1,200 rpm for 10 min. Finally, the cells were suspended with the X-VIVO + 100 U IL2 complete culture medium and counted. 1 mL of isolated T lymphocytes with a density of 1×10^6 was inoculated into one well of a 24-well plate, and added with 25 µl of CD3/CD28 magnetic beads. The cells were cultured in an incubator with 5% CO₂ at 37°C and used for lentiviral infection 48 h later. The corresponding lentivirus was added according to an MOI=0.5. The next day, the cell culture medium was replenished to 30 ml per well, transferred to a T 75 bottle, and placed horizontally for culture.

### 1.2.5 Determination of CAR-positive rates of CAR T cells

Three days later, the CAR T cells were transferred to a sterile flow cytometry tube. Each tube of cells was placed on a biolegend magnet for 2 min to remove the magnetic beads. Then, some cells were taken and stained with the flow cytometry antibody (AF647-protein L) to determine the CAR expressing rates.

### 1.2.6 Determination of in vitro killing efficiencies of CAR T cells

In a blank plate for detection, a sample well was inoculated with the U87MG-EGFRvIII target cells at 1×10^4 cells/well, which was repeated for 6 wells; and 6 control wells were set with 100 µl per well. After an overnight, the supernatants were discarded, and the CAR-T cells were added at an effector-to-target ratio (E/T ratio) = 2.5:1, 100 µl per well. After 6 h, centrifugation was performed at 300 g for 5 min, and the supernatants in all wells were removed as completely as possible. 100 µl of lysis solution was added to each well for lysis at room temperature for 10 min, followed by detection on the machine. 100 µL of firefly luciferase detection reagent was added to each well. The killing efficiency was calculated according to the following formula: killing efficiency (%) = (fluorescence intensity of control well - fluorescence intensity of killing well)/(fluorescence intensity of control well - fluorescence intensity of blank well) × 100%.

### 1.2.7 Study of in vivo efficacies of CAR T cells

Male NOG mice aged 6-8 weeks were selected as the experimental animals. After dissociation of U87MG-EGFRvIII cells, the cells were resuspended in and washed twice with sterile 1 × PBS for living cell count. The cell density was adjusted to 5×10^5 cells/50 µl/mouse. For injection, the cells were mixed with 50 µl of matrigel at a volume ratio of 1:1 for each mouse; that is, each mouse was finally injected with a total volume of 100 µl of cell suspension. The transplantation was directed to the subcutaneous site of the lower right side of the back of the mouse. After tumor transplantation, the tumor volume was measured with a caliper three times a week, and calculated as TV = 0.5a × b2. When the tumor size reached 100 mm³, the CAR-T cells at 5 x 10^6 cells/mouse or physiological saline were/was reinfused. The tumor size and mouse survival were continuously observed.

### 1.3 Humanization of murine antibody sequences

Sequences of the top two clones having the best *in vivo* efficacies were selected for antibody sequence humanization. (1) The hybridoma-derived V gene was codon optimized to be more suitable for expression in a mammal and synthesized through overlap extension PCR. (2) The mouse monoclonal antibody V gene will be grafted to the most suitable framework through CDR grafting for humanization. 12-16 humanized variants will be designed with minimal framework differences from the human germline V-gene. (3) Up to 3 variants will be designed for each site to remove the key PTM sites in the murine antibody sequences. (4) After codon optimization for expression in a mammal, the humanized V-genes and the PTM-removed V-genes will be synthesized. The mouse and all variant genes will be cloned to the human IgG expression vectors to produce the human IgG1 constructs of the required isotype. The antibody genes are synthesized, expressed, and purified. After purification, the antibody affinities are determined using the SPR method. The humanized antibody sequences having the best affinities are selected.

### 1.4 Preparation and functional studies of humanized antibody-based CAR T cells

### 1.4.1 Design of CAR structure

The CAR structure comprised a developed ScFv structure of murine antibody sequence against an EGFRvIII antigen, and further comprised but not limited to the CD8 hinge region and transmembrane region, the 4-1BB activation signal and the CD3z activation domain. In addition to this, other modifications were possible, including secretion of a cytokine, secretion of a chemokine, modification of an immune checkpoint inhibitor gene, blocking of an inhibitory signal from tumor microenvironment and conversion of the inhibitory signal to an activation signal, etc. for the fourth-generation CAR T cells.

### 1.4.2 CAR gene synthesis

The synthesized CAR gene sequences were linked to the lentiviral expression plasmids through molecular cloning. The competent Stbl3 cells were transfected with the synthesized plasmids, kept under shaking, and then used to extract the plasmids in large quantities for subsequent lentiviral packaging.

### 1.4.3 Lentiviral packaging and titer testing

The various CAR plasmids and the packaging plasmids were co-transfected into the 293T cells respectively. The cell supernatants were collected 48 h after transfection and centrifuged at 4,000 rpm for 10 min. The supernatants were collected and filtered with a 0.45 µm filter membrane. The lentivirus pellets were collected using an ultracentrifuge machine. The centrifugation was carried out at 100,000×g and 4°C for 90 min without using a brake. After concentration, the lentivirus was added into 0.5-mL cryogenic storage vials at 200 µl per vial, cryopreserved at -80°C separately, and then tested for its titer by flow cytometry.

### 1.4.4 Preparation of CAR T cells

20 mL of the peripheral blood of a healthy person was taken and added to a 50-mL centrifuge tube. 1 mL of RosetteSep^{™} Cocktail was added to the blood and mixed well to obtain an antibody concentration of 50 µL/mL. 20 mL of GE Ficoll solution was added into another 50-mL centrifuge tube. 20 mL of diluted blood was drawn carefully using a pipette and slowly added to the upper layer of the Ficoll solution in a ratio of 1:1. The centrifuge tube with blood sample added was put into a centrifuge machine and centrifuged at 400 g for 30 min. A sterile pipette was used to draw and remove the upper layer of plasma and platelets without touching the layer containing mononuclear cells. The second layer, a ring-shaped milky white layer containing lymphocytes, was drawn carefully and added into another centrifuge tube. Each tube of lymphocyte suspension was added with PBS three-fold of the volume of the suspension to wash the cells, and centrifuged at 400 g for 10 min. The supernatant was discarded, and the thus obtained cells were resuspended with 5 mL of PBS and centrifuged at 1,200 rpm for 10 min. Finally, the cells were suspended with the X-VIVO + 100 U IL2 complete culture medium and counted. 1 mL of isolated T lymphocytes with a density of 1×10^6 was inoculated into one well of a 24-well plate, and added with 25 µl of CD3/CD28 magnetic beads. The cells were cultured in an incubator with 5% CO₂ at 37°C and used for lentiviral infection 48 h later. The corresponding lentivirus was added according to an MOI=0.5. The next day, the cell culture medium was replenished to 30 ml per well, transferred to a T 75 bottle, and placed horizontally for culture.

### 1.4.5 Determination of CAR-positive rates of CAR T cells

Three days later, the CAR T cells were transferred to a sterile flow cytometry tube. Each tube of cells was placed on a biolegend magnet for 2 min to remove the magnetic beads. Then, some cells were taken and stained with the flow cytometry antibody (AF647-protein L) to determine the CAR expressing rates.

### 1.4.6 Determination of in vitro killing efficiencies of CAR T cells

In a blank plate for detection, a sample well was inoculated with the U87MG-EGFRvIII target cells at 1×10^4 cells/well, which was repeated for 6 wells; and 6 control wells were set with 100 µl per well. After an overnight, the supernatants were discarded, and the CAR-T cells were added at an effector-to-target ratio (E/T ratio) = 2.5:1, 100 µl per well. After 6 h, centrifugation was performed at 300 g for 5 min, and the supernatants in all wells were removed as completely as possible. 100 µl of lysis solution was added to each well for lysis at room temperature for 10 min, followed by detection on the machine. 100 µL of firefly luciferase detection reagent was added to each well. The killing efficiency was calculated according to the following formula: killing efficiency (%) = (fluorescence intensity of control well - fluorescence intensity of killing well)/(fluorescence intensity of control well - fluorescence intensity of blank well) × 100%.

### 1.4.7 Study of in vivo efficacies of CAR T cells

Male NOG mice aged 6-8 weeks were selected as the experimental animals. After dissociation of U87MG-EGFRvIII cells, the cells were resuspended in and washed twice with sterile 1 × PBS for living cell count. The cell density was adjusted to 5×10^5 cells/50 µl/mouse. For injection, the cells were mixed with 50 µl of matrigel at a volume ratio of 1:1 for each mouse; that is, each mouse was finally injected with a total volume of 100 µl of cell suspension. The transplantation was directed to the subcutaneous site of the lower right side of the back of the mouse. After tumor transplantation, the tumor volume was measured with a caliper three times a week, and calculated as TV = 0.5a × b2. When the tumor size reached 100 mm³, the CAR-T cells at 5 x 10^6 cells/mouse or physiological saline were/was reinfused. The tumor size and mouse survival were continuously observed.

### 1.5 Preparation and functional studies of humanized antibody-based CAR NK cells

### 1.5.1 Preparation of CAR NK cells

(1) The NK cells were cultured and added to a Retronectin coated 24-well plate. Then the lentivirus was added to the cultured NK cells according to MOI = 20. Centrifugation was carried out at 32°C for 30 min, and the cells were transferred to an incubator with 5% CO₂ at 37°C for culture.
(2) After 48 hours from infection, the cells were washed with a complete medium and cultured in a complete medium containing IL2 at 300 IU/ml.

### 1.5.2 Determination of CAR-positive rates of CAR NK cells

The CAR-NK cells were transferred to a sterile flow cytometry tube and stained with the flow cytometry antibody (AF647-protein L) to determine the CAR expressing rates.

### 1.5.3 Determination of in vitro killing efficiencies of CAR NK cells

In a blank plate for detection, a sample well was inoculated with the U87MG-EGFRvIII target cells at 1×10^4 cells/well, which was repeated for 6 wells; and 6 control wells were set with 100 µl per well. After an overnight, the supernatants were discarded, and the CAR-NK cells were added at an effector-to-target ratio (E/T ratio) = 1:1, 100 µl per well. After 6 h, centrifugation was performed at 300 g for 5 min, and the supernatants in all wells were removed as completely as possible. 100 µl of lysis solution was added to each well for lysis at room temperature for 10 min, followed by detection on the machine. 100 µL of firefly luciferase detection reagent was added to each well. The killing efficiency was calculated according to the following formula: killing efficiency (%) = (fluorescence intensity of control well - fluorescence intensity of killing well)/(fluorescence intensity of control well - fluorescence intensity of blank well) × 100%.

### 1.6 Preparation and functional studies of CAR T cells resisting inhibition by TGFβ

### 1.6.1 Design of CAR structure

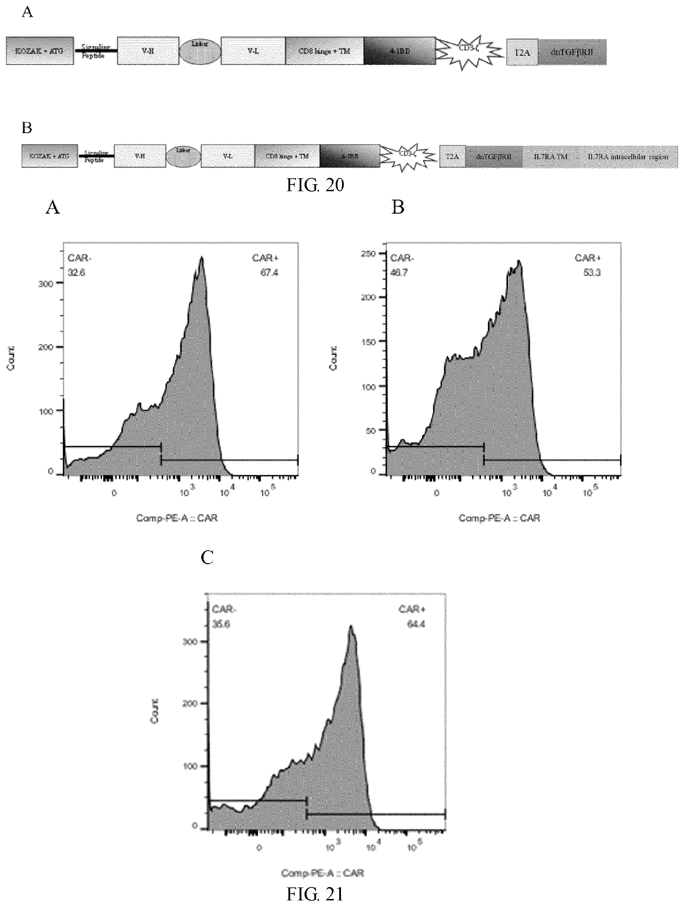

Two CAR constructs were designed to resist inhibition by TGFβ. The first one was designed with the dominant negative receptor dnTGFβRII connected by a T2A self-cleaving peptide (FIG 20A). As a receptor, the dnTGFBRII had the ability to bind TGFβ, but lacked the ability to switch binding of TGFβ into an intracellular signal (for example, it lacked an intracellular domain or lacked a kinase activity required for signaling). The second one is designed with the chimeric switch receptor linked by a T2A self-cleaving peptide (dnTGFβRII-IL7RA TM-IL7RA intracellular region) (FIG 20B). The presence of the chimeric switch receptor allowed resistance against inhibition by TGFβ, and switch of binding of TGFβ into an intracellular IL7RA receptor stimulating signal to promote the proliferation and/or activities of the CAR T cells.

### 1.6.2 Preparation of CAR T cells

The specific method was the same as that for preparing the humanized antibody-based CAR T cells.

### 1.6.3 Study of function of CAR T cells

In a blank plate for detection, a sample well was inoculated with the U87MG-EGFRvIII target cells at 1×10^4 cells/well, which was repeated for 6 wells; and 6 control wells were set with 100 µl per well. After an overnight, the supernatants were discarded, and the CAR-T cells were added at an effector-to-target ratio (E/T ratio) = 2.5:1, 100 µl per well. After 6 h, centrifugation was performed at 300 g for 5 min, and the supernatants in all wells were removed as completely as possible. 100 µl of lysis solution was added to each well for lysis at room temperature for 10 min, followed by detection on the machine. 100 µL of firefly luciferase detection reagent was added to each well. The killing efficiency was calculated according to the following formula: killing efficiency (%) = (fluorescence intensity of control well - fluorescence intensity of killing well)/(fluorescence intensity of control well - fluorescence intensity of blank well) × 100%.

In order to compare the modified fourth-generation CAR T cells in terms of resistance to inhibition by TGFβ, a TGFβ inhibition group was set up for comparison in the killing experiment. Before the CAR T cells were used for the killing experiment, some of the cells were taken and 30 ng/ml of TGFβ was applied to inhibit the CAR T cells. Then the killing efficiencies of the inhibited and the uninhibited CAR T cells were monitored.

### 1.6.4 Study of in vivo function of CAR T cells

Male NOG mice aged 6-8 weeks were selected as the experimental animals. After dissociation of U87MG-EGFRvIII cells, the cells were resuspended in and washed twice with sterile 1 × PBS for living cell count. The cell density was adjusted to 5×10^5 cells/50 µl/mouse. For injection, the cells were mixed with 50 µl of matrigel at a volume ratio of 1:1 for each mouse; that is, each mouse was finally injected with a total volume of 100 µl of cell suspension. The transplantation was directed to the subcutaneous site of the lower right side of the back of the mouse. After tumor transplantation, the tumor volume was measured with a caliper three times a week, and calculated as TV = 0.5a × b2. When the tumor size reached 100 mm³, the CAR-T cells at 3 x 10^6 cells/mouse or physiological saline were/was reinfused. The tumor size and mouse survival were continuously observed.

The information of some of the sequences involved in this Example was as follows:

| Name | Amino acid sequence | Nucleotide sequence |
|---|---|---|
| T2A | EGRGSLLTCGDVEENPGP (SEQ ID NO: 191) | GAGGGCAGAGGAAGTCTTCTAACATGCGG TGACGTGGAGGAGAATCCCGGCCCT (SEQ ID NO: 192) |
| dnTGFβRII | MGRGLLRGLWPLHIVLWTRIASTIPP HVQKSVNNDMIVTDNNGAVKFPQL CKFCDVRFSTCDNQKSCMSNCSITSI CEKPQEVCVAVWRKNDENITLETVC HDPKLPYHDFILEDAASPKCIMKEK KKPGETFFMCSCSSDECNDNIIFSEE YNTSNPDLLLVIFQ (SEQ ID NO: 193) | ATGGGTCGGGGGCTGCTCAGGGGCCTGTG GCCGCTGCACATCGTCCTGTGGACGCGTAT CGCCAGCACGATCCCACCGCACGTTCAGA AGTCGGATGTGGAAATGGAGGCCCAGAAA GATGAAATCATCTGCCCCAGCTGTAATAGG ACTGCCCATCCACTGAGACATATTAATAAC GACATGATAGTCACTGACAACAACGGTGC AGTCAAGTTTCCACAACTGTGTAAATTTTG TGATGTGAGATTTTCCACCTGTGACAACCA GAAATCCTGCATGAGCAACTGCAGCATCAC CTCCATCTGTGAGAAGCCACAGGAAGTCT GTGTGGCTGTATGGAGAAAGAATGACGAG AACATAACACTAGAGACAGTTTGCCATGAC CCCAAGCTCCCCTACCATGACTTTATTCTGG AAGATGCTGCTTCTCCAAAGTGCATTATGA AGGAAAAAAAAAAGCCTGGTGAGACTTTC TTCATGTGTTCCTGTAGCTCTGATGAGTGC AATGACAACATCATCTTCTCAGAAGAATAT AACACCAGCAATCCTGACTTGTTGCTAGTC |
| | | ATATTTCAAGTGACAGGCATCAGCCTCCTG CCA (SEQ ID NO: 194) |
| IL7RA TM | PILLTISILSFFSVALLVILACVLW (SEQ ID NO: 195) | CCAATTCTGCTGACCATTTCAATCCTTTCCT TTTTCTCCGTGGCTCTCCTGGTGATTCTGGC ATGCGTGCTGTGG (SEQ ID NO: 196) |
| IL7RA intracellular region | KKRIKPIVWPSLPDHKKTLEHLCKK PRKNLNVSFNPESFLDCQIHRVDDIQ ARDEVEGFLQDTFPQQLEESEKQRL GGDVQSPNCPSEDVVITPESFGRDSS LTCLAGNVSACDAPILSSSRSLDCRE SGKNGPHVYQDLLLSLGTTNSTLPP PFSLQSGILTLNPVAQGQPILTSLGSN QEEAYVTMSSFYQNQ (SEQ ID NO: 197) | AAGAAGCGCATCAAGCCCATCGTTTGGCCC TCTCTTCCCGATCACAAGAAGACCCTGGAG CACCTGTGCAAGAAGCCGCGCAAGAACCT CAACGTGTCCTTCAACCCAGAGTCCTTCCT GGACTGTCAAATCCACCGCGTCGATGACAT CCAGGCCCGTGATGAAGTCGAGGGCTTTCT GCAGGACACGTTCCCGCAACAGCTAGAAG AGTCGGAGAAGCAGCGCCTGGGTGGTGAT GTGCAGAGCCCTAACTGCCCATCGGAAGAT GTGGTCATCACCCCGGAGAGCTTCGGCCG GGATTCCTCTTTGACTTGCCTGGCCGGGAA CGTGTCCGCGTGCGACGCGCCCATCCTATC CTCCTCCAGATCTCTGGACTGTAGGGAGTC TGGCAAAAACGGACCTCACGTATATCAGGA CCTGTTGCTGAGCCTGGGAACCACCAACTC GACCCTTCCTCCGCCCTTCTCCTTACAGTC CGGCATCCTGACCCTCAACCCTGTTGCCCA GGGCCAGCCGATCCTCACTTCACTGGGCTC CAACCAGGAGGAGGCCTACGTGACCATGT CTAGCTTCTACCAGAACCAG (SEQ ID NO: 198) |

### ◆ Experimental results

### 1. Determination results of affinities of murine antibodies

### 1.1. Determination results of affinities of antibodies in the supernatants of murine antibody-based subclones

The abilities of the subclone-based supernatants to bind to F98npEGFR and F98npEGFRvIII were determined by flow cytometry respectively. The results showed that the selected positive subclones could specifically bind to the target cell F98npEGFRvIII which overexpressed EGFRvIII, but did not bind to the target cell F98npEGFR which overexpressed EGFR. This demonstrated that specific antibodies which could specifically bind to the mutant form of target antigen EGFRvIII were obtained through screening (FIG 2). 2).

### 1.2. Determination results of antibody affinities of purified murine antibodies

The SPR results showed that the murine antibodies with good *in vitro* efficacies had relatively high affinities for the target antigen EGFRvIII (Table 1 and FIG. 3).

**Table 1 Affinities of purified murine antibodies as determined by the SPR method**

| **Ligand** | **Analyte** | **Sequence** | **Chi² (RU²)** | **ka (1/Ms)** | **kd (1/s)** | **KD (M)** | **Rmax (RL)** |
|---|---|---|---|---|---|---|---|
| EGFRvIII | 83F7B2 | 20-2-1 | 3.99E-01 | 5.01E+04 | 1.19E-03 | 2.37E-08 | 35.7 |
| EGFRvIII | 84C3G8H7 | 20-2-28 | 2.87E-01 | 1.97E+05 | 1.65E-02 | 8.37E-08 | 41.9 |
| EGFRvIII | 88A3C8 | 20-2-4 | 7.36E-01 | 1.33E+05 | 8.64E-03 | 6.50E-08 | 34.1 |
| EGFRvIII | 98G2E9 | 20-2-40 | 5.75E-01 | 5.25E+04 | 2.80E-03 | 5.33E-08 | 34.7 |

### 2. Sequences of murine antibodies (using IMGT numbering scheme)

| **plamid ID** | **Clone ID** | **Amino acid sequence of antibody** | |
|---|---|---|---|
| | | **Heavy chain: Amino acid sequence *FR1-*CDR1*-FR2-*CDR2*-FR3-*CDR3*-FR 4*** | **Light chain: Amino acid sequence *FR1-*CDR1*-FR2-*CDR2*-FR3-*CDR3-*FR4*** |
| **20-2-1** | **83F7 B2** | QIQLVQSGPELKKPGETVKISCKASGYT FTDFSMHWVKQAPGKGLKWMGWIN TETGEPSYADDFKGRFAFSLETSASTA YLQINNLKNEDTATYFCANYGYDVRG DYWGQGTTLTVSS (SEQ ID NO: 115) | EIVLTQSPTTMAASPGEKITITCSASS SISSNYLHWYQQKPGFSPKLLIYGT SNLASGVPARFSGSGSGTSYSLTIGT MEAEDVATYYCHQGSSIPLTFGAGT KLELK (SEQ ID NO: 116) |
| **20-2-4** | **88A3 C8** | EVQLQQSGAELVRPGALVKLSCKASGF NIKDYYMHWVKQRPEQGLEWIGWID PENGNTIYDPKFQGKASITADTSSNTA YLQLSSLTSEDTAVYYCARGWFAYWGQ GTLVTVSA (SEQ ID NO: 117) | DILMTQSPSSLAMSVGQKVTMSCKS SQSLLNSSNQKNHLAWYQQKPGQ SPKLLVYFASTRASGVPDRFIGSGSG TDFTLTISSVQAEDLADYFCQQHYS TPLTFGAGTKLELK (SEQ ID NO: 118) |
| **20-2-7** | **89C2 A11** | QIQLVQSGPELKKPGETVKISCKASGYT FTDYSIHWVKQAPGKGLKWMGWINT ETGEPTYADDFKGRFAFSLETSASTAY LQINNLKNEDTATYFCSNYGYDVRGD YWGQGTTLTVSS (SEQ ID NO: 119) | EIVLTQSPTTMAASPGENITITCSASS GISSNYLHWYQQKPGFSPELLIYST SNLASGVPARFSGSGSGTSYSLTIGT MEAEDVATYYCHQGSDIPLTFGAGT RLELK (SEQ ID NO: 120) |
| **20-2-10** | **75H11 B1** | EVQLQQSGADLVRPGALVKLSCKASGF NIKDYYLHWVKQRPEQGLEWIGWID PENGNTIYDPKFQGKASITADTSSNTA YLQLSSLTSEDTAVYYCARGYLTYWGQ GTLVTVSA (SEQ ID NO: 121) | DIVMTQSPSSLAMSVGQKVTMNCKS SQNLLNSSNQKNYLAWFQQKPG QSPKLLVYFASTRYSGVPDRFMGSG SGTDFTLTVSSVQAEDLADYFCQQH YSTPLTFGAGTKLELK (SEQ ID NO: 122) |
| **20-2-13** | **90F6 C1** | QVQLQQPGAELVKPGASVKLSCKASGY SFTRYWMHWVKQRPGQGLEWIGEIN PSNGRANYNEKFMSKATLTVDKSSST AYMQLSSLTSEDSAVYYCARGREITTG FAYWGQGTLVTVSA (SEQ ID NO: 123) | SIVMTQTPKFLLVSAGDRVTITCKAS QSVSNDVVWYQQKPGQSPKLLIYY ASNRYTGVPDRFTGSGYGTDFTFTI TTVQAEDLAVYFCQQDYSSPWTFG GGTKLEIK (SEQ ID NO: 124) |
| **20-2-16** | **90H3 D2** | EVQLQQSGAELVRPGALVKLSCKASGF NIKDYYMHWVKQRPEQGLEWIGWID PENGNTIYDPKFQGKATITADTSSNTA YLQLSSLTSEDTAVYYCARGYLVYWGQ GTLVTVSA (SEQ ID NO: 125) | DIVMTQSPSSLAMSVGQKVTMSCKS SQSLLNSSNQKNYLAWYQQKPGQ SPKLLVYFASTRESGVPDRFIGSGSG TDFTLTISSVQAEDLADYFCQQHYSI PLTFGAGTKLELK (SEQ ID NO: 126) |
| **20-2-19** | **53H2 H7(re combi nantly expre ssed)** | EVQLQQSGAELVRPGALVKLSCKASGF NIKDYYMHWVKQRPEQGLEWIGWID PENGNTIYDPKFQGKASITADTSSNTA YLQLSSLTSEDTAVYYCARGYLAYWGQ GTLVTVSA (SEQ ID NO: 127) | DIVMTQSPSSLAMSVGQKVTMSCKS SQSLLNSSNQKNYLAWYQQKPGQ SPKLLVYFASTRKSGVPDRFIGSGS GTDFTLTISSVQAEDLADYFCQQHY STPLTFGAGTKLELK (SEQ ID NO: 128) |
| **20-2-22** | **57D8 C3** | EVQLQQSGAELVRPGALVKLSCKASGF NIKDYYMHWVKQRPEQGLEWIGWID PENGNTIYDPKFQGKASITADTSSNTA CLQLSSLTSEDTAVYYCARGWFAYWG QGTLVTVSA (SEQ ID NO: 129) | DILMTQSPSSLAMSVGQKVTMSCKS SQSLLNSSNQKNHLAWYQQKPGQ SPKLLVYFASTRQSGVPDRFIGSGS GTDFTLTISSVQAEDLADYFCQQHY STPLTFGAGTKLELK (SEQ ID NO: 130) |
| **20-2-25** | **70B6 F6H1 1** | EVQLQQSGAELVRPGALVKLSCKASGF NIKDYYMHWVKQRPEQGLEWIGWID PENGNTIYDPKFQGKASITADTSSNTA YLQLSSLTSEDTAVYYCARGYLVYWGQ GTLVTVSA (SEQ ID NO: 131) | DIVMTQSPSSLAMSVGQKVTMSCKS SQSLLNSSNQKNYLAWYQQKPGQ SPKLLVYFASTRQSGVPDRFIGSGS GTDFTLTISSVQAEDLADYFCQQHY STPLTFGAGTKLELK (SEQ ID NO: 132) |
| **20-2-28** | **84C3 G8H7** | EVQLQQSGAELVRPGALVKLSCKASGF NIKDYYMHWVKQRPEQGLEWIGWID PENGNTIYDPKFQGKASITADTSSNTA YLQLSSLTSEDTAVYYCARGWFAYWGQ GTLVTVSA (SEQ ID NO: 133) | DIVMTQSPSSLAMSVGQKVTMSCKS SQSLLNSSNQKNHLAWYQQKPGQ SPKLLVYFASTRGSGVPDRFIGSGS GTDFTLTISSVQAEDLADYFCQQHY STPLTFGAGTKLELK (SEQ ID NO: 134) |
| **20-2-31** | **95G1 0G8E 4** | EVQLQQSGAELVRPGALVKLSCKASGF NIKDYYMHWVKQRPEQGLEWIGWID PENGNTIYDPKFQGKASITADTSSNTA | DIVMTQSPSSLAMSVGQKVTMSCKS SQSLLNSSNQKNYLAWYQQKPGQ SPKLLVYFASTRDSGVPDRFIGSGSG |
| | | YLQLSSLTSEDTAVYYCARGYLVYWGQ GTLVTVSA (SEQ ID NO: 135) | TDFTLTISSVQAEDLADYFCQQHYS TPLTFGAGTKLELK (SEQ ID NO: 136) |
| **20-2-34** | **84G9 F9** | EVQLQQSGAELVRPGALVKLSCKASGF NIKDYYMHWVKQRPEQGLEWIGWID PENGNTIYDPKFQGKASITADTSSNTA YLQLSSLTSEDTAVYYCARGYLVYWGQ GTLVTVSA (SEQ ID NO: 137) | DIVMTQSPSSLAMSVGQKVTMSCKS SQSLLNSSNQKNYLAWYQQKPGQ SPKLLVYFASTRESGVPDRFIGSGSG TDFTLTISSVQAEDLADYFCQQHYS TPLTFGAGTKLELK (SEQ ID NO: 138) |
| **20-2-37** | **98B10 B12** | EVMLVESGGGLVKPGGSLKLSCAASRF TFSNYAMSWVRQTPEKRLEWVATITS GGSYTYYPDSVKGRFTISRDNAKNTL YLQMSSLRSEDTAMYYCARKDYGNY WFAYWGQGTLVTVSA (SEQ ID NO: 139) | DVVMTQTPLSLPVSLGDQASISCRSS QSLVHSDGNTYLHWYLQKPGQSP KLLIYKVSNRFSGVPDRFSGSGSGT DFTLKISRVEAEDLGVYFCSQSIHVP WTFGGGTKLEIK (SEQ ID NO: 140) |
| **20-2-40-1** | **98G2 E9** | EVLLVESGGGLVKPGGSLKLSCAASGF TFSGYAMSWVRQTPEKRLEWVATITS GGSYTYYPDSVKGRFTISRDNAKNTL YLQMSSLRSEDTAMYYCARKDYGNY WFAYWGQGTLVTVSA (SEQ ID NO: 141) | QIVLTQSPAIMSASPGEKVTITCSASS SVSYMHWFQQKPGTSPKLWIYSTS NLASGVPARFSGSGSGTSYSLTISRM EAEDAATYYCQQRSSYPLTVGAGTK LELK (SEQ ID NO: 142) |
| **20-2-40-2** | **98G2 E9** | EVLLVESGGGLVKPGGSLKLSCAASGF TFSGYAMSWVRQTPEKRLEWVATITS GGSYTYYPDSVKGRFTISRDNAKNTL YLQMSSLRSEDTAMYYCARKDYGNY WFAYWGQGTLVTVSA (SEQ ID NO: 143) | DVVMTQTPLSLPVSLGDQASISCRSS QSLVHSDGNTYLHWYLQKPGQSP KLLIYKVSNRFSGVPDRFSGSGSGT DFTLKISRVEAEDLGVYFCSQTTQV PWTFGGGTKLEIK (SEQ ID NO: 144) |
| **20-2-43** | **115A1 0H4** | DVQLQESGPDLVKPSQSLSLNCTVTGY SITSGYSWHWIRQFPGNKLEWMGYIH YSGSTNYNPPLKSRIFIARDTSKNQFF LQLTSVTTEDTATYYCARGVVSNYAM GNWGQGTSVTVSS (SEQ ID NO: 145) | ETTVTQSPASLSMAIGEKVTIRCITNT DIDDDMNWYQQKPGEPPKLLISEG NTLRPGVPSRFSSSGYGTDFVFTIEN MLSEDVADYYCLQSDDLPLTFGSG TKLEIK (SEQ ID NO: 146) |
| **20-2-49** | **121E9 D12** | QVQLQQSGAELAKPGASVKMSCKASAY TFTTYWMHWVKQRPGQGLEWIGYIN PNTAYTEYNQNFKDKATLTADKSSST AYMQLGSLTSEDSAVYYCARGAYYRTY YAMDYWGQGTSVTVSS (SEQ ID NO: 147) | DIVLTQSPASLAVSLGQRATISCKASQ SVDYDGDSYMNWYQQKPGQPPKL LIYAASNLESGIPARFSGSGSGTDFT LNIHPVEEEDAATYYCLQSNEDPYT FGGGTRLEIN (SEQ ID NO: 148) |
| **20-2-52** | **124A5 F2** | QIQLVQSGPELKKPGETVKISCKASGYT FINYGMNWVKQTPGKGLKWMGWIN TYTGEPTYADDFKGRFAFSLETSASTA YLQINNLKNEDMATYFCSREEFYSRG AMDYWGQGTSVTVSS (SEQ ID NO: 149) | DVLMTQTPLSLPVSLGDQASISCRSS QFIVHSNGNTYLEWYLQKPGQSP KLLIYKISNRFSGVPDRFSGSGSGTD FTLKISRVEAEDLGVYYCFQGSHVP FTFGSGTKLEIK (SEQ ID NO: 150) |
| **20-2-55** | **136C1 1E7** | QIQLQQSGPELVKPGASVKISCKASGYS FTDYYINWVKQKPGQGLEWIGWIYP GSGNTKYNEKFKGKATLTVDTSSSTA YMQLNSLTSEDTAVYFCARSSRCDFWG QGTTLTVSS (SEQ ID NO: 151) | DIQMTQSASSFSVSLGDSVTITCKAS EDIYNRLAWYQQKPGNTPRLLISG ATSLETGVPSRFSGSGSGKDYTLSIT SLQTEDVATYYCQQYWSSPLTFGSG TKLELK (SEQ ID NO: 152) |

| | | | |
|---|---|---|---|
| Note: Different antibody sequences were obtained when the single clone 20-2-40 was sequenced, which were recorded as 20-2-40-1 and 20-2-40-2 respectively. | | | |

### 3. Sequences of CAR structures

| Name | Nucleotide sequence | Amino acid sequence |
|---|---|---|
| CD8a signal peptide | ATGGCCCTCCCTGTCACCGCCCTGCT GCTTCCGCTGGCTCTTCTGCTCCACGC CGCTCGGCCC (SEQ ID NO: 153) | MALPVTALLLPLAL LLHAARP (SEQ ID NO: 154) |
| CD8 Hinge | ACCACGACGCCAGCGCCGCGACCACCAAC ACCGGCGCCCACCATCGCGTCGCAGCCCCT GTCCCTGCGCCCAGAGGCGTGCCGGCCAG CGGCGGGGGGCGCAGTGCACACGAGGGGG CTGGACTTCGCCTGTGAT (SEQ ID NO: 155) | TTTPAPRPPTPAPTIA SQPLSLRPEACRPAA GGAVHTRGLDFACD (SEQ ID NO: 156) |
| CD8a transmembrane region | ATCTACATCTGGGCGCCCTTGGCCGGGACT TGTGGGGTCCTTCTCCTGTCACTGGTTATCA CCCTTTACTGC (SEQ ID NO: 157) | IYIWAPLAGTCGVL LLSLVITLYC (SEQ ID NO: 158) |
| 41BB co-stimulatory domain (which was used in the Example) | AAACGGGGCAGAAAGAAACTCCTGTATATA TTCAAACAACCATTTATGAGACCAGTACAA ACTACTCAAGAGGAAGATGGCTGTAGCTGC CGATTTCCAGAAGAAGAAGAAGGAGGATG TGAACTG (SEQ ID NO: 159) | KRGRKKLLYIFKQP FMRPVQTTQEEDGC SCRFPEEEEGGCEL (SEQ ID NO: 160) |
| CD28 co-stimulatory domain | TCTAAGCGGTCCAGACTGCTCCACAGTGAT TACATGAACATGACCCCACGCAGGCCTGGG CCAACACGAAAACACTATCAGCCTTACGCA CCTCCCCGCGACTTTGCTGCCTACCGCTCT (SEQ ID NO: 161) | SKRSRLLHSDYMN MTPRRPGPTRKHYQ PYAPPRDFAAYRS (SEQ ID NO: 162) |
| cD3ζ activation domain | AGAGTGAAGTTCAGCAGGAGCGCAGACGC CCCCGCGTACAAGCAGGGCCAGAACCAGC TCTATAACGAGCTCAATCTAGGACGAAGAG AGGAGTACGATGTTTTGGACAAGAGACGTG GCCGGGACCCTGAGATGGGGGGAAAGCCG AGAAGGAAGAACCCTCAGGAAGGCCTGTA CAATGAACTGCAGAAAGATAAGATGGCGG AGGCCTACAGTGAGATTGGGATGAAAGGC GAGCGCCGGAGGGGCAAGGGGCACGATGG CCTTTACCAGGGTCTCAGTACAGCCACCAA GGACACCTACGACGCCCTTCACATGCAGGC CCTGCCCCCTCGC (SEQ ID NO: 163) | RVKFSRSADAPAYK QGQNQLYNELNLG RREEYDVLDKRRG RDPEMGGKPRRKN PQEGLYNELQKDK MAEAYSEIGMKGER RRGKGHDGLYQGL STATKDTYDALHM QALPPR (SEQ ID NO: 164) |
| linker | GGCGGAGGCGGGTCAGGAGGTGGCGGCAG CGGAGGAGGAGGGTCCGGCGGAGGTGGGT CC (SEQ ID NO: 165) | GGGGSGGGGSGGG GSGGGGS (SEQ ID NO: 166) |

### 4. Study results of in vitro efficacies of CAR T cells prepared with murine antibodies

### 4.1 CAR-positive rates of CAR T cells prepared with murine antibodies

Flow cytometry was used to determine the CAR-positive rates of CAR T cells. Results showed that the CAR-positive rates of CAR T cells prepared with the murine antibody sequences were between 50%-90% (FIG 5).

### 4.2 Results of killing efficiencies of CAR T cells prepared with murine antibodies

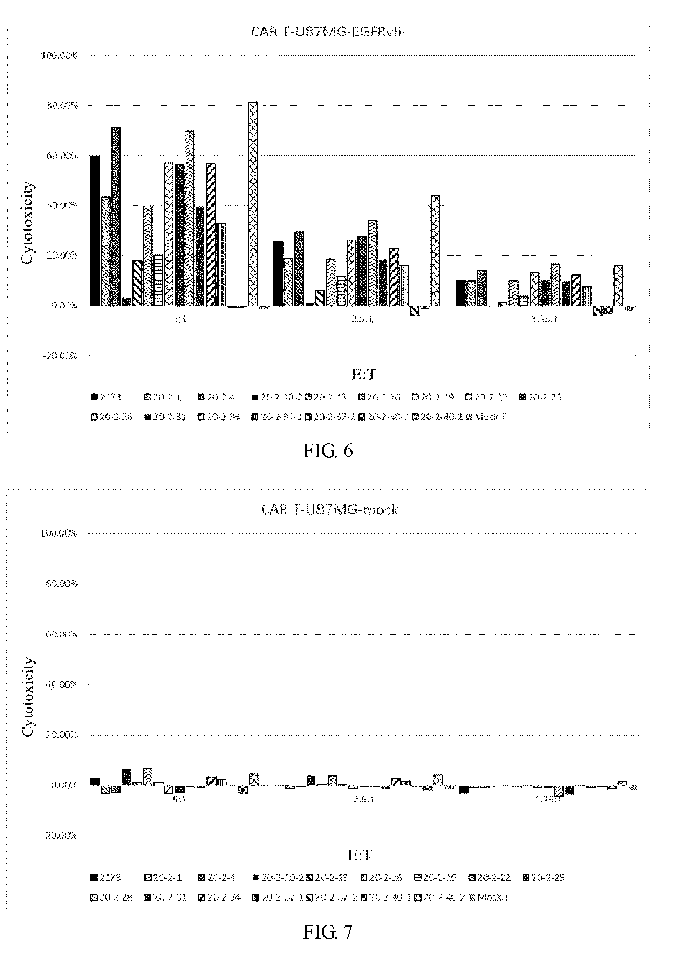

The CAR T cells prepared with the murine antibody sequences were tested for their killing efficiencies against the target cells U87MG-EGFRvIII overexpressing EGFRvIII and U87MG-EGFR expressing EGFR respectively. The results showed that all the prepared CAR T cells specifically killed the target cells overexpressing EGFRvIII, but had almost no killing efficiencies against the target cells expressing EGFR. This demonstrated that the CAR T cells prepared with the developed antibodies had specific killing efficiencies ( FIGs. 6 and 7). Among them, the 20-2-1, 20-2-4, 20-2-25, 20-2-28, 20-2-34 and 20-2-40-2 had relatively high killing efficiencies.

### 5. In vivo tumor inhibition efficiencies of CAR T cells prepared with murine antibodies

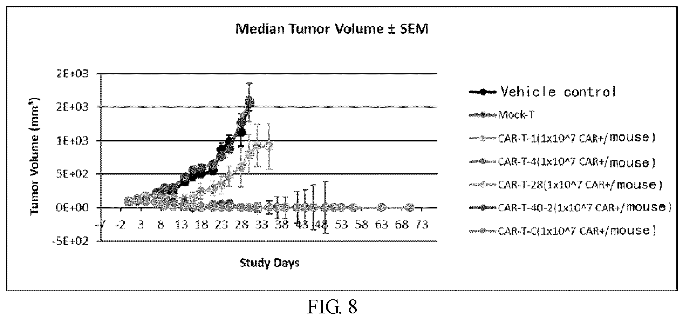

The U87MG-EGFRvIII was used to form a subcutaneous tumor in a mouse. When the tumor grew to a volume of about 100 mm³, the CAR T cells were reinfused. After the reinfusion, the mouse tumor volume was measured every other week. The results showed that the CAR T cells prepared with the antibody sequences from clones #4, #28, and #40-2 had significant tumor inhibition effects *in vivo* (FIG 8).

### 6. Determination results of affinities of humanized antibodies

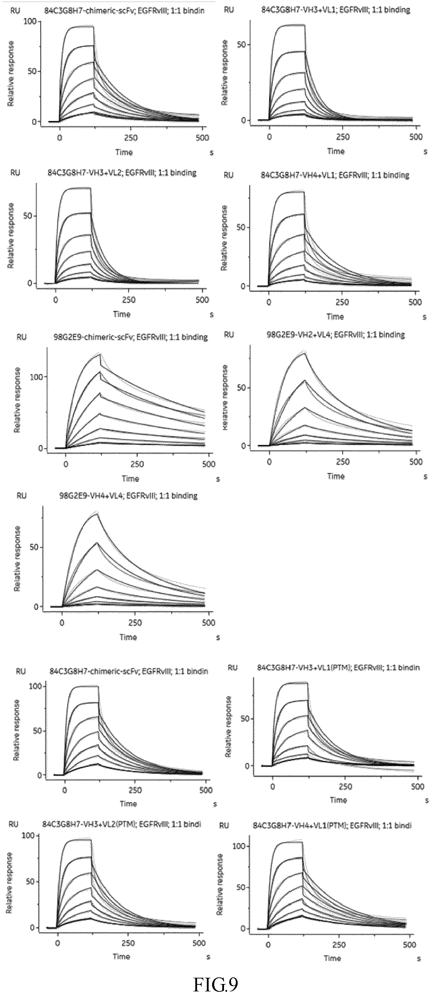

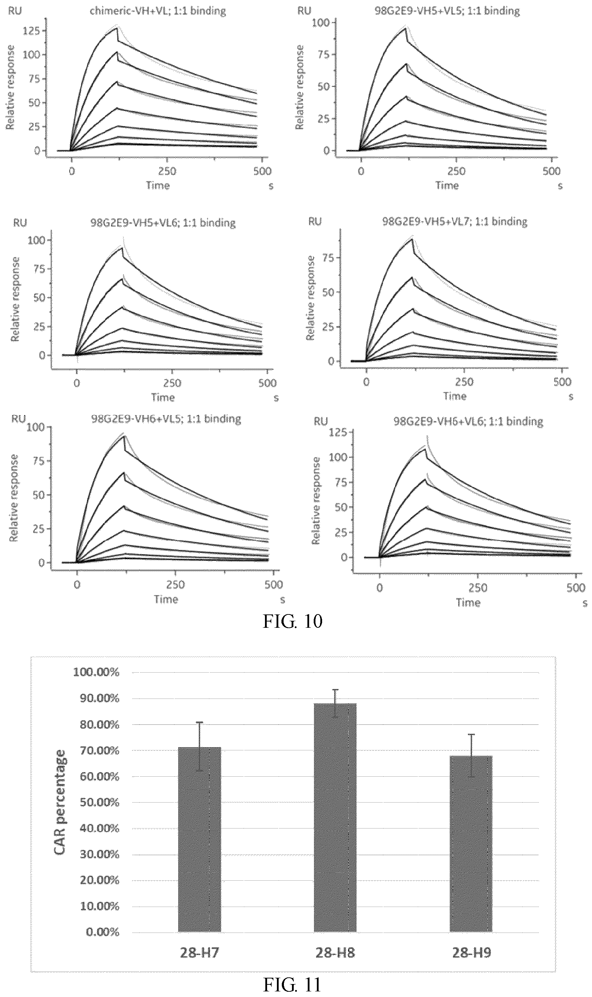

The murine antibodies were humanized through CDR grafting and removal of post-translational modification sites. The results showed that after humanization ofclone #28, a humanized antibody sequence having an affinity consistent with that of the murine antibody was obtained. The affinity of the murine antibody from clone #40-2 was reduced by about 2-fold after humanization (Tables 2 and 3, FIGs. 9 and 10).

**Table 2 Affinities of the purified humanized antibodies from clone #28 as determined by the SPR method**

| **Number** | **Analyte** | **Chi² (RU²)** | **ka (1/Ms)** | **kd (1/s)** | **KD (M)** | **Rmax (RU)** |
|---|---|---|---|---|---|---|
| 20-2-28 | 84C3G8H7-chimeric-scFv | 5.03E+00 | 1.50E+05 | 8.83E-03 | 5.90E-08 | 97.9 |
| 28-H3 | 84C3G8H7-VH3+VL1 | 1.27E+00 | 1.32E+05 | 2.21E-02 | 1.67E-07 | 85.3 |
| 28-H4 | 84C3G8H7-VH3+VL2 | 2.51E+00 | 1.34E+05 | 2.06E-02 | 1.54E-07 | 92.3 |
| 28-H5 | 84C3G8H7-VH4+VL1 | 4.73E+00 | 1.11E+05 | 8.91E-03 | 8.03E-08 | 92.9 |
| 28-H7 | 84C3G8H7-VH3+VL1(PTM) | 5.47E+00 | 1.46E+05 | 1.12E-02 | 7.72E-08 | 97.4 |
| 28-H8 | 84C3G8H7-VH3+VL2(PTM) | 4.12E+00 | 1.42E+05 | 1.03E-02 | 7.26E-08 | 101 |
| 28-H9 | 84C3G8H7-VH4+VL1(PTM) | 7.12E+00 | 1.40E+05 | 6.65E-03 | 4.75E-08 | 101.4 |

**Table 3 Affinities of the purified humanized antibodies from clone #40-2 as determined by the SPR method**

| **Number** | **Analyte** | **Chi² (RU²)** | **ka (1/Ms)** | **kd (1/s)** | **KD (M)** | **Rmax** (RU) |
|---|---|---|---|---|---|---|
| 20-2-40-2 | chimeric-VH+VL | 3.25E+00 | 3.62E+04 | 1.81E-03 | 4.99E-08 | 125.4 |
| 40-2-H9 | 98G2E9-VH5+VL5 | 1.59E+00 | 2.74E+04 | 3.07E-03 | 1.12E-07 | 112.3 |
| 40-2-H10 | 98G2E9-VH5+VL6 | | 2.56E+04 | 3.45E-03 | 1.35E-07 | 109.7 |
| 40-2-H11 | 98G2E9-VH5+VL7 | | 2.48E+04 | 3.43E-03 | 1.38E-07 | 105.1 |
| 40-2-H12 | 98G2E9-VH6+VL5 | 1.49E+00 | 2.60E+04 | 2.66E-03 | 1.02E-07 | 103.7 |
| 40-2-H13 | 98G2E9-VH6+VL6 | 2.80E+00 | 2.94E+04 | 3.01E-03 | 1.02E-07 | 123 |

### 7. Sequences of humanized antibodies

| Numb er | Amino acid sequence | Nucleotide sequence |
|---|---|---|
| 28-H3 | QVQLVQSGAEVKKPGAS VKVSCKASGFNIKDYYM HWVRQAPGQRLEWIGWI DPENGNTIYDPKFQGRATI TADTSASTAYMELSSLRSE DTAVYYCARGWFAYWGQ GTLVTVSSggggsggggsgggg sggggsDIVMTQSPDSLAVS LGERATINCKSSQSLLNSS NQKNHLAWYQQKPGQPP KLLIYFASTRASGVPDRFS GSGSGTDFTLTISSLQAED VAVYYCQQHYSTPLTFGG GTKLELK (SEQ ID NO: 167) | CAAGTGCAGCTGGTGCAGTCCGGCGCTGAAG TGAAGAAGCCCGGCGCCTCCGTGAAAGTGTC CTGCAAGGCCTCTGGCTTCAACATCAAGGAC TACTATATGCACTGGGTGCGGCAGGCTCCTGG CCAGCGGCTGGAGTGGATCGGCTGGATCGAC CCTGAGAACGGCAACACCATCTACGACCCCA AGTTCCAGGGCAGAGCCACCATCACCGCCGA CACCAGCGCCAGCACTGCTTATATGGAACTG AGCTCTCTGAGAAGCGAGGATACCGCTGTCT ACTACTGTGCCAGAGGCTGGTTCGCCTACTG GGGCCAAGGCACCCTCGTCACAGTGTCTAGC GGAGGGGGCGGATCTGGGGGCGGCGGCTCC GGAGGAGGCGGTTCTGGCGGTGGCGGCTCG GACATCGTGATGACACAGTCTCCTGATAGCCT GGCTGTGTCCCTGGGCGAGCGGGCAACCATC AACTGCAAGTCCAGCCAGAGCCTGCTGAACA GCAGCAATCAGAAGAACCACCTGGCCTGGTA CCAGCAGAAACCAGGCCAGCCTCCTAAGCTG CTGATTTACTTTGCCTCTACCAGAGCCAGCGG CGTGCCCGATAGATTCAGCGGCTCCGGATCCG GCACCGACTTCACCCTGACAATCTCTAGCCTG CAGGCCGAGGACGTGGCCGTGTACTACTGCC AGCAGCACTACTCCACCCCTCTGACCTTCGG CGGCGGAACAAAGCTGGAACTGAAG (SEQ ID NO: 168) |
| 28-H4 | QVQLVQSGAEVKKPGAS VKVSCKASGFNIKDYYM HWVRQAPGQRLEWIGWI DPENGNTIYDPKFQGRATI TADTSASTAYMELSSLRSE DTAVYYCARGWFAYWGQ GTLVTVSSggggsggggsgggg sggggsDIVMTQSPDSLAVS LGERATINCKSSQSLLNSS NQKNHLAWYQQKPGQPP | CAAGTGCAGCTGGTGCAGTCTGGGGCCGAGG TGAAGAAGCCCGGCGCTAGCGTGAAAGTGTC CTGCAAGGCTAGCGGCTTCAACATCAAGGAC TACTACATGCACTGGGTGCGGCAGGCCCCTG GACAGAGACTGGAATGGATCGGCTGGATCGA CCCCGAGAACGGCAACACCATCTACGACCCT AAGTTCCAGGGCAGAGCCACAATCACCGCCG ATACAAGCGCCTCTACTGCCTACATGGAACTG AGCTCTCTGCGGTCTGAAGATACCGCTGTCTA CTACTGCGCCAGAGGCTGGTTCGCCTACTGG |
| | KLLVYFASTRASGVPDRF SGSGSGTDFTLTISSLQAE DVAVYFCQQHYSTPLTFG GGTKLELK (SEQ ID NO: 169) | GGCCAGGGCACACTGGTGACAGTGTCCTCGG GCGGTGGAGGCTCCGGCGGCGGCTCTGG CGGCGGTGGCAGCGGAGGAGGCGGATCCGA CATCGTGATGACCCAGTCCCCCGACAGCCTG GCTGTGTCTCTGGGCGAGCGGGCCACCATCA ACTGCAAGTCCTCTCAGTCCCTGCTGAACAG CAGCAATCAGAAGAACCACCTGGCCTGGTAC CAGCAAAAACCTGGCCAGCCTCCAAAGCTGC TGGTCTATTTTGCTTCTACCAGAGCCTCCGGC GTGCCTGATAGATTCTCCGGCAGCGGCTCCG GCACCGACTTCACCCTCACCATCAGCAGCCT GCAGGCTGAGGACGTGGCCGTGTACTTCTGT CAGCAGCACTACAGCACCCCTCTGACCTTCG GCGGAGGCACCAAGCTGGAGCTGAAG (SEQ ID NO: 170) |
| 28-H5 | QVQLVQSGAEVKKPGAS VKVSCKASGFNIKDYYM HWVKQAPGQRLEWIGWI DPENGNTIYDPKFQGRATI TADTSANTAYMELSSLRS EDTAVYYCARGWFAYWG QGTLVTVSSggggsggggsggg gsggggsDIVMTQSPDSLAV SLGERATINCKSSQSLLNS SNQKNHLAWYQQKPGQP PKLLIYFASTRASGVPDRF SGSGSGTDFTLTISSLQAE DVAVYYCQQHYSTPLTFG GGTKLELK (SEQ ID NO: 171) | CAAGTGCAGCTCGTCCAGTCTGGCGCCGAAG TGAAGAAGCCCGGCGCTTCCGTGAAAGTGTC CTGCAAGGCCTCTGGCTTCAACATCAAGGAC TACTACATGCACTGGGTCAAGCAGGCCCCTG GCCAGCGGCTGGAATGGATTGGCTGGATCGA CCCTGAGAACGGCAACACCATCTACGACCCC AAGTTCCAGGGCAGAGCCACTATCACCGCCG ATACAAGCGCCAATACAGCTTATATGGAACTG TCCTCCCTGAGAAGCGAGGATACAGCCGTGT ACTACTGCGCCAGAGGCTGGTTCGCCTACTG GGGCCAAGGCACCCTGGTGACCGTGTCTTCT GGCGGCGGCGGCTCTGGCGGCGGAGGATCTG GCGGAGGCGGCAGTGGCGGAGGCGGAAGCG ACATCGTGATGACCCAGTCCCCAGATTCCCTG GCCGTGTCTCTGGGCGAGCGGGCTACCATCA ACTGCAAGTCCAGCCAGAGCCTGCTGAACAG CAGCAACCAGAAGAACCACCTGGCCTGGTAC CAGCAGAAACCCGGCCAGCCTCCTAAGCTGC TGATCTACTTTGCTTCCACCAGAGCTAGCGGC GTGCCTGACCGGTTCAGCGGCTCCGGTTCTG GAACAGACTTCACCCTGACCATCAGCTCCCT GCAGGCCGAGGACGTGGCCGTGTACTACTGT CAGCAGCACTACAGCACCCCTCTGACCTTCG |
| | | GCGGCGGCACCAAGCTGGAGCTGAAG (SEQ ID NO: 172) |
| 28-H7 | QVQLVQSGAEVKKPGAS VKVSCKASGFNIKDYYM HWVRQAPGQRLEWIGWI DPENGNTIYDPKFQGRATI TADTSASTAYMELSSLRSE DTAVYYCARGWFAYWGQ GTLVTVSSGGGGSGGGGS GGGGSGGGGSDIVMTQSP DSLAVSLGERATINCKSSQ SLLNSSNQKNHLAWYQQ KPGQPPKLLIYFASTRGSG VPDRFSGSGSGTDFTLTIS SLQAEDVAVYYCQQHYST PLTFGGGTKLELK (SEQ ID NO: 173) | CAAGTGCAGCTGGTGCAGTCCGGCGCTGAAG TGAAGAAGCCCGGCGCCTCCGTGAAAGTGTC CTGCAAGGCCTCTGGCTTCAACATCAAGGAC TACTATATGCACTGGGTGCGGCAGGCTCCTGG CCAGCGGCTGGAGTGGATCGGCTGGATCGAC CCTGAGAACGGCAACACCATCTACGACCCCA AGTTCCAGGGCAGAGCCACCATCACCGCCGA CACCAGCGCCAGCACTGCTTATATGGAACTG AGCTCTCTGAGAAGCGAGGATACCGCTGTCT ACTACTGTGCCAGAGGCTGGTTCGCCTACTG GGGCCAAGGCACCCTCGTCACAGTGTCTAGC GGAGGGGGCGGATCTGGGGGCGGCGGCTCC GGAGGAGGCGGTTCTGGCGGTGGCGGCTCG GACATCGTGATGACACAGTCTCCTGATAGCCT GGCTGTGTCCCTGGGCGAGCGGGCAACCATC AACTGCAAGTCCAGCCAGAGCCTGCTGAACA GCAGCAATCAGAAGAACCACCTGGCCTGGTA CCAGCAGAAACCAGGCCAGCCTCCTAAGCTG CTGATTTACTTTGCCTCTACCAGAGGCAGCGG CGTGCCCGATAGATTCAGCGGCTCCGGATCCG GCACCGACTTCACCCTGACAATCTCTAGCCTG CAGGCCGAGGACGTGGCCGTGTACTACTGCC AGCAGCACTACTCCACCCCTCTGACCTTCGG CGGCGGAACAAAGCTGGAACTGAAG (SEQ ID NO: 174) |
| 28-H8 | QVQLVQSGAEVKKPGAS VKVSCKASGFNIKDYYM HWVRQAPGQRLEWIGWI DPENGNTIYDPKFQGRATI TADTSASTAYMELSSLRSE DTAVYYCARGWFAYWGQ GTLVTVSSGGGGSGGGGS GGGGSGGGGSDIVMTQSP DSLAVSLGERATINCKSSQ SLLNSSNQKNHLAWYQQ KPGQPPKLLVYFASTRGS GVPDRFSGSGSGTDFTLTI SSLQAEDVAVYFCQQHYS TPLTFGGGTKLELK (SEQ ID NO: 175) | CAAGTGCAGCTGGTGCAGTCTGGGGCCGAGG TGAAGAAGCCCGGCGCTAGCGTGAAAGTGTC CTGCAAGGCTAGCGGCTTCAACATCAAGGAC TACTACATGCACTGGGTGCGGCAGGCCCCTG GACAGAGACTGGAATGGATCGGCTGGATCGA CCCCGAGAACGGCAACACCATCTACGACCCT AAGTTCCAGGGCAGAGCCACAATCACCGCCG ATACAAGCGCCTCTACTGCCTACATGGAACTG AGCTCTCTGCGGTCTGAAGATACCGCTGTCTA CTACTGCGCCAGAGGCTGGTTCGCCTACTGG GGCCAGGGCACACTGGTGACAGTGTCCTCGG GCGGTGGAGGCTCCGGCGGCGGCGGCTCTGG CGGCGGTGGCAGCGGAGGAGGCGGATCCGA CATCGTGATGACCCAGTCCCCCGACAGCCTG GCTGTGTCTCTGGGCGAGCGGGCCACCATCA ACTGCAAGTCCTCTCAGTCCCTGCTGAACAG CAGCAATCAGAAGAACCACCTGGCCTGGTAC CAGCAAAAACCTGGCCAGCCTCCAAAGCTGC TGGTCTATTTTGCTTCTACCAGAGGCTCCGGC GTGCCTGATAGATTCTCCGGCAGCGGCTCCG GCACCGACTTCACCCTCACCATCAGCAGCCT GCAGGCTGAGGACGTGGCCGTGTACTTCTGT CAGCAGCACTACAGCACCCCTCTGACCTTCG GCGGAGGCACCAAGCTGGAGCTGAAG (SEQ ID NO: 176) |
| 28-H9 | QVQLVQSGAEVKKPGAS VKVSCKASGFNIKDYYM HWVKQAPGQRLEWIGWI DPENGNTIYDPKFQGRATI TADTSANTAYMELSSLRS EDTAVYYCARGWFAYWG QGTLVTVSSGGGGSGGG GSGGGGSGGGGSDIVMT QSPDSLAVSLGERATINCK SSQSLLNSSNQKNHLAWY QQKPGQPPKLLIYFASTRG SGVPDRFSGSGSGTDFTLT ISSLQAEDVAVYYCQQHY | CAAGTGCAGCTCGTCCAGTCTGGCGCCGAAG TGAAGAAGCCCGGCGCTTCCGTGAAAGTGTC CTGCAAGGCCTCTGGCTTCAACATCAAGGAC TACTACATGCACTGGGTCAAGCAGGCCCCTG GCCAGCGGCTGGAATGGATTGGCTGGATCGA CCCTGAGAACGGCAACACCATCTACGACCCC AAGTTCCAGGGCAGAGCCACTATCACCGCCG ATACAAGCGCCAATACAGCTTATATGGAACTG TCCTCCCTGAGAAGCGAGGATACAGCCGTGT ACTACTGCGCCAGAGGCTGGTTCGCCTACTG GGGCCAAGGCACCCTGGTGACCGTGTCTTCT GGCGGCGGCGGCTCTGGCGGCGGAGGATCTG GCGGAGGCGGCAGTGGCGGAGGCGGAAGCG |
| | STPLTFGGGTKLELK (SEQ ID NO: 177) | ACATCGTGATGACCCAGTCCCCAGATTCCCTG GCCGTGTCTCTGGGCGAGCGGGCTACCATCA ACTGCAAGTCCAGCCAGAGCCTGCTGAACAG CAGCAACCAGAAGAACCACCTGGCCTGGTAC CAGCAGAAACCCGGCCAGCCTCCTAAGCTGC TGATCTACTTTGCTTCCACCAGAGGTAGCGGC GTGCCTGACCGGTTCAGCGGCTCCGGTTCTG GAACAGACTTCACCCTGACCATCAGCTCCCT GCAGGCCGAGGACGTGGCCGTGTACTACTGT CAGCAGCACTACAGCACCCCTCTGACCTTCG GCGGCGGCACCAAGCTGGAGCTGAAG (SEQ ID NO: 178) |
| 40-2-H9 | EVQLLESGGGLVQPGGSL KLSCAASGFTFSGYAMSW VRQTPGKGLEWVATITSG GSYTYYPDSVKGRFTISR DNSKNTLYLQMNSLRAE DTAMYYCARKDYGNYW FAYWGQGTLVTVSSGGG GSGGGGSGGGGSGGGGS DVVMTQSPLSLPVTLGDP ASISCRSSQSLVHSDGNTY LHWYLQRPGQSPRLLIYK VSNRFSGVPDRFSGSGSG TDFTLKISRVEAEDVGVY FCSQTTQVPWTFGGGTKL EIK (SEQ ID NO: 179) | GAGGTGCAGCTGCTGGAAAGCGGCGGAGGA CTGGTGCAACCTGGAGGATCTCTGAAGCTGA GCTGCGCCGCTTCTGGCTTTACATTCAGCGGC TACGCCATGAGCTGGGTCAGACAGACCCCTG GCAAGGGCCTCGAGTGGGTGGCCACAATCAC CAGCGGCGGCAGCTACACCTACTACCCCGAC AGCGTGAAAGGCAGATTCACCATCAGCCGGG ACAATTCTAAGAACACCCTGTACCTGCAGATG AACTCCCTGCGGGCCGAAGATACCGCCATGT ACTATTGTGCTAGAAAGGACTACGGCAACTA CTGGTTCGCCTACTGGGGCCAGGGCACCCTG GTGACAGTGTCCAGCGGCGGCGGCGGCTCTG GCGGCGGCGGAAGCGGCGGCGGGGGTTCCG GCGGAGGAGGCTCCGATGTGGTGATGACCCA AAGCCCTCTGAGCCTGCCTGTGACCCTGGGC GACCCCGCCAGCATCTCTTGTAGAAGCAGCC AGTCCCTCGTGCACAGCGACGGCAACACCTA CCTGCACTGGTACCTGCAGCGGCCTGGCCAG TCTCCAAGACTGCTGATCTATAAGGTGTCCAA CCGGTTCAGCGGAGTTCCTGATAGATTTAGCG GCAGCGGCTCTGGAACAGACTTCACACTGAA AATCTCTAGAGTGGAGGCCGAGGACGTGGGC GTCTACTTCTGCAGCCAGACCACACAGGTGC CCTGGACCTTCGGCGGAGGCACCAAGCTGGA AATCAAG (SEQ ID NO: 180) |
| 40-2-H10 | EVQLLESGGGLVQPGGSL KLSCAASGFTFSGYAMSW VRQTPGKGLEWVATITSG GSYTYYPDSVKGRFTISR DNSKNTLYLQMNSLRAE DTAMYYCARKDYGNYW FAYWGQGTLVTVSSGGG GSGGGGSGGGGSGGGGS DVVMTQSPLSLPVTLGQQ ASISCRSSQSLVHSDGNTY LHWYLQKPGQSPKLLIYK VSNRFSGVPDRFSGSGSG TDFTLKISRVEAEDVGVY FCSQTTQVPWTFGGGTKL EIK (SEQ ID NO: 181) | GAGGTGCAGCTGCTGGAAAGCGGCGGAGGA CTGGTGCAACCTGGAGGATCTCTGAAGCTGA GCTGCGCCGCTTCTGGCTTTACATTCAGCGGC TACGCCATGAGCTGGGTCAGACAGACCCCTG GCAAGGGCCTCGAGTGGGTGGCCACAATCAC CAGCGGCGGCAGCTACACCTACTACCCCGAC AGCGTGAAAGGCAGATTCACCATCAGCCGGG ACAATTCTAAGAACACCCTGTACCTGCAGATG AACTCCCTGCGGGCCGAAGATACCGCCATGT ACTATTGTGCTAGAAAGGACTACGGCAACTA CTGGTTCGCCTACTGGGGCCAGGGCACCCTG GTGACAGTGTCCAGCGGCGGCGGCGGCTCTG GCGGCGGCGGAAGCGGCGGCGGGGGTTCCG GCGGAGGAGGCTCCGACGTCGTGATGACACA GAGCCCCCTGAGCCTGCCTGTGACCCTGGGC CAGCAGGCCAGCATCAGCTGTAGAAGCAGCC AGTCCCTGGTGCACTCCGACGGCAACACCTA CCTGCACTGGTACCTGCAGAAACCTGGCCAG TCTCCAAAGCTGCTGATCTACAAGGTGTCCA ACCGGTTTAGCGGCGTTCCTGATAGATTCAGC GGATCTGGCAGCGGCACAGATTTCACCCTGA AGATCTCTAGAGTGGAAGCCGAGGACGTGGG CGTGTATTTCTGCAGCCAGACCACCCAAGTG CCCTGGACCTTCGGCGGAGGCACAAAGCTCG AGATCAAG (SEQ ID NO: 182) |
| 40-2-H11 | EVQLLESGGGLVQPGGSL KLSCAASGFTFSGYAMSW VRQTPGKGLEWVATITSG GSYTYYPDSVKGRFTISR DNSKNTLYLQMNSLRAE DTAMYYCARKDYGNYW FAYWGQGTLVTVSSGGG GSGGGGSGGGGSGGGGS DVVMTQSPLSLPVTLGDQ ASISCRSSQSLVHSDGNTY LHWYLQKPGQSPKLLIYK VSNRFSGVPDRFSGSGSG TDFTLKISRVEAEDVGVY | GAGGTGCAGCTGCTGGAAAGCGGCGGAGGA CTGGTGCAACCTGGAGGATCTCTGAAGCTGA GCTGCGCCGCTTCTGGCTTTACATTCAGCGGC TACGCCATGAGCTGGGTCAGACAGACCCCTG GCAAGGGCCTCGAGTGGGTGGCCACAATCAC CAGCGGCGGCAGCTACACCTACTACCCCGAC AGCGTGAAAGGCAGATTCACCATCAGCCGGG ACAATTCTAAGAACACCCTGTACCTGCAGATG AACTCCCTGCGGGCCGAAGATACCGCCATGT ACTATTGTGCTAGAAAGGACTACGGCAACTA CTGGTTCGCCTACTGGGGCCAGGGCACCCTG GTGACAGTGTCCAGCGGCGGCGGCGGCTCTG GCGGCGGCGGAAGCGGCGGCGGGGGTTCCG |
| | FCSQTTQVPWTFGGGTKL EIK (SEQ ID NO: 183) | GCGGAGGAGGCTCCGATGTGGTGATGACCCA GTCCCCACTGAGCCTGCCTGTGACCCTGGGC GACCAGGCCAGCATCAGCTGTAGAAGCAGCC AATCTCTGGTGCACAGCGACGGCAACACATA CCTGCACTGGTATCTGCAGAAACCTGGCCAG AGCCCCAAGCTGCTGATCTACAAGGTGTCCA ACCGGTTTAGCGGAGTGCCTGATAGATTCAGC GGCTCTGGCTCCGGCACCGACTTCACCCTCA AGATCTCTAGAGTCGAGGCCGAGGACGTGGG CGTGTACTTCTGCAGCCAGACCACACAGGTG CCCTGGACCTTCGGCGGCGGAACAAAGCTGG AAATCAAG (SEQ ID NO: 184) |
| 40-2-H12 | EVQLLESGGGLVQPGGSL KLSCAASGFTFSGYAMSW VRQTPEKGLEWVATITSG GSYTYYPDSVKGRFTISR DNAKNTLYLQMNSLRAE DTAMYYCARKDYGNYW FAYWGQGTLVTVSSGGG GSGGGGSGGGGSGGGGS DVVMTQSPLSLPVTLGDP ASISCRSSQSLVHSDGNTY LHWYLQRPGQSPRLLIYK VSNRFSGVPDRFSGSGSG TDFTLKISRVEAEDVGVY FCSQTTQVPWTFGGGTKL EIK (SEQ ID NO: 185) | GAGGTGCAGCTGCTGGAAAGCGGCGGCGGA CTCGTGCAACCTGGCGGATCTCTGAAGCTGT CTTGTGCTGCTTCTGGATTTACCTTCAGCGGC TACGCCATGAGCTGGGTGCGGCAGACACCTG AGAAGGGCCTGGAATGGGTCGCCACAATCAC CAGCGGCGGCTCCTACACCTACTACCCCGATA GCGTGAAAGGCAGATTCACAATCAGCCGGGA CAACGCCAAGAACACCCTGTACCTGCAGATG AACAGCCTGAGAGCCGAGGACACCGCCATGT ACTACTGCGCCAGAAAGGACTACGGCAATTA CTGGTTCGCCTATTGGGGCCAGGGCACCCTG GTGACCGTGTCCAGCGGCGGCGGCTCTG GCGGCGGCGGAAGCGGCGGCGGGGGTTCCG GCGGAGGAGGCTCCGATGTGGTGATGACCCA AAGCCCTCTGAGCCTGCCTGTGACCCTGGGC GACCCCGCCAGCATCTCTTGTAGAAGCAGCC AGTCCCTCGTGCACAGCGACGGCAACACCTA CCTGCACTGGTACCTGCAGCGGCCTGGCCAG TCTCCAAGACTGCTGATCTATAAGGTGTCCAA CCGGTTCAGCGGAGTTCCTGATAGATTTAGCG GCAGCGGCTCTGGAACAGACTTCACACTGAA AATCTCTAGAGTGGAGGCCGAGGACGTGGGC GTCTACTTCTGCAGCCAGACCACACAGGTGC CCTGGACCTTCGGCGGAGGCACCAAGCTGGA AATCAAG (SEQ ID NO: 186) |
| 40-2-H13 | EVQLLESGGGLVQPGGSL KLSCAASGFTFSGYAMSW VRQTPEKGLEWVATITSG GSYTYYPDSVKGRFTISR DNAKNTLYLQMNSLRAE DTAMYYCARKDYGNYW FAYWGQGTLVTVSSGGG GSGGGGSGGGGSGGGGS DVVMTQSPLSLPVTLGQQ ASISCRSSQSLVHSDGNTY LHWYLQKPGQSPKLLIYK VSNRFSGVPDRFSGSGSG TDFTLKISRVEAEDVGVY FCSQTTQVPWTFGGGTKL EIK (SEQ ID NO: 187) | GAGGTGCAGCTGCTGGAAAGCGGCGGCGGA CTCGTGCAACCTGGCGGATCTCTGAAGCTGT CTTGTGCTGCTTCTGGATTTACCTTCAGCGGC TACGCCATGAGCTGGGTGCGGCAGACACCTG AGAAGGGCCTGGAATGGGTCGCCACAATCAC CAGCGGCGGCTCCTACACCTACTACCCCGATA GCGTGAAAGGCAGATTCACAATCAGCCGGGA CAACGCCAAGAACACCCTGTACCTGCAGATG AACAGCCTGAGAGCCGAGGACACCGCCATGT ACTACTGCGCCAGAAAGGACTACGGCAATTA CTGGTTCGCCTATTGGGGCCAGGGCACCCTG GTGACCGTGTCCAGCGGCGGCGGCGGCTCTG GCGGCGGCGGAAGCGGCGGCGGGGGTTCCG GCGGAGGAGGCTCCGACGTCGTGATGACACA GAGCCCCCTGAGCCTGCCTGTGACCCTGGGC CAGCAGGCCAGCATCAGCTGTAGAAGCAGCC AGTCCCTGGTGCACTCCGACGGCAACACCTA CCTGCACTGGTACCTGCAGAAACCTGGCCAG TCTCCAAAGCTGCTGATCTACAAGGTGTCCA ACCGGTTTAGCGGCGTTCCTGATAGATTCAGC GGATCTGGCAGCGGCACAGATTTCACCCTGA AGATCTCTAGAGTGGAAGCCGAGGACGTGGG CGTGTATTTCTGCAGCCAGACCACCCAAGTG CCCTGGACCTTCGGCGGAGGCACAAAGCTCG AGATCAAG (SEQ ID NO: 188) |
| 40-2-H15 | EVQLLESGGGLVQPGGSL KLSCAASGFTFSGYAMSW VRQTPEKGLEWVATITSG GSYTYYPDSVKGRFTISR DNAKNTLYLQMNSLRAE DTAMYYCARKDYGNYW FAYWGQGTLVTVSSGGG GSGGGGSGGGGSGGGGS DVVMTQSPLSLPVTLGDQ ASISCRSSQSLVHSDGNTY LHWYLQKPGQSPKLLIYK VSNRFSGVPDRFSGSGSG TDFTLKISRVEAEDVGVY | GAGGTGCAACTGCTGGAATCCGGCGGCGGAC TGGTGCAGCCCGGCGGCTCTCTGAAGCTGAG CTGTGCTGCTTCTGGCTTTACCTTCAGCGGCT ACGCCATGAGCTGGGTCAGACAGACACCTGA GAAGGGCCTGGAATGGGTGGCCACAATCACC AGCGGAGGATCTTATACATACTACCCTGATAG CGTGAAAGGCAGATTCACCATCAGCCGGGAC AACGCCAAGAACACCCTGTACCTGCAGATGA ACAGCCTGCGGGCCGAGGACACCGCCATGTA CTACTGCGCCAGAAAGGACTACGGCAATTAC TGGTTCGCCTACTGGGGCCAGGGCACCCTCG TGACCGTGTCCAGC GGCGGCGGCGGCTCTGGCGGCGGCGGAAGC |
| | FCSQTTQVPWTFGGGTKL EIK (SEQ ID NO: 189) | GGCGGCGGGGGTTCCGGCGGAGGAGGCTCC GATGTGGTTATGACCCAGTCTCCACTGAGCCT GCCTGTGACACTGGGAGATCAGGCCAGCATC AGCTGTAGAAGCAGCCAGTCCCTGGTGCACA GCGACGGCAACACCTACCTGCACTGGTATCT GCAGAAACCTGGCCAGAGCCCCAAGCTGCTG ATCTACAAGGTGTCCAACCGGTTTAGCGGCG TGCCCGACAGATTCTCCGGCAGCGGATCTGG CACCGACTTCACCCTGAAGATCTCTAGAGTG GAAGCCGAGGACGTGGGCGTCTACTTCTGCA GCCAGACCACCCAAGTGCCTTGGACATTCGG CGGCGGAACAAAGCTCGAGATCAAG (SEQ ID NO: 190) |

### 8. Study results of in vitro efficacies of CAR T cells prepared with humanized antibodies

### 8.1 Determination of CAR-positive rates of CAR T cells with humanized antibodies

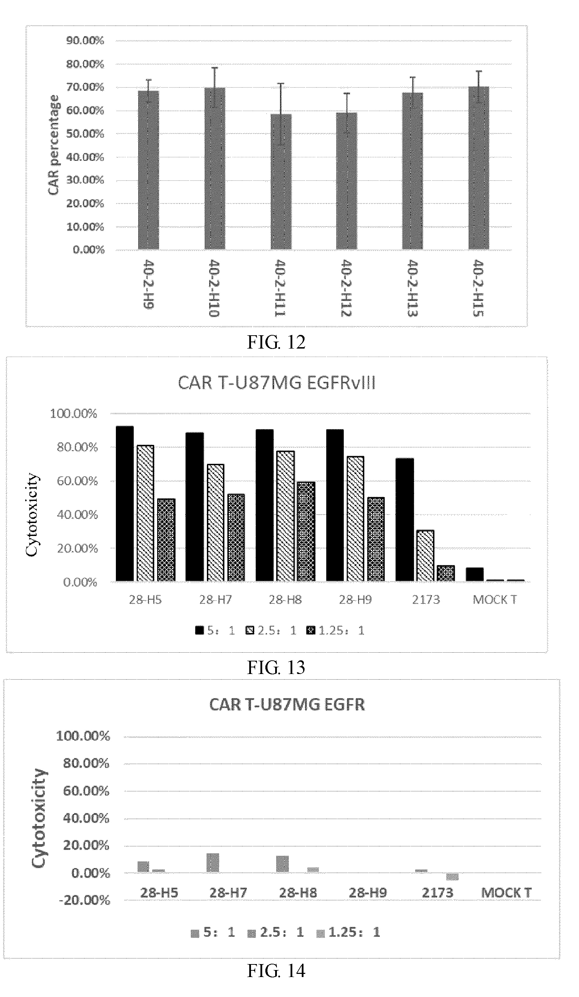

Flow cytometry was used to determine the CAR-positive rates of CAR T cells. Results showed that the CAR-positive rates of CAR T cells prepared with the murine antibody sequences were between 50%-90% (FIGs. 11 and 12).

### 8.2 Determination results of in vitro killing efficiencies of CAR T cells and CAR NK cells with humanized antibodies

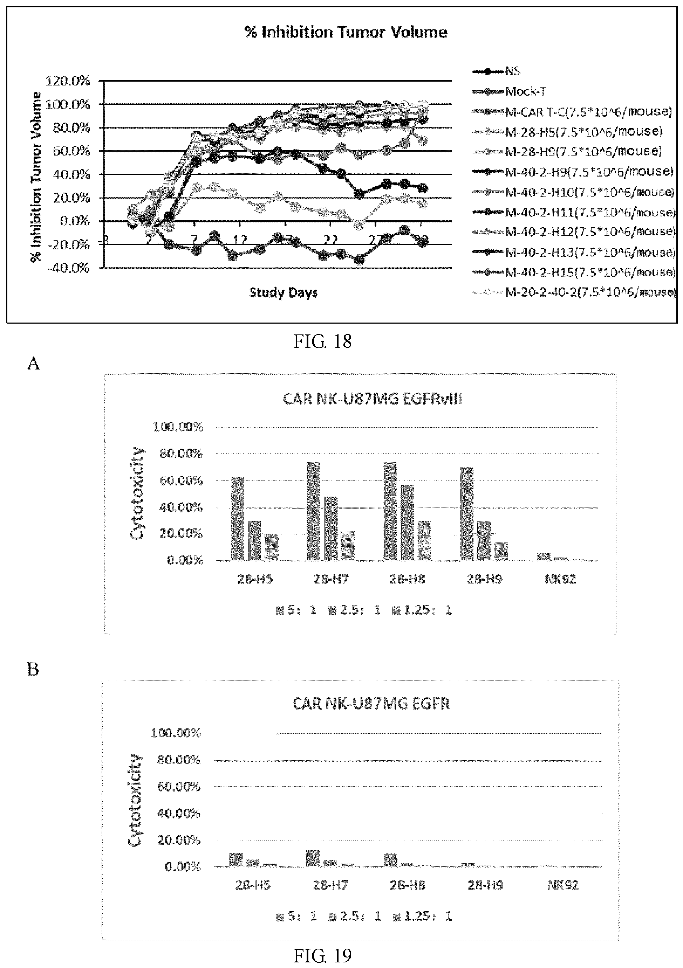

The CAR T cells prepared with the humanized antibody sequences were tested for their killing efficiencies against the target cells U87MG-EGFRvIII overexpressing EGFRvIII and the target cells U87MG-EGFR expressing EGFR respectively. The determination results showed that all the prepared CAR T cells specifically killed the target cells overexpressing EGFRvIII, but had almost no killing efficiencies against the target cells expressing EGFR. This demonstrated that the CAR T cells prepared with the developed humanized antibodies had specific killing efficiencies (FIGs. 13, 14, 15 and 16). Similarly, the CAR NK cells prepared with the human antibody sequences specifically killed the target cells overexpressing EGFRvIII, but had almost no killing efficiencies against target cells expressing EGFR (FIGs. 19A and 19B).

### 9. Study results of in vivo tumor inhibition efficiencies of the CAR T cells prepared with the humanized antibody sequences

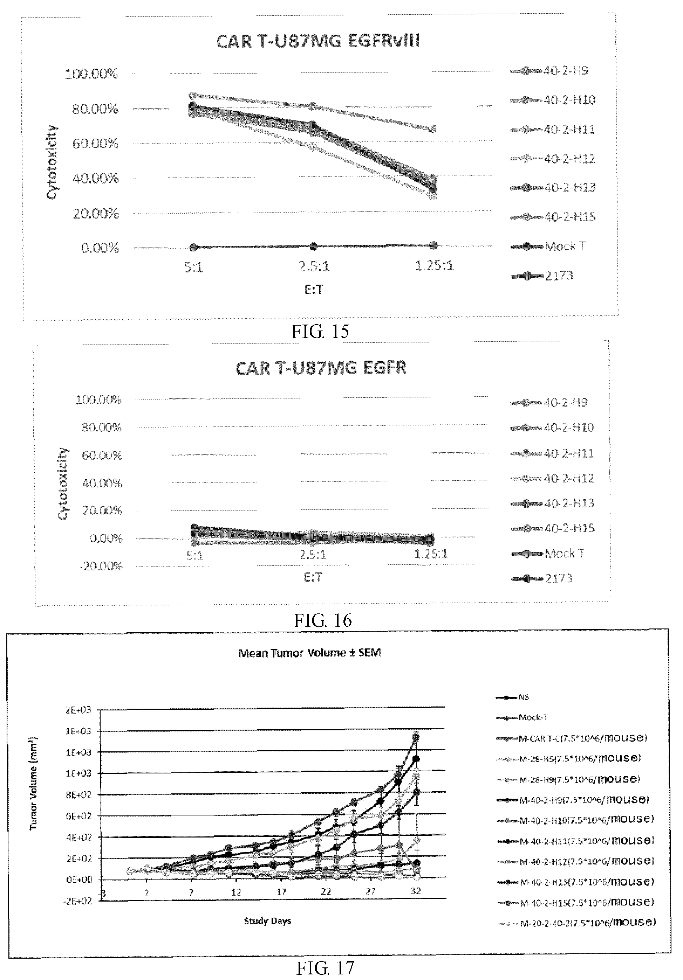

The U87MG-EGFRvIII was used to form a subcutaneous tumor in a mouse. When the tumor grew to a volume of about 100 mm³, the CAR T cells were reinfused. After the reinfusion, the mouse tumor volume was measured every other week. The measurement results showed that the CAR T cells prepared with the antibody sequences from clones #28-H9, #40-2-H10, #40-2-H12, #40-2-H13, and #40-2-H15 had significant tumor inhibition effects *in vivo* (FIGs. 17 and 18).

### 10. Study results of in vitro efficacies of TGFβ-resisting CAR T cells

### 10.1 Determination results of CAR-positive rates

The determination results of the CAR-positive rates were shown in Table 4 and FIGs. 21A-21C. The PE-EGFRvIII antigen protein was used to determine the CAR-positive rates of the RII (CAR T cells expressing dnTGFβRII), the T7R (CAR T cells expressing the chimeric switch receptor dnTGFβRII-IL7Rα) or the CAR T C cells (traditional second-generation CAR T cells expressing no DNR or CSR).

**Table 4. Determination results of CAR-positive rates of the fourth-generation CAR T cells**

| Cell | CAR+ (%) |
|---|---|
| RII | 67.4% |
| T7R | 53.3% |
| CAR T C | 64.4% |

The results showed that the CAR-positive rates of the prepared CAR T cells were more than 50%.

### 10.2 Determination results of in vitro killing efficiencies of CAR T cells

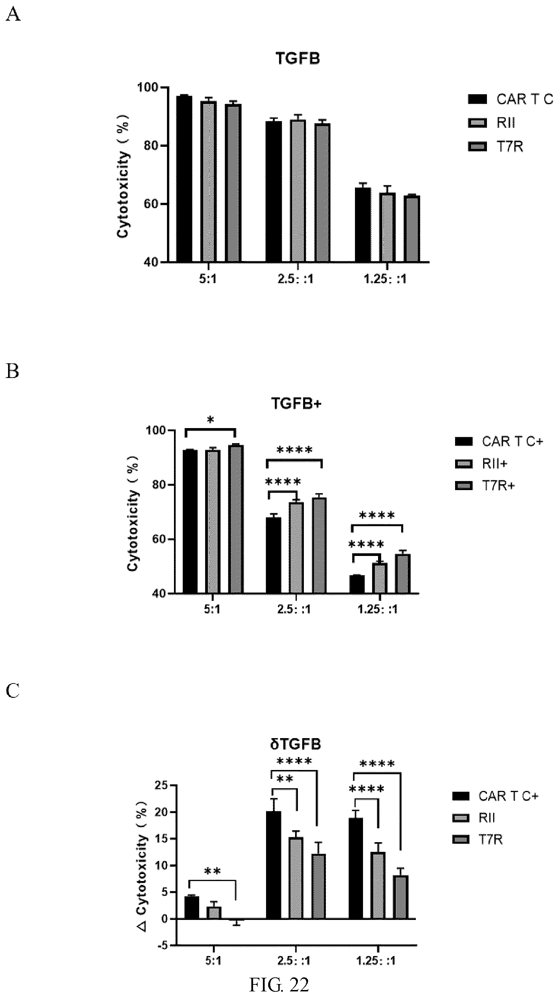

The results showed that when no inhibition by TGEβ was introduced, there was no significant difference observed in the killing efficiencies for the CAR T cells overexpressing TGFβRII and those overexpressing TGFβRII-IL7R (FIG 22A). After introduction of inhibition by TGFβ, the overall killing efficiencies of the CAR T cells decreased significantly (FIG 22B), but the ratio of killing efficiency decrease was significant lower for the CAR T cells overexpressing TGFβRII and those overexpressing TGFβRII-IL21R (FIG 22C).

### 10.3 Determination results of in vivo tumor inhibition effects of CAR T cells

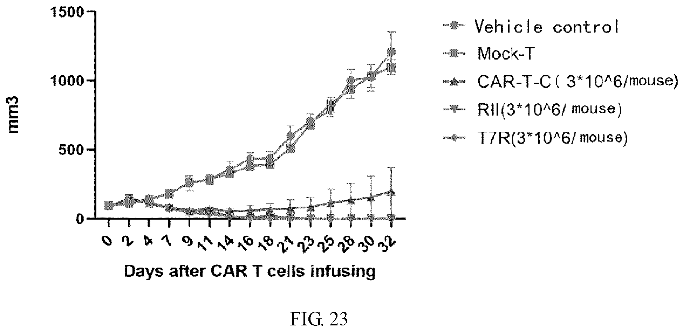

The results showed that the CAR T cells overexpressing TGFβRII and those overexpressing TGFβRII-IL21R had significantly higher *in vivo* tumor inhibition efficiencies than the CAR T cells in the control group (FIG 23).

### Discussion

EGFRvIII is a mutant form of EGFR. It is based on deletion in exons 2-7 of EGFR, and addition of a glycine at the junction of the deleted sequence to form a new antigenic epitope. Therefore, the sequences of EGFRvIII and EGFR have a very high homology, which is a great challenge to antibody sequence screening, since EGFR is expressed in all epithelial cells, while EGFRvIII is a tumor-specific antigen and only expressed on a tumor. For the development of medicaments targeting the EGFRvIII target, it is required to select a medicament which only specifically recognizes the EGFRvIII target antigen and does not recognize the wild-type antigen EGFR. Thus, the specificity of an antibody is required to be high. If an antibody also recognizes the EGFR antigen, there will be an off-target risk. Thus, in the present studies, optimization was carried out in a variety of mouse immunization strategies and antibody screening strategies to select murine antibody sequences with high specificities and high affinities. And the murine sequences could be used to prepare immune cells having a significant tumor inhibition effect. In order to reduce the immunogenicity of the medicament, in the present studies, based on the murine antibody sequences, humanization thereof was carried out, and humanized antibodies having high affinities and high specificities were obtained through screening. Also developed were immune cells prepared with the humanized antibodies, which had a significant tumor inhibition effect. These provided a basis for subsequent development of anti-tumor medicaments.

In recent years, antibody drugs and cellular immunotherapies have achieved significant progress in the field of tumor treatment. Glioma is a highly malignant tumor with no effective treatment at present. Thus, there is an urgent need to develop a medicament that can effectively treat a brain glioma. Researches show that the EGFRvIII antigen is expressed in 30%-60% of brain glioma patients. Thus, the developed EGFRvIII-targeting antibodies and the immune cells prepared with the sequences of these antibodies will be applicable to EGFRvIII-expressing brain gliomas and other EGFRvIII-expressing tumors.

Since an antibody confers a targeting property on a drug, antibody drugs currently come in various forms, including monoclonal antibodies, bispecific antibodies, polyclonal antibodies, ADCs, and immune cells, etc. As a result, the antibodies developed in the present studies can be used in different fields. In addition, since the current targeted therapies require corresponding targeted diagnoses, the antibodies developed in the present studies can also be used in the development of diagnostic kits and diagnosis related applications.

At present, the CAR T cells have made significant progress in blood tumors, and some have been marketed. The CAR structure consists of an antigen recognition domain, a hinge region, a transmembrane region, a co-stimulatory domain and an activation domain. The antibodies developed in the present studies can be assembled with different structures to prepare various types of immune cells, including but not limited to T cells, NK cells, macrophages, monocytes, B cells, and red blood cells. Since the treatment of a solid tumor is limited by the immunosuppressive microenvironment, infiltration, and persistence, different modifications are needed for the immune cells developed, including but not limited to gene editing, gene knockout, and RNAi. These modifications confer upon the CAR T cells *in vivo* persistence, an infiltration property, and an ability to eliminate the suppression by the immunosuppressive microenvironment. Thus, various modifications can be made to the immune cells prepared with the antibodies developed in the present studies to improve their abilities to inhibit tumors.

### REFERENCE

1. Akhavan, D., Alizadeh, D., Wang, D., Weist, M.R., Shepphird, J.K., and Brown, C.E. (2019). CAR T cells for brain tumors: Lessons learned and road ahead. Immunological reviews 290, 60-84.
An, Z., Aksoy, O., Zheng, T., Fan, Q., and Weiss, W.J.O. (2018). Epidermal growth factor receptor and EGFRvIII in glioblastoma: signaling pathways and targeted therapies. 37, 1561-1575.
Chistiakov, D., Chekhonin, I., and Chekhonin, V.J.E.j.o.p. (2017). The EGFR variant III mutant as a target for immunotherapy of glioblastoma multiforme. 810, 70-82.
Del Vecchio, C., Li, G, and Wong, A.J.E.r.o.v. (2012). Targeting EGF receptor variant III: tumor-specific peptide vaccination for malignant gliomas. 11, 133-144.
Feldman, L., Brown, C., and Badie, B. (2022). Chimeric Antigen Receptor (CAR) T Cell Therapy for Glioblastoma. Neuromolecular Med 24, 35-40.
Londhe, V, and Date, V.J.E.r.o.v. (2020). Personalized neoantigen vaccines: a glimmer of hope for glioblastoma. 19, 407-417.
O'Rourke, D.M., Nasrallah, M.P., Desai, A., Melenhorst, J.J., Mansfield, K., Morrissette, J.J.D., Martinez-Lage, M., Brem, S., Maloney, E., Shen, A., et al. (2017). A single dose of peripherally infused EGFRvIII-directed CAR T cells mediates antigen loss and induces adaptive resistance in patients with recurrent glioblastoma. Science translational medicine 9.
Zhu, G, Zhang, Q., Zhang, J., and Liu, F. (2021). Targeting Tumor-Associated Antigen: A Promising CAR-T Therapeutic Strategy for Glioblastoma Treatment. Frontiers in pharmacology 12, 661606.

## Claims

1. An antibody targeting EGFRvIII or antigen-binding fragment thereof, wherein the antibody comprises a heavy chain variable region (HCVR) which includes an HCDR1, an HCDR2, and an HCDR3 selected from one of the following combinations:
(1) HCDR1 whose amino acid sequence is DFSMH (SEQ ID NO: 1);
HCDR2 whose amino acid sequence is WINTETGEPSYADDFKG (SEQ ID NO: 2); and
HCDR3 whose amino acid sequence is YGYDVRGDY (SEQ ID NO: 3);
(2) HCDR1 whose amino acid sequence is DYYMH (SEQ ID NO: 4);
HCDR2 whose amino acid sequence is WIDPENGNTIYDPKFQG (SEQ ID NO: 5); and
HCDR3 whose amino acid sequence is GWFAY (SEQ ID NO: 6);
(3) HCDR1 whose amino acid sequence is DYSIH (SEQ ID NO: 7);
HCDR2 whose amino acid sequence is WINTETGEPTYADDFKG (SEQ ID NO: 8); and
HCDR3 whose amino acid sequence is YGYDVRGDY (SEQ ID NO: 9);
(4) HCDR1 whose amino acid sequence is DYYLH (SEQ ID NO: 10);
HCDR2 whose amino acid sequence is WIDPENGNTIYDPKFQG (SEQ ID NO: 11); and
HCDR3 whose amino acid sequence is GYLTY (SEQ ID NO: 12);
(5) HCDR1 whose amino acid sequence is RYWMH (SEQ ID NO: 13);
HCDR2 whose amino acid sequence is EINPSNGRANYNEKFMS (SEQ ID NO: 14); and
HCDR3 whose amino acid sequence is GREITTGFAY (SEQ ID NO: 15);
(6) HCDR1 whose amino acid sequence is DYYMH (SEQ ID NO: 16);
HCDR2 whose amino acid sequence is WIDPENGNTIYDPKFQG (SEQ ID NO: 17); and
HCDR3 whose amino acid sequence is GYLVY (SEQ ID NO: 18);
(7) HCDR1 whose amino acid sequence is DYYMH (SEQ ID NO: 19);
HCDR2 whose amino acid sequence is WIDPENGNTIYDPKFQG (SEQ ID NO: 20); and
HCDR3 whose amino acid sequence is GYLAY (SEQ ID NO: 21);
(8) HCDR1 whose amino acid sequence is DYYMH (SEQ ID NO: 22);
HCDR2 whose amino acid sequence is WIDPENGNTIYDPKFQG (SEQ ID NO: 23); and
HCDR3 whose amino acid sequence is GWFAY (SEQ ID NO: 25);
(9) HCDR1 whose amino acid sequence is DYYMH (SEQ ID NO: 25);
HCDR2 whose amino acid sequence is WIDPENGNTIYDPKFQG (SEQ ID NO: 26); and
HCDR3 whose amino acid sequence is GYLVY (SEQ ID NO: 27);
(10) HCDR1 whose amino acid sequence is DYYMH (SEQ ID NO: 28);
HCDR2 whose amino acid sequence is WIDPENGNTIYDPKFQG (SEQ ID NO: 29); and
HCDR3 whose amino acid sequence is GWFAY (SEQ ID NO: 30);
(11) HCDR1 whose amino acid sequence is DYYMH (SEQ ID NO: 31);
HCDR2 whose amino acid sequence is WIDPENGNTIYDPKFQG (SEQ ID NO: 32); and
HCDR3 whose amino acid sequence is GYLVY (SEQ ID NO: 33);
(12) HCDR1 whose amino acid sequence is DYYMH (SEQ ID NO: 34);
HCDR2 whose amino acid sequence is WIDPENGNTIYDPKFQG (SEQ ID NO: 35); and
HCDR3 whose amino acid sequence is GYLVY (SEQ ID NO: 36);
(13) HCDR1 whose amino acid sequence is NYAMS (SEQ ID NO: 37);
HCDR2 whose amino acid sequence is TITSGGSYTYYPDSVKG (SEQ ID NO: 38); and
HCDR3 whose amino acid sequence is KDYGNYWFAY (SEQ ID NO: 39);
(14) HCDR1 whose amino acid sequence is GYAMS (SEQ ID NO: 40);
HCDR2 whose amino acid sequence is TITSGGSYTYYPDSVKG (SEQ ID NO: 41); and
HCDR3 whose amino acid sequence is KDYGNYWFAY (SEQ ID NO: 42);
(15) HCDR1 whose amino acid sequence is GYAMS (SEQ ID NO: 43);
HCDR2 whose amino acid sequence is TITSGGSYTYYPDSVKG (SEQ ID NO: 44); and
HCDR3 whose amino acid sequence is KDYGNYWFAY (SEQ ID NO: 45);
(16) HCDR1 whose amino acid sequence is SGYSWH (SEQ ID NO: 46);
HCDR2 whose amino acid sequence is YIHYSGSTNYNPPLKS (SEQ ID NO: 47); and
HCDR3 whose amino acid sequence is GVVSNYAMGN (SEQ ID NO: 48);
(17) HCDR1 whose amino acid sequence is TYWMH (SEQ ID NO: 49);
HCDR2 whose amino acid sequence is YINPNTAYTEYNQNFKD (SEQ ID NO: 50); and
HCDR3 whose amino acid sequence is GAYYRTYYAMDY (SEQ ID NO: 51);
(18) HCDR1 whose amino acid sequence is NYGMN (SEQ ID NO: 52);
HCDR2 whose amino acid sequence is WINTYTGEPTYADDFKG (SEQ ID NO: 53); and
HCDR3 whose amino acid sequence is EEFYSRGAMDY (SEQ ID NO: 54); and
(19) HCDR1 whose amino acid sequence is DYYIN (SEQ ID NO: 55);
HCDR2 whose amino acid sequence is WIYPGSGNTKYNEKFKG (SEQ ID NO: 56); and
HCDR3 whose amino acid sequence is SSRCDF (SEQ ID NO: 57), or
the antibody comprises a variant of an HCDR sequence combination in any one of (1)-(19), wherein the variant has at least 90% sequence identity with the HCDR sequences in any one of (1)-(19), or comprises a total of at least 1 and no more than 10 or no more than 5, 4, 3, or 2 amino acid changes to the HCDR sequences.

2. The antibody or antigen-binding fragment thereof according to claim 1, wherein the antibody further comprises a light chain variable region (LCVR) which includes an LCDR1, an LCDR2, and an LCDR3 selected from one of the following combinations:
(1) LCDR1 whose amino acid sequence is SASSSISSNYLH (SEQ ID NO: 58);
LCDR2 whose amino acid sequence is GTSNLAS (SEQ ID NO: 59); and
LCDR3 whose amino acid sequence is HQGSSIPLT (SEQ ID NO: 60);
(2) LCDR1 whose amino acid sequence is KSSQSLLNSSNQKNHLA (SEQ ID NO: 61);
LCDR2 whose amino acid sequence is FASTRAS (SEQ ID NO: 62); and
LCDR3 whose amino acid sequence is QQHYSTPLT (SEQ ID NO: 63);
(3) LCDR1 whose amino acid sequence is SASSGISSNYLH (SEQ ID NO: 64);
LCDR2 whose amino acid sequence is STSNLAS (SEQ ID NO: 65); and
LCDR3 whose amino acid sequence is HQGSDIPLT (SEQ ID NO: 66);
(4) LCDR1 whose amino acid sequence is KSSQNLLNSSNQKNYLA (SEQ ID NO: 67);
LCDR2 whose amino acid sequence is FASTRYS (SEQ ID NO: 68); and
LCDR3 whose amino acid sequence is QQHYSTPLT (SEQ ID NO: 69);
(5) LCDR1 whose amino acid sequence is KASQSVSNDVV (SEQ ID NO: 70);
LCDR2 whose amino acid sequence is YASNRYT (SEQ ID NO: 71); and
LCDR3 whose amino acid sequence is QQDYSSPWT (SEQ ID NO: 72);
(6) LCDR1 whose amino acid sequence is KSSQSLLNSSNQKNYLA (SEQ ID NO: 73);
LCDR2 whose amino acid sequence is FASTRES (SEQ ID NO: 74); and
LCDR3 whose amino acid sequence is QQHYSIPLT (SEQ ID NO: 75);
(7) LCDR1 whose amino acid sequence is KSSQSLLNSSNQKNYLA (SEQ ID NO: 76);
LCDR2 whose amino acid sequence is FASTRKS (SEQ ID NO: 77); and
LCDR3 whose amino acid sequence is QQHYSTPLT (SEQ ID NO: 78);
(8) LCDR1 whose amino acid sequence is KSSQSLLNSSNQKNHLA (SEQ ID NO: 79);
LCDR2 whose amino acid sequence is FASTRQS (SEQ ID NO: 80); and
LCDR3 whose amino acid sequence is QQHYSTPLT (SEQ ID NO: 81);
(9) LCDR1 whose amino acid sequence is KSSQSLLNSSNQKNYLA (SEQ ID NO: 82);
LCDR2 whose amino acid sequence is FASTRQS (SEQ ID NO: 83); and
LCDR3 whose amino acid sequence is QQHYSTPLT (SEQ ID NO: 84);
(10) LCDR1 whose amino acid sequence is KSSQSLLNSSNQKNHLA (SEQ ID NO: 85);
LCDR2 whose amino acid sequence is FASTRGS (SEQ ID NO: 86); and
LCDR3 whose amino acid sequence is QQHYSTPLT (SEQ ID NO: 87);
(11) LCDR1 whose amino acid sequence is KSSQSLLNSSNQKNYLA (SEQ ID NO: 88);
LCDR2 whose amino acid sequence is FASTRDS (SEQ ID NO: 89); and LCDR3 whose amino acid sequence is QQHYSTPLT (SEQ ID NO: 90);
(12) LCDR1 whose amino acid sequence is KSSQSLLNSSNQKNYLA (SEQ ID NO: 91);
LCDR2 whose amino acid sequence is FASTRES (SEQ ID NO: 92); and
LCDR3 whose amino acid sequence is QQHYSTPLT (SEQ ID NO: 93);
(13) LCDR1 whose amino acid sequence is RSSQSLVHSDGNTYLH (SEQ ID NO: 94);
LCDR2 whose amino acid sequence is KVSNRFS (SEQ ID NO: 95); and
LCDR3 whose amino acid sequence is SQSIHVPWT (SEQ ID NO: 96);
(14) LCDR1 whose amino acid sequence is SASSSVSYMH (SEQ ID NO: 97);
LCDR2 whose amino acid sequence is STSNLAS (SEQ ID NO: 98); and
LCDR3 whose amino acid sequence is QQRSSYPLT (SEQ ID NO: 99);
(15) LCDR1 whose amino acid sequence is RSSQSLVHSDGNTYLH (SEQ ID NO: 100);
LCDR2 whose amino acid sequence is KVSNRFS (SEQ ID NO: 101); and
LCDR3 whose amino acid sequence is SQTTQVPWT (SEQ ID NO: 102);
(16) LCDR1 whose amino acid sequence is ITNTDIDDDMN (SEQ ID NO: 103);
LCDR2 whose amino acid sequence is EGNTLRP (SEQ ID NO: 104); and
LCDR3 whose amino acid sequence is LQSDDLPLT (SEQ ID NO: 105);
(17) LCDR1 whose amino acid sequence is KASQSVDYDGDSYMN (SEQ ID NO: 106);
LCDR2 whose amino acid sequence is AASNLES (SEQ ID NO: 107); and
LCDR3 whose amino acid sequence is LQSNEDPYT (SEQ ID NO: 108);
(18) LCDR1 whose amino acid sequence is RSSQFIVHSNGNTYLE (SEQ ID NO: 109);
LCDR2 whose amino acid sequence is KISNRFS (SEQ ID NO: 110); and
LCDR3 whose amino acid sequence is FQGSHVPFT (SEQ ID NO: 111); and
(19) LCDR1 whose amino acid sequence is KASEDIYNRLA (SEQ ID NO: 112);
LCDR2 whose amino acid sequence is GATSLET (SEQ ID NO: 113); and
LCDR3 whose amino acid sequence is QQYWSSPLT (SEQ ID NO: 114), or
the antibody comprises a variant of an LCDR sequence combination in any one of (1)-(19), wherein the variant has at least 90% sequence identity with the LCDR sequences in any one of (1)-(19), or comprises a total of at least 1 and no more than 10 or no more than 5, 4, 3, or 2 amino acid changes to the LCDR sequences.

3. The antibody or antigen-binding fragment thereof according to claim 1 or claim 2, wherein the amino acid sequence of the heavy chain variable region is selected from any of the following:
(1) the sequence shown in SEQ ID NO: 115 or a heavy chain variable region sequence having at least 90%, 95%, 96%, 97%, 98% or 99% sequence identity therewith;
(2) the sequence shown in SEQ ID NO: 117 or a heavy chain variable region sequence having at least 90%, 95%, 96%, 97%, 98% or 99% sequence identity therewith;
(3) the sequence shown in SEQ ID NO: 119 or a heavy chain variable region sequence having at least 90%, 95%, 96%, 97%, 98% or 99% sequence identity therewith;
(4) the sequence shown in SEQ ID NO: 121 or a heavy chain variable region sequence having at least 90%, 95%, 96%, 97%, 98% or 99% sequence identity therewith;
(5) the sequence shown in SEQ ID NO: 123 or a heavy chain variable region sequence having at least 90%, 95%, 96%, 97%, 98% or 99% sequence identity therewith;
(6) the sequence shown in SEQ ID NO: 125 or a heavy chain variable region sequence having at least 90%, 95%, 96%, 97%, 98% or 99% sequence identity therewith;
(7) the sequence shown in SEQ ID NO: 127 or a heavy chain variable region sequence having at least 90%, 95%, 96%, 97%, 98% or 99% sequence identity therewith;
(8) the sequence shown in SEQ ID NO: 129 or a heavy chain variable region sequence having at least 90%, 95%, 96%, 97%, 98% or 99% sequence identity therewith;
(9) the sequence shown in SEQ ID NO: 131 or a heavy chain variable region sequence having at least 90%, 95%, 96%, 97%, 98% or 99% sequence identity therewith;
(10) the sequence shown in SEQ ID NO: 133 or a heavy chain variable region sequence having at least 90%, 95%, 96%, 97%, 98% or 99% sequence identity therewith;
(11) the sequence shown in SEQ ID NO: 135 or a heavy chain variable region sequence having at least 90%, 95%, 96%, 97%, 98% or 99% sequence identity therewith;
(12) the sequence shown in SEQ ID NO: 137 or a heavy chain variable region sequence having at least 90%, 95%, 96%, 97%, 98% or 99% sequence identity therewith;
(13) the sequence shown in SEQ ID NO: 139 or a heavy chain variable region sequence having at least 90%, 95%, 96%, 97%, 98% or 99% sequence identity therewith;
(14) the sequence shown in SEQ ID NO: 141 or a heavy chain variable region sequence having at least 90%, 95%, 96%, 97%, 98% or 99% sequence identity therewith;
(15) the sequence shown in SEQ ID NO: 143 or a heavy chain variable region sequence having at least 90%, 95%, 96%, 97%, 98% or 99% sequence identity therewith;
(16) the sequence shown in SEQ ID NO: 145 or a heavy chain variable region sequence having at least 90%, 95%, 96%, 97%, 98% or 99% sequence identity therewith;
(17) the sequence shown in SEQ ID NO: 147 or a heavy chain variable region sequence having at least 90%, 95%, 96%, 97%, 98% or 99% sequence identity therewith;
(18) the sequence shown in SEQ ID NO: 149 or a heavy chain variable region sequence having at least 90%, 95%, 96%, 97%, 98% or 99% sequence identity therewith; and
(19) the sequence shown in SEQ ID NO: 151 or a heavy chain variable region sequence having at least 90%, 95%, 96%, 97%, 98% or 99% sequence identity therewith.

4. The antibody or antigen-binding fragment thereof according to any one of claims 1-3, wherein the amino acid sequence of the heavy chain variable region and the amino acid sequence of the light chain variable region of the antibody are selected from any of the following combinations:
(1) the sequence shown in SEQ ID NO: 115 or a heavy chain variable region sequence having at least 90%, 95%, 96%, 97%, 98% or 99% sequence identity therewith, and the sequence shown in SEQ ID NO: 116 or a light chain variable region sequence having at least 90%, 95%, 96%, 97%, 98% or 99% sequence identity therewith;
(2) the sequence shown in SEQ ID NO: 117 or a heavy chain variable region sequence having at least 90%, 95%, 96%, 97%, 98% or 99% sequence identity therewith, and the sequence shown in SEQ ID NO: 118 or a light chain variable region sequence having at least 90%, 95%, 96%, 97%, 98% or 99% sequence identity therewith;
(3) the sequence shown in SEQ ID NO: 119 or a heavy chain variable region sequence having at least 90%, 95%, 96%, 97%, 98% or 99% sequence identity therewith, and the sequence shown in SEQ ID NO: 120 or a light chain variable region sequence having at least 90%, 95%, 96%, 97%, 98% or 99% sequence identity therewith;
(4) the sequence shown in SEQ ID NO: 121 or a heavy chain variable region sequence having at least 90%, 95%, 96%, 97%, 98% or 99% sequence identity therewith, and the sequence shown in SEQ ID NO: 122 or a light chain variable region sequence having at least 90%, 95%, 96%, 97%, 98% or 99% sequence identity therewith;
(5) the sequence shown in SEQ ID NO: 123 or a heavy chain variable region sequence having at least 90%, 95%, 96%, 97%, 98% or 99% sequence identity therewith, and the sequence shown in SEQ ID NO: 124 or a light chain variable region sequence having at least 90%, 95%, 96%, 97%, 98% or 99% sequence identity therewith;
(6) the sequence shown in SEQ ID NO: 125 or a heavy chain variable region sequence having at least 90%, 95%, 96%, 97%, 98% or 99% sequence identity therewith, and the sequence shown in SEQ ID NO: 126 or a light chain variable region sequence having at least 90%, 95%, 96%, 97%, 98% or 99% sequence identity therewith;
(7) the sequence shown in SEQ ID NO: 127 or a heavy chain variable region sequence having at least 90%, 95%, 96%, 97%, 98% or 99% sequence identity therewith, and the sequence shown in SEQ ID NO: 128 or a light chain variable region sequence having at least 90%, 95%, 96%, 97%, 98% or 99% sequence identity therewith;
(8) the sequence shown in SEQ ID NO: 129 or a heavy chain variable region sequence having at least 90%, 95%, 96%, 97%, 98% or 99% sequence identity therewith, and the sequence shown in SEQ ID NO: 130 or a light chain variable region sequence having at least 90%, 95%, 96%, 97%, 98% or 99% sequence identity therewith;
(9) the sequence shown in SEQ ID NO: 131 or a heavy chain variable region sequence having at least 90%, 95%, 96%, 97%, 98% or 99% sequence identity therewith, and the sequence shown in SEQ ID NO: 132 or a light chain variable region sequence having at least 90%, 95%, 96%, 97%, 98% or 99% sequence identity therewith;
(10) the sequence shown in SEQ ID NO: 133 or a heavy chain variable region sequence having at least 90%, 95%, 96%, 97%, 98% or 99% sequence identity therewith, and the sequence shown in SEQ ID NO: 134 or a light chain variable region sequence having at least 90%, 95%, 96%, 97%, 98% or 99% sequence identity therewith;
(11) the sequence shown in SEQ ID NO: 135 or a heavy chain variable region sequence having at least 90%, 95%, 96%, 97%, 98% or 99% sequence identity therewith, and the sequence shown in SEQ ID NO: 136 or a light chain variable region sequence having at least 90%, 95%, 96%, 97%, 98% or 99% sequence identity therewith;
(12) the sequence shown in SEQ ID NO: 137 or a heavy chain variable region sequence having at least 90%, 95%, 96%, 97%, 98% or 99% sequence identity therewith, and the sequence shown in SEQ ID NO: 138 or a light chain variable region sequence having at least 90%, 95%, 96%, 97%, 98% or 99% sequence identity therewith;
(13) the sequence shown in SEQ ID NO: 139 or a heavy chain variable region sequence having at least 90%, 95%, 96%, 97%, 98% or 99% sequence identity therewith, and the sequence shown in SEQ ID NO: 140 or a light chain variable region sequence having at least 90%, 95%, 96%, 97%, 98% or 99% sequence identity therewith;
(14) the sequence shown in SEQ ID NO: 141 or a heavy chain variable region sequence having at least 90%, 95%, 96%, 97%, 98% or 99% sequence identity therewith, and the sequence shown in SEQ ID NO: 142 or a light chain variable region sequence having at least 90%, 95%, 96%, 97%, 98% or 99% sequence identity therewith;
(15) the sequence shown in SEQ ID NO: 143 or a heavy chain variable region sequence having at least 90%, 95%, 96%, 97%, 98% or 99% sequence identity therewith, and the sequence shown in SEQ ID NO: 144 or a light chain variable region sequence having at least 90%, 95%, 96%, 97%, 98% or 99% sequence identity therewith;
(16) the sequence shown in SEQ ID NO: 145 or a heavy chain variable region sequence having at least 90%, 95%, 96%, 97%, 98% or 99% sequence identity therewith, and the sequence shown in SEQ ID NO: 146 or a light chain variable region sequence having at least 90%, 95%, 96%, 97%, 98% or 99% sequence identity therewith;
(17) the sequence shown in SEQ ID NO: 147 or a heavy chain variable region sequence having at least 90%, 95%, 96%, 97%, 98% or 99% sequence identity therewith, and the sequence shown in SEQ ID NO: 148 or a light chain variable region sequence having at least 90%, 95%, 96%, 97%, 98% or 99% sequence identity therewith;
(18) the sequence shown in SEQ ID NO: 149 or a heavy chain variable region sequence having at least 90%, 95%, 96%, 97%, 98% or 99% sequence identity therewith, and the sequence shown in SEQ ID NO: 150 or a light chain variable region sequence having at least 90%, 95%, 96%, 97%, 98% or 99% sequence identity therewith; and
(19) the sequence shown in SEQ ID NO: 151 or a heavy chain variable region sequence having at least 90%, 95%, 96%, 97%, 98% or 99% sequence identity therewith, and the sequence shown in SEQ ID NO: 152 or a light chain variable region sequence having at least 90%, 95%, 96%, 97%, 98% or 99% sequence identity therewith.

5. The antibody or antigen-binding fragment thereof according to any one of claims 1-4, wherein the antibody binds to an EGFRvIII antigen with a KD value of less than 10⁻⁶ M and preferably less than 10⁻⁷ M, as determined by surface plasmon resonance (SPR).

6. The antibody or antigen-binding fragment thereof according to any one of claims 1-5, wherein the antibody is selected from a murine antibody, a chimeric antibody, a humanized antibody, or a human antibody.

7. The antibody or antigen-binding fragment thereof according to any one of claims 1-6, wherein the humanized antibody binds to an EGFRvIII antigen with a KD value of less than 10⁻⁶ M and preferably less than 10⁻⁷ M, as determined by surface plasmon resonance (SPR).

8. The antibody or antigen-binding fragment thereof according to any one of claims 1-7, wherein the antigen-binding fragment comprises an Fab, an Fab', an F(ab')₂, a single domain antibody, or a single chain antibody.

9. The antibody or antigen-binding fragment thereof according to any one of claims 1-8, wherein the humanized antibody is a single chain antibody whose amino acid sequence is selected from any of the following:
(1) the sequence shown in SEQ ID NO: 167 or a heavy chain variable region sequence having at least 90%, 95%, 96%, 97%, 98% or 99% sequence identity therewith;
(2) the sequence shown in SEQ ID NO: 169 or a heavy chain variable region sequence having at least 90%, 95%, 96%, 97%, 98% or 99% sequence identity therewith;
(3) the sequence shown in SEQ ID NO: 171 or a heavy chain variable region sequence having at least 90%, 95%, 96%, 97%, 98% or 99% sequence identity therewith;
(4) the sequence shown in SEQ ID NO: 173 or a heavy chain variable region sequence having at least 90%, 95%, 96%, 97%, 98% or 99% sequence identity therewith;
(5) the sequence shown in SEQ ID NO: 175 or a heavy chain variable region sequence having at least 90%, 95%, 96%, 97%, 98% or 99% sequence identity therewith;
(6) the sequence shown in SEQ ID NO: 177 or a heavy chain variable region sequence having at least 90%, 95%, 96%, 97%, 98% or 99% sequence identity therewith;
(7) the sequence shown in SEQ ID NO: 179 or a heavy chain variable region sequence having at least 90%, 95%, 96%, 97%, 98% or 99% sequence identity therewith;
(8) the sequence shown in SEQ ID NO: 181 or a heavy chain variable region sequence having at least 90%, 95%, 96%, 97%, 98% or 99% sequence identity therewith;
(9) the sequence shown in SEQ ID NO: 183 or a heavy chain variable region sequence having at least 90%, 95%, 96%, 97%, 98% or 99% sequence identity therewith;
(10) the sequence shown in SEQ ID NO: 185 or a heavy chain variable region sequence having at least 90%, 95%, 96%, 97%, 98% or 99% sequence identity therewith; and
(11) the sequence shown in SEQ ID NO: 187 or a heavy chain variable region sequence having at least 90%, 95%, 96%, 97%, 98% or 99% sequence identity therewith.

10. The antibody or antigen-binding fragment thereof according to any one of claims 1-9, further comprising an Fc fragment.

11. The antibody or antigen-binding fragment thereof according to any one of claims 1-10, wherein the antigen-binding fragment is a single chain antibody; preferably, a linker for linking the heavy chain variable region with the light chain variable region of the single chain antibody comprises the sequence shown in SEQ ID NO: 166.

12. A chimeric antigen receptor (CAR) whose extracellular antigen-binding domain includes one or more antibody molecules targeting EGFRvIII or antigen-binding fragments thereof, wherein the HCDR1, HCDR2, and HCDR3 of the heavy chain variable region of the antibody molecule are selected from one of the following combinations:
(1) HCDR1 whose amino acid sequence is DFSMH (SEQ ID NO: 1);
HCDR2 whose amino acid sequence is WINTETGEPSYADDFKG (SEQ ID NO: 2); and
HCDR3 whose amino acid sequence is YGYDVRGDY (SEQ ID NO: 3);
(2) HCDR1 whose amino acid sequence is DYYMH (SEQ ID NO: 4);
HCDR2 whose amino acid sequence is WIDPENGNTIYDPKFQG (SEQ ID NO: 5); and
HCDR3 whose amino acid sequence is GWFAY (SEQ ID NO: 6);
(3) HCDR1 whose amino acid sequence is DYSIH (SEQ ID NO: 7);
HCDR2 whose amino acid sequence is WINTETGEPTYADDFKG (SEQ ID NO: 8); and
HCDR3 whose amino acid sequence is YGYDVRGDY (SEQ ID NO: 9);
(4) HCDR1 whose amino acid sequence is DYYLH (SEQ ID NO: 10);
HCDR2 whose amino acid sequence is WIDPENGNTIYDPKFQG (SEQ ID NO: 11); and
HCDR3 whose amino acid sequence is GYLTY (SEQ ID NO: 12);
(5) HCDR1 whose amino acid sequence is RYWMH (SEQ ID NO: 13);
HCDR2 whose amino acid sequence is EINPSNGRANYNEKFMS (SEQ ID NO: 14); and
HCDR3 whose amino acid sequence is GREITTGFAY (SEQ ID NO: 15);
(6) HCDR1 whose amino acid sequence is DYYMH (SEQ ID NO: 16);
HCDR2 whose amino acid sequence is WIDPENGNTIYDPKFQG (SEQ ID NO: 17); and
HCDR3 whose amino acid sequence is GYLVY (SEQ ID NO: 18);
(7) HCDR1 whose amino acid sequence is DYYMH (SEQ ID NO: 19);
HCDR2 whose amino acid sequence is WIDPENGNTIYDPKFQG (SEQ ID NO: 20); and
HCDR3 whose amino acid sequence is GYLAY (SEQ ID NO: 21);
(8) HCDR1 whose amino acid sequence is DYYMH (SEQ ID NO: 22);
HCDR2 whose amino acid sequence is WIDPENGNTIYDPKFQG (SEQ ID NO: 23); and
HCDR3 whose amino acid sequence is GWFAY (SEQ ID NO: 25);
(9) HCDR1 whose amino acid sequence is DYYMH (SEQ ID NO: 25);
HCDR2 whose amino acid sequence is WIDPENGNTIYDPKFQG (SEQ ID NO: 26); and
HCDR3 whose amino acid sequence is GYLVY (SEQ ID NO: 27);
(10) HCDR1 whose amino acid sequence is DYYMH (SEQ ID NO: 28);
HCDR2 whose amino acid sequence is WIDPENGNTIYDPKFQG (SEQ ID NO: 29); and
HCDR3 whose amino acid sequence is GWFAY (SEQ ID NO: 30);
(11) HCDR1 whose amino acid sequence is DYYMH (SEQ ID NO: 31);
HCDR2 whose amino acid sequence is WIDPENGNTIYDPKFQG (SEQ ID NO: 32); and
HCDR3 whose amino acid sequence is GYLVY (SEQ ID NO: 33);
(12) HCDR1 whose amino acid sequence is DYYMH (SEQ ID NO: 34);
HCDR2 whose amino acid sequence is WIDPENGNTIYDPKFQG (SEQ ID NO: 35); and
HCDR3 whose amino acid sequence is GYLVY (SEQ ID NO: 36);
(13) HCDR1 whose amino acid sequence is NYAMS (SEQ ID NO: 37);
HCDR2 whose amino acid sequence is TITSGGSYTYYPDSVKG (SEQ ID NO: 38); and
HCDR3 whose amino acid sequence is KDYGNYWFAY (SEQ ID NO: 39);
(14) HCDR1 whose amino acid sequence is GYAMS (SEQ ID NO: 40);
HCDR2 whose amino acid sequence is TITSGGSYTYYPDSVKG (SEQ ID NO: 41); and
HCDR3 whose amino acid sequence is KDYGNYWFAY (SEQ ID NO: 42);
(15) HCDR1 whose amino acid sequence is GYAMS (SEQ ID NO: 43);
HCDR2 whose amino acid sequence is TITSGGSYTYYPDSVKG (SEQ ID NO: 44); and
HCDR3 whose amino acid sequence is KDYGNYWFAY (SEQ ID NO: 45);
(16) HCDR1 whose amino acid sequence is SGYSWH (SEQ ID NO: 46);
HCDR2 whose amino acid sequence is YIHYSGSTNYNPPLKS (SEQ ID NO: 47); and
HCDR3 whose amino acid sequence is GVVSNYAMGN (SEQ ID NO: 48);
(17) HCDR1 whose amino acid sequence is TYWMH (SEQ ID NO: 49);
HCDR2 whose amino acid sequence is YINPNTAYTEYNQNFKD (SEQ ID NO: 50); and
HCDR3 whose amino acid sequence is GAYYRTYYAMDY (SEQ ID NO: 51);
(18) HCDR1 whose amino acid sequence is NYGMN (SEQ ID NO: 52);
HCDR2 whose amino acid sequence is WINTYTGEPTYADDFKG (SEQ ID NO: 53); and
HCDR3 whose amino acid sequence is EEFYSRGAMDY (SEQ ID NO: 54); and
(19) HCDR1 whose amino acid sequence is DYYIN (SEQ ID NO: 55);
HCDR2 whose amino acid sequence is WIYPGSGNTKYNEKFKG (SEQ ID NO: 56); and
HCDR3 whose amino acid sequence is SSRCDF (SEQ ID NO: 57), or
the antibody comprises a variant of an HCDR sequence combination in any one of (1)-(19), wherein the variant has at least 90% sequence identity with the HCDR sequences in any one of (1)-(19), or comprises a total of at least 1 and no more than 10 or no more than 5, 4, 3, or 2 amino acid changes to the HCDR sequences.

13. The CAR according to claim 12, wherein the antibody further comprises a light chain variable region (LCVR) which includes an LCDR1, an LCDR2, and an LCDR3 selected from one of the following combinations:
(1) LCDR1 whose amino acid sequence is SASSSISSNYLH (SEQ ID NO: 58);
LCDR2 whose amino acid sequence is GTSNLAS (SEQ ID NO: 59); and
LCDR3 whose amino acid sequence is HQGSSIPLT (SEQ ID NO: 60);
(2) LCDR1 whose amino acid sequence is KSSQSLLNSSNQKNHLA (SEQ ID NO: 61);
LCDR2 whose amino acid sequence is FASTRAS (SEQ ID NO: 62); and
LCDR3 whose amino acid sequence is QQHYSTPLT (SEQ ID NO: 63);
(3) LCDR1 whose amino acid sequence is SASSGISSNYLH (SEQ ID NO: 64);
LCDR2 whose amino acid sequence is STSNLAS (SEQ ID NO: 65); and
LCDR3 whose amino acid sequence is HQGSDIPLT (SEQ ID NO: 66);
(4) LCDR1 whose amino acid sequence is KSSQNLLNSSNQKNYLA (SEQ ID NO: 67);
LCDR2 whose amino acid sequence is FASTRYS (SEQ ID NO: 68); and
LCDR3 whose amino acid sequence is QQHYSTPLT (SEQ ID NO: 69);
(5) LCDR1 whose amino acid sequence is KASQSVSNDVV (SEQ ID NO: 70);
LCDR2 whose amino acid sequence is YASNRYT (SEQ ID NO: 71); and
LCDR3 whose amino acid sequence is QQDYSSPWT (SEQ ID NO: 72);
(6) LCDR1 whose amino acid sequence is KSSQSLLNSSNQKNYLA (SEQ ID NO: 73);
LCDR2 whose amino acid sequence is FASTRES (SEQ ID NO: 74); and
LCDR3 whose amino acid sequence is QQHYSIPLT (SEQ ID NO: 75);
(7) LCDR1 whose amino acid sequence is KSSQSLLNSSNQKNYLA (SEQ ID NO: 76);
LCDR2 whose amino acid sequence is FASTRKS (SEQ ID NO: 77); and
LCDR3 whose amino acid sequence is QQHYSTPLT (SEQ ID NO: 78);
(8) LCDR1 whose amino acid sequence is KSSQSLLNSSNQKNHLA (SEQ ID NO: 79);
LCDR2 whose amino acid sequence is FASTRQS (SEQ ID NO: 80); and
LCDR3 whose amino acid sequence is QQHYSTPLT (SEQ ID NO: 81);
(9) LCDR1 whose amino acid sequence is KSSQSLLNSSNQKNYLA (SEQ ID NO: 82);
LCDR2 whose amino acid sequence is FASTRQS (SEQ ID NO: 83); and
LCDR3 whose amino acid sequence is QQHYSTPLT (SEQ ID NO: 84);
(10) LCDR1 whose amino acid sequence is KSSQSLLNSSNQKNHLA (SEQ ID NO: 85);
LCDR2 whose amino acid sequence is FASTRGS (SEQ ID NO: 86); and
LCDR3 whose amino acid sequence is QQHYSTPLT (SEQ ID NO: 87);
(11) LCDR1 whose amino acid sequence is KSSQSLLNSSNQKNYLA (SEQ ID NO: 88);
LCDR2 whose amino acid sequence is FASTRDS (SEQ ID NO: 89); and
LCDR3 whose amino acid sequence is QQHYSTPLT (SEQ ID NO: 90);
(12) LCDR1 whose amino acid sequence is KSSQSLLNSSNQKNYLA (SEQ ID NO: 91);
LCDR2 whose amino acid sequence is FASTRES (SEQ ID NO: 92); and
LCDR3 whose amino acid sequence is QQHYSTPLT (SEQ ID NO: 93);
(13) LCDR1 whose amino acid sequence is RSSQSLVHSDGNTYLH (SEQ ID NO: 94);
LCDR2 whose amino acid sequence is KVSNRFS (SEQ ID NO: 95); and
LCDR3 whose amino acid sequence is SQSIHVPWT (SEQ ID NO: 96);
(14) LCDR1 whose amino acid sequence is SASSSVSYMH (SEQ ID NO: 97);
LCDR2 whose amino acid sequence is STSNLAS (SEQ ID NO: 98); and
LCDR3 whose amino acid sequence is QQRSSYPLT (SEQ ID NO: 99);
(15) LCDR1 whose amino acid sequence is RSSQSLVHSDGNTYLH (SEQ ID NO: 100);
LCDR2 whose amino acid sequence is KVSNRFS (SEQ ID NO: 101); and LCDR3 whose amino acid sequence is SQTTQVPWT (SEQ ID NO: 102);
(16) LCDR1 whose amino acid sequence is ITNTDIDDDMN (SEQ ID NO: 103);
LCDR2 whose amino acid sequence is EGNTLRP (SEQ ID NO: 104); and
LCDR3 whose amino acid sequence is LQSDDLPLT (SEQ ID NO: 105);
(17) LCDR1 whose amino acid sequence is KASQSVDYDGDSYMN (SEQ ID NO: 106);
LCDR2 whose amino acid sequence is AASNLES (SEQ ID NO: 107); and
LCDR3 whose amino acid sequence is LQSNEDPYT (SEQ ID NO: 108);
(18) LCDR1 whose amino acid sequence is RSSQFIVHSNGNTYLE (SEQ ID NO: 109);
LCDR2 whose amino acid sequence is KISNRFS (SEQ ID NO: 110); and
LCDR3 whose amino acid sequence is FQGSHVPFT (SEQ ID NO: 111); and
(19) LCDR1 whose amino acid sequence is KASEDIYNRLA (SEQ ID NO: 112);
LCDR2 whose amino acid sequence is GATSLET (SEQ ID NO: 113); and
LCDR3 whose amino acid sequence is QQYWSSPLT (SEQ ID NO: 114), or
the antibody comprises a variant of an LCDR sequence combination in any one of (1)-(19), wherein the variant has at least 90% sequence identity with the LCDR sequences in any one of (1)-(19), or comprises a total of at least 1 and no more than 10 or no more than 5, 4, 3, or 2 amino acid changes to the LCDR sequences.

14. The CAR according to claim 12 or claim 13, wherein the amino acid sequence of the heavy chain variable region is selected from any of the following:
(1) the sequence shown in SEQ ID NO: 115 or a heavy chain variable region sequence having at least 90%, 95%, 96%, 97%, 98% or 99% sequence identity therewith;
(2) the sequence shown in SEQ ID NO: 117 or a heavy chain variable region sequence having at least 90%, 95%, 96%, 97%, 98% or 99% sequence identity therewith;
(3) the sequence shown in SEQ ID NO: 119 or a heavy chain variable region sequence having at least 90%, 95%, 96%, 97%, 98% or 99% sequence identity therewith;
(4) the sequence shown in SEQ ID NO: 121 or a heavy chain variable region sequence having at least 90%, 95%, 96%, 97%, 98% or 99% sequence identity therewith;
(5) the sequence shown in SEQ ID NO: 123 or a heavy chain variable region sequence having at least 90%, 95%, 96%, 97%, 98% or 99% sequence identity therewith;
(6) the sequence shown in SEQ ID NO: 125 or a heavy chain variable region sequence having at least 90%, 95%, 96%, 97%, 98% or 99% sequence identity therewith;
(7) the sequence shown in SEQ ID NO: 127 or a heavy chain variable region sequence having at least 90%, 95%, 96%, 97%, 98% or 99% sequence identity therewith;
(8) the sequence shown in SEQ ID NO: 129 or a heavy chain variable region sequence having at least 90%, 95%, 96%, 97%, 98% or 99% sequence identity therewith;
(9) the sequence shown in SEQ ID NO: 131 or a heavy chain variable region sequence having at least 90%, 95%, 96%, 97%, 98% or 99% sequence identity therewith;
(10) the sequence shown in SEQ ID NO: 133 or a heavy chain variable region sequence having at least 90%, 95%, 96%, 97%, 98% or 99% sequence identity therewith;
(11) the sequence shown in SEQ ID NO: 135 or a heavy chain variable region sequence having at least 90%, 95%, 96%, 97%, 98% or 99% sequence identity therewith;
(12) the sequence shown in SEQ ID NO: 137 or a heavy chain variable region sequence having at least 90%, 95%, 96%, 97%, 98% or 99% sequence identity therewith;
(13) the sequence shown in SEQ ID NO: 139 or a heavy chain variable region sequence having at least 90%, 95%, 96%, 97%, 98% or 99% sequence identity therewith;
(14) the sequence shown in SEQ ID NO: 141 or a heavy chain variable region sequence having at least 90%, 95%, 96%, 97%, 98% or 99% sequence identity therewith;
(15) the sequence shown in SEQ ID NO: 143 or a heavy chain variable region sequence having at least 90%, 95%, 96%, 97%, 98% or 99% sequence identity therewith;
(16) the sequence shown in SEQ ID NO: 145 or a heavy chain variable region sequence having at least 90%, 95%, 96%, 97%, 98% or 99% sequence identity therewith;
(17) the sequence shown in SEQ ID NO: 147 or a heavy chain variable region sequence having at least 90%, 95%, 96%, 97%, 98% or 99% sequence identity therewith;
(18) the sequence shown in SEQ ID NO: 149 or a heavy chain variable region sequence having at least 90%, 95%, 96%, 97%, 98% or 99% sequence identity therewith; and
(19) the sequence shown in SEQ ID NO: 151 or a heavy chain variable region sequence having at least 90%, 95%, 96%, 97%, 98% or 99% sequence identity therewith.

15. The CAR according to any one of claims 12-14, wherein the amino acid sequence of the heavy chain variable region and the amino acid sequence of the light chain variable region of the antibody are selected from any of the following combinations:
(1) the sequence shown in SEQ ID NO: 115 or a heavy chain variable region sequence having at least 90%, 95%, 96%, 97%, 98% or 99% sequence identity therewith, and the sequence shown in SEQ ID NO: 116 or a light chain variable region sequence having at least 90%, 95%, 96%, 97%, 98% or 99% sequence identity therewith;
(2) the sequence shown in SEQ ID NO: 117 or a heavy chain variable region sequence having at least 90%, 95%, 96%, 97%, 98% or 99% sequence identity therewith, and the sequence shown in SEQ ID NO: 118 or a light chain variable region sequence having at least 90%, 95%, 96%, 97%, 98% or 99% sequence identity therewith;
(3) the sequence shown in SEQ ID NO: 119 or a heavy chain variable region sequence having at least 90%, 95%, 96%, 97%, 98% or 99% sequence identity therewith, and the sequence shown in SEQ ID NO: 120 or a light chain variable region sequence having at least 90%, 95%, 96%, 97%, 98% or 99% sequence identity therewith;
(4) the sequence shown in SEQ ID NO: 121 or a heavy chain variable region sequence having at least 90%, 95%, 96%, 97%, 98% or 99% sequence identity therewith, and the sequence shown in SEQ ID NO: 122 or a light chain variable region sequence having at least 90%, 95%, 96%, 97%, 98% or 99% sequence identity therewith;
(5) the sequence shown in SEQ ID NO: 123 or a heavy chain variable region sequence having at least 90%, 95%, 96%, 97%, 98% or 99% sequence identity therewith, and the sequence shown in SEQ ID NO: 124 or a light chain variable region sequence having at least 90%, 95%, 96%, 97%, 98% or 99% sequence identity therewith;
(6) the sequence shown in SEQ ID NO: 125 or a heavy chain variable region sequence having at least 90%, 95%, 96%, 97%, 98% or 99% sequence identity therewith, and the sequence shown in SEQ ID NO: 126 or a light chain variable region sequence having at least 90%, 95%, 96%, 97%, 98% or 99% sequence identity therewith;
(7) the sequence shown in SEQ ID NO: 127 or a heavy chain variable region sequence having at least 90%, 95%, 96%, 97%, 98% or 99% sequence identity therewith, and the sequence shown in SEQ ID NO: 128 or a light chain variable region sequence having at least 90%, 95%, 96%, 97%, 98% or 99% sequence identity therewith;
(8) the sequence shown in SEQ ID NO: 129 or a heavy chain variable region sequence having at least 90%, 95%, 96%, 97%, 98% or 99% sequence identity therewith, and the sequence shown in SEQ ID NO: 130 or a light chain variable region sequence having at least 90%, 95%, 96%, 97%, 98% or 99% sequence identity therewith;
(9) the sequence shown in SEQ ID NO: 131 or a heavy chain variable region sequence having at least 90%, 95%, 96%, 97%, 98% or 99% sequence identity therewith, and the sequence shown in SEQ ID NO: 132 or a light chain variable region sequence having at least 90%, 95%, 96%, 97%, 98% or 99% sequence identity therewith;
(10) the sequence shown in SEQ ID NO: 133 or a heavy chain variable region sequence having at least 90%, 95%, 96%, 97%, 98% or 99% sequence identity therewith, and the sequence shown in SEQ ID NO: 134 or a light chain variable region sequence having at least 90%, 95%, 96%, 97%, 98% or 99% sequence identity therewith;
(11) the sequence shown in SEQ ID NO: 135 or a heavy chain variable region sequence having at least 90%, 95%, 96%, 97%, 98% or 99% sequence identity therewith, and the sequence shown in SEQ ID NO: 136 or a light chain variable region sequence having at least 90%, 95%, 96%, 97%, 98% or 99% sequence identity therewith;
(12) the sequence shown in SEQ ID NO: 137 or a heavy chain variable region sequence having at least 90%, 95%, 96%, 97%, 98% or 99% sequence identity therewith, and the sequence shown in SEQ ID NO: 138 or a light chain variable region sequence having at least 90%, 95%, 96%, 97%, 98% or 99% sequence identity therewith;
(13) the sequence shown in SEQ ID NO: 139 or a heavy chain variable region sequence having at least 90%, 95%, 96%, 97%, 98% or 99% sequence identity therewith, and the sequence shown in SEQ ID NO: 140 or a light chain variable region sequence having at least 90%, 95%, 96%, 97%, 98% or 99% sequence identity therewith;
(14) the sequence shown in SEQ ID NO: 141 or a heavy chain variable region sequence having at least 90%, 95%, 96%, 97%, 98% or 99% sequence identity therewith, and the sequence shown in SEQ ID NO: 142 or a light chain variable region sequence having at least 90%, 95%, 96%, 97%, 98% or 99% sequence identity therewith;
(15) the sequence shown in SEQ ID NO: 143 or a heavy chain variable region sequence having at least 90%, 95%, 96%, 97%, 98% or 99% sequence identity therewith, and the sequence shown in SEQ ID NO: 144 or a light chain variable region sequence having at least 90%, 95%, 96%, 97%, 98% or 99% sequence identity therewith;
(16) the sequence shown in SEQ ID NO: 145 or a heavy chain variable region sequence having at least 90%, 95%, 96%, 97%, 98% or 99% sequence identity therewith, and the sequence shown in SEQ ID NO: 146 or a light chain variable region sequence having at least 90%, 95%, 96%, 97%, 98% or 99% sequence identity therewith;
(17) the sequence shown in SEQ ID NO: 147 or a heavy chain variable region sequence having at least 90%, 95%, 96%, 97%, 98% or 99% sequence identity therewith, and the sequence shown in SEQ ID NO: 148 or a light chain variable region sequence having at least 90%, 95%, 96%, 97%, 98% or 99% sequence identity therewith;
(18) the sequence shown in SEQ ID NO: 149 or a heavy chain variable region sequence having at least 90%, 95%, 96%, 97%, 98% or 99% sequence identity therewith, and the sequence shown in SEQ ID NO: 150 or a light chain variable region sequence having at least 90%, 95%, 96%, 97%, 98% or 99% sequence identity therewith; and
(19) the sequence shown in SEQ ID NO: 151 or a heavy chain variable region sequence having at least 90%, 95%, 96%, 97%, 98% or 99% sequence identity therewith, and the sequence shown in SEQ ID NO: 152 or a light chain variable region sequence having at least 90%, 95%, 96%, 97%, 98% or 99% sequence identity therewith.

16. The CAR according to any one of claims 12-15, wherein the antibody binds to an EGFRvIII antigen with a KD value of less than 10⁻⁶ M and preferably less than 10⁻⁷ M, as determined by surface plasmon resonance (SPR).

17. The CAR according to any one of claims 12-16, wherein the antibody is selected from a murine antibody, a chimeric antibody, a humanized antibody, or a human antibody.

18. The CAR according to any one of claims 12-17, wherein the humanized antibody binds to an EGFRvIII antigen with a KD value of less than 10⁻⁶ M and preferably less than 10⁻⁷ M, as determined by surface plasmon resonance (SPR).

19. The CAR according to any one of claims 12-18, wherein the antigen-binding fragment is a single chain antibody; preferably, a linker for linking the heavy chain variable region with the light chain variable region of the single chain antibody comprises the sequence shown in SEQ ID NO: 166.

20. The CAR according to any one of claims 12-19, wherein the humanized antibody is a single chain antibody whose amino acid sequence is selected from any of the following:
(1) the sequence shown in SEQ ID NO: 167 or a heavy chain variable region sequence having at least 90%, 95%, 96%, 97%, 98% or 99% sequence identity therewith;
(2) the sequence shown in SEQ ID NO: 169 or a heavy chain variable region sequence having at least 90%, 95%, 96%, 97%, 98% or 99% sequence identity therewith;
(3) the sequence shown in SEQ ID NO: 171 or a heavy chain variable region sequence having at least 90%, 95%, 96%, 97%, 98% or 99% sequence identity therewith;
(4) the sequence shown in SEQ ID NO: 173 or a heavy chain variable region sequence having at least 90%, 95%, 96%, 97%, 98% or 99% sequence identity therewith;
(5) the sequence shown in SEQ ID NO: 175 or a heavy chain variable region sequence having at least 90%, 95%, 96%, 97%, 98% or 99% sequence identity therewith;
(6) the sequence shown in SEQ ID NO: 177 or a heavy chain variable region sequence having at least 90%, 95%, 96%, 97%, 98% or 99% sequence identity therewith;
(7) the sequence shown in SEQ ID NO: 179 or a heavy chain variable region sequence having at least 90%, 95%, 96%, 97%, 98% or 99% sequence identity therewith;
(8) the sequence shown in SEQ ID NO: 181 or a heavy chain variable region sequence having at least 90%, 95%, 96%, 97%, 98% or 99% sequence identity therewith;
(9) the sequence shown in SEQ ID NO: 183 or a heavy chain variable region sequence having at least 90%, 95%, 96%, 97%, 98% or 99% sequence identity therewith;
(10) the sequence shown in SEQ ID NO: 185 or a heavy chain variable region sequence having at least 90%, 95%, 96%, 97%, 98% or 99% sequence identity therewith; and
(11) the sequence shown in SEQ ID NO: 187 or a heavy chain variable region sequence having at least 90%, 95%, 96%, 97%, 98% or 99% sequence identity therewith.

21. The CAR according to any one of claims 12-20, further comprising a hinge region, a transmembrane region, a co-stimulatory domain and an activation domain; preferably, the hinge region is a CD8 hinge region, the transmembrane region is a CD8α transmembrane region; the co-stimulatory domain is a 41BB co-stimulatory domain or a CD28 co-stimulatory domain; and the activation domain is a CD3ζ activation domain.

22. The CAR according to any one of claims 12-21, wherein the hinge region comprises the sequence shown in SEQ ID NO: 156; the transmembrane region comprises the sequence shown in SEQ ID NO: 158; the co-stimulatory domain comprises the sequence shown in SEQ ID NO: 160 and/or SEQ ID NO: 162; and the activation domain comprises the sequence shown in SEQ ID NO: 164.

23. The CAR according to any one of claims 12-22, wherein the CAR further comprises a signal peptide; preferably, the signal peptide is a CD8α signal peptide; and more preferably, the signal peptide comprises the sequence shown in SEQ ID NO: 154.

24. The CAR according to any one of claims 12-23, wherein the signal peptide comprises the sequence shown in SEQ ID NO: 154.

25. A nucleic acid molecule encoding:
1) the antibody or antigen-binding fragment thereof according to any one of claims 1-11, or the heavy chain variable region and/or the light chain variable region of said antibody or antigen-binding fragment thereof; or
2) the CAR according to any one of claims 12-24.

26. The nucleic acid molecule according to claim 25, comprising a nucleotide sequence shown in any one of SEQ ID NO: 153, 155, 157, 159, 161, 163, 165, 168, 170, 172, 174, 176, 178, 180, 182, 184, 186, or 188.

27. An expression vector comprising the nucleic acid molecule according to claim 25 or claim 26; preferably, the expression vector is a lentiviral vector.

28. A cell comprising the expression vector according to claim 27.

29. A cell expressing the CAR according to any one of claims 12-24.

30. The cell according to claim 28 or claim 29, which is an immune cell.

31. The cell according to any one of claims 28-30, which is a T cell or an NK cell.

32. The cell according to any one of claims 29-31, wherein the cell is further engineered to express a dominant negative receptor (DNR) or a chimeric switch receptor (CSR); preferably, the DNR is dnTGFβRII and/or dnPD1; preferably, the intracellular domain of the CSR is from IL7Rα; more preferably, the dnTGFβRII comprises the sequence shown in SEQ ID NO: 193, and the intracellular domain of the CSR comprises the sequence shown in SEQ ID NO: 197.

33. A pharmaceutical composition comprising:
1) the antibody or antigen-binding fragment thereof according to any one of claims 1-11, the nucleic acid molecule according to claim 25 or claim 26, the expression vector according to claim 27, or the cell according to any one of claims 29-32; and
2) a pharmaceutically acceptable carrier.

34. Use of the antibody or antigen-binding fragment thereof according to any one of claims 1-11, the nucleic acid molecule according to claim 25 or claim 26, the expression vector according to claim 27, or the cell according to any one of claims 29-32 in the preparation of a medicament for treating a tumor.

35. The use according to claim 34, wherein the tumor expresses EGFRvIII.

36. The use according to claim 34 or claim 35, wherein the tumor is glioblastoma.

37. A method for treating a tumor in a subject, comprising administering to the subject a therapeutically effective amount of the antibody or antigen-binding fragment thereof according to any one of claims 1-11, the nucleic acid molecule according to claim 25 or claim 26, the expression vector according to claim 27, the cell according to any one of claims 29-32, or the pharmaceutical composition according to claim 33.

38. The method according to claim 37, wherein the tumor expresses EGFRvIII.

39. The method according to claim 37 or claim 38, wherein the tumor is glioblastoma.

40. A multispecific antibody molecule comprising at least a first functional portion and a second functional portion, wherein the first functional portion comprises the antibody or antigen-binding fragment thereof according to any one of claims 1-11, and the second functional portion has a different antigen-binding specificity from the first functional portion.

41. The multispecific antibody molecule according to claim 40, wherein the second functional portion has a binding specificity for a T cell.

42. An immunoconjugate comprising the antibody or antigen-binding fragment thereof according to any one of claims 1-11 which is linked with a therapeutic agent.

43. The immunoconjugate according to claim 42, wherein the therapeutic agent is a drug.

44. The immunoconjugate according to claim 42 or claim 43, wherein the therapeutic agent is a cytotoxin.

45. The immunoconjugate according to any one of claims 42-44, wherein the therapeutic agent is a radioactive isotope.

46. A kit for determining the presence or content of EGFRvIII in a sample, wherein the kit comprises the antibody or antigen-binding fragment thereof according to any one of claims 1-11.
